# EUROPEAN PATENT SPECIFICATION

(11) **EP 2 831 104 B1**
(45) Date of publication and mention of the grant of the patent: **21.04.2021**
(21) Application number: 13768248.0
(22) Date of filing: 15.03.2013
(51) Int. Cl.: C07K 14/495, G01N 33/68

(54) **BIOMARKERS FOR USE IN INTEGRIN THERAPY APPLICATIONS**
BIOMARKER ZUR VERWENDUNG IN INTEGRINTHERAPIEANWENDUNGEN
BIOMARQUEURS UTILISABLES DANS DES APPLICATIONS DE TRAITEMENT PAR L'INTÉGRINE

(30) Priority: 29.03.2012 US 201261617451 P; 17.05.2012 US 201261648199 P
(43) Date of publication of application: 04.02.2015
(73) Proprietor: Biogen MA Inc., Cambridge, MA 02142 (US); The Regents of the University of California, Oakland, CA 94607 (US)
(72) Inventor: VIOLETTE, Shelia M., Lexington, Massachusetts 02420 (US); SHEPPARD, Dean, Oakland, California 94610 (US)
(74) Representative: Pohlman, Sandra M.
(86) International application number: PCT/US2013/032082
(87) International publication number: WO 2013/148316

(56) References cited:
- WO-A1-03/097615
- WO-A1-2005/039547
- WO-A1-2008/147434
- WO-A1-2009/103542
- WO-A1-2010/072348
- WO-A2-2005/044794
- WO-A2-2008/008315
- WO-A2-2012/031008
- US-A1- 2011 305 629
- XU ET AL.: 'Lysophosphatidic Acid Induces avB6 ntegrin-Mediated TGF-B Activation via the LPA2 Receptor and the Small G Protein Gaq.' AMERICAN JOURNAL OF PATHOLOGY vol. 174, April 2009, pages 1264 - 1279, XP055169217

## Description

### CROSS-REFERENCE TO RELATED APPLICATIONS

### FIELD OF THE INVENTION

The present invention relates generally to the field of pharmacogenomics, and more specifically to methods and procedures to determine drug sensitivity in patients to allow the identification of individualized genetic profiles which will aid in treating diseases and disorders which are amenable to treatment with therapies designed against αvβ6-integrin.

### BACKGROUND OF THE INVENTION

It is increasingly being realized that there is no "one-size fits all" therapy for the treatment of complex multifactorial diseases. While modern medicaments save millions of lives a year, it is well understood that any particular medication or treatment regimen may not work in a particular individual or may cause severe side effects in one individual but be adequate for the treatment of the same disorder in another individual. This has led to an ever increasing interest in pharmacogenomics as a mechanism by which to provide personalized medicine tailored to a specific individual's disease. Although conventional histological and clinical features are increasingly used to correlate with prognosis, there remains a need for providing more specific parameters by which to determine responsiveness to therapy and consequent survival of the patient.

New prognostic and predictive markers, which would facilitate an individualization of therapy for each patient, are needed to accurately predict patient response to treatments. This is particularly the case in the use of biological molecule drugs, in the clinic. The problem may be addressed by clearly identifying predictive parameters that could be used to assess a patient's sensitivity to a particular treatment regimen. The classification samples can lend a great deal of certainty to diagnosis and treatment for a specific condition and patient. By correlating molecular and genetic markers with a patient's response to a treatment, it is possible to develop new treatments in non-responding patients, to tailor the treatment regimen for the specific patient or distinguish a treatment's indication among other treatment choices because of higher confidence in the efficacy. In addition, the availability of specific biomarkers for a particular disorder will allow pre-selection of patients clinical intervention.

There are numerous microarray technologies that readily allow for the large scale characterization of gene expression patterns. Such molecular tools have made it possible to monitor the expression level of a large number of transcripts from a biological sample. Numerous studies have demonstrated that gene expression information generated by microarray analysis of human disease can predict clinical outcome. These findings bring hope that cancer treatment will be vastly improved by better predicting the response of individual tumors to therapy. Similar tactics can be employed with other disorders. Despite this promise, markers still need to be identified for their predictive value for response to a particular therapeutic regimen.

Tissue fibrosis is a pathological process characterized by the replacement of diseased tissue with excess extracellular matrix, leading to organ scarring and failure. It is a progressive process that that is promoted by epithelial injury, fibroblast activation, inflammation, and reorganization of cellular interactions with the extracellular matrix (ECM). There is a strong rationale for targeting the TGF-β pathway as a means of inhibiting fibrosis. This cytokine is central to the initiation and maintenance of fibrosis and it has been shown in a variety of tissues that blocking this pathway provides potent anti-fibrotic effects.

TGF-β is secreted as an inactive latent complex requiring activation prior to engaging its cognate receptors. A critical regulator of TGF-β activation is the αvβ6 integrin, which binds to the N-terminal region of this cytokine converting it to an activated form. αvβ6 is expressed at low or undetectable levels on normal tissue but is highly up-regulated on epithelial cells during tissue injury and fibrosis. αvβ6 has been found to be most prominently up-regulated in the kidney, lung, liver, and skin inducing tissue specific activation of TGF-β. Several studies have clearly demonstrated that blocking αvβ6 function provides potent anti-fibrotic activity by interfering with TGF-β activation and downstream signaling events.

WO 2008/008315 relates to the discovery that there is a relationship between the expression levels of the tumor suppressor gene smad4 (also known as dpc4) and integrin αvβ6, and the responsiveness of patient populations to αvβ6-active compounds and compositions (e.g., antibodies and other ligands that bind αvβ6), particularly in cancer cells from such patient populations, more particularly on carcinomas such as pancreatic carcinomas.

US 2011/305629 provides humanized antibodies that recognize αvβ6 integrins, which antibodies comprise a variable region of nonhuman origin and at least a portion of an immunoglobulin of human origin. Also provided are methods for preparation of such antibodies, pharmaceutical compositions comprising them, and methods of treating, diagnosing and/or preventing various diseases and disorders by administering the humanized anti-αvβ6 antibodies.

It is proposed that one can monitor the response to anti-αvβ6 antibody treatment by monitoring genes that are differentially expressed in mammalian cells, tissue, or body fluids as a result of treatment with such antibodies. Likewise, we propose that one can also monitor response to anti-αvβ6 antibody treatment by monitoring protein expression changes (including post-translational modifications such as phosphorylation) in mammalian cells, tissue, or body fluids as a result of treatment with such antibodies. Transcriptional changes in gene expression, and changes in protein expression, have the potential to be used as markers of disease progression in humans and for monitoring the effectiveness of therapeutic intervention.

Despite the studies in the field that show that biomarkers would be useful for providing specific information regarding therapeutic intervention of various diseases, there still remains the need to identify specific diagnostic marker panels that allow for the tailored approach to therapy of a particular disease. There is also the need to identify biomarkers that are predictive of response to anti-fibrotic agents. The present invention is related to new methods and procedures for use in identifying patients that are responders to particular therapy to allow the development of individualized genetic profiles which are necessary to treat diseases and disorders involving intervention with an anti- αvβ6-integrin antibody based on patient response at a molecular level. This invention is also related to identifying biomarkers that can be used to monitor the response to anti-fibrotic agents and may be predictive of a clinical response to these agents.

### BRIEF SUMMARY OF THE INVENTION

The present invention identifies biomarkers that are useful in αvβ6-integrin-directed therapy.

The invention provides a method for predicting whether a human subject who has an αvβ6 mediated disorder will respond to treatment with an αvβ6-integrin inhibitor, the method comprising: a) providing a biological sample that has been obtained from the human subject after having been administered the αvβ6-integrin inhibitor; and b) measuring the expression level of one or more gene(s) or protein(s) selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in the biological sample, wherein a decrease in the expression level of the one or more gene(s) or protein(s) relative to a control expression level predicts that the human subject will respond, or has an increased likelihood of responding, to the treatment with the αvβ6-integrin inhibitor, wherein the αvβ6-meditated disorder is selected from the group consisting of lung fibrosis, idiopathic pulmonary fibrosis, radiation induced fibrosis, bleomycin induced fibrosis, asbestos induced fibrosis, lung cancer, and acute lung injury, scleroderma, wherein the αvβ6-integrin inhibitor as an anti-αvβ6-integrin antibody and wherein the biological sample is bronchoalveolar lavage fluid. The invention further provides an αvβ6-integrin inhibitor for use in a method for predicting responsiveness of a human subject who has an αvβ6-mediated disorder to the αvβ6-integrin inhibitor, the method comprising: (a) measuring the expression level of a gene or protein selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in a first biological sample obtained from the human subject before the αvβ6-integrin inhibitor is administered; and (b) measuring the expression level of the gene or protein selected from the group consisting of arachidonate 5-lipoxygenase 5 (ALOX5), fibronectin (FN1),oxidized low density lipoprotein receptor 1 (OLR1), plasminogen activator inhibitor-1 (PAI-1 or SERPINE 1 transglutaminase 2 (TGM2), and triggering receptor expressed on myeloid cells 1 (TREM1) in a second biological sample obtained from the human subject after the αvβ6-integrin inhibitor has been administered, wherein a decrease in the level of expression of the gene or protein measured in step (b), compared to the level of expression of the gene or protein measured in step (a) predicts that the human subject will respond, or has an increased likelihood of responding, to treatment with the αvβ6-integrin inhibitor, wherein the αvβ6-mediated disorder is selected from the group consisting of lung fibrosis, idiopathic pulmonary fibrosis, radiation induced fibrosis, bleomycin induced fibrosis, asbestos induced fibrosis,/ lung cancer, and acute lung injury, scleroderma, wherein the αvβ6-integrin inhibitor is an anti-αvβ6-integrin antibody, and wherein the biological sample is bronchoalveolar lavage fluid. The invention further provides a therapeutically effective amount of an αvβ6 integrin inhibitor for use in treating an αvβ6-mediated disorder in a human subject in need thereof, wherein the human subject has been identified as having: an increased expression level of a gene or protein selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in a biological sample obtained from the human subject, compared to a control expression level, wherein the αvβ6-mediated disorder is selected from the group consisting of lung fibrosis, idiopathic pulmonary fibrosis, radiation induced fibrosis, bleomycin induced fibrosis, asbestos induced fibrosis, lung cancer, and acute lung injury, scleroderma, wherein the αvβ6-integrin inhibitor is an anti-αvβ6-integrin antibody, and wherein the biological sample is bronchoalveolar lavage fluid. The invention also relates to a biomarker panel comprising a probe for each of ALOX5, FN1, OLR1, SERPINE1, TGM2, and TREM1 and no additional genes or proteins other than one or more of the genes or proteins selected from any one of SEQ ID NOs:1-1175.

The disclosure features a method for predicting whether a human subject who has an αvβ6-mediated disorder will respond to treatment with an αvβ6-integrin inhibitor. The method involves providing a biological sample obtained from the human subject after administration of the αvβ6-integrin inhibitor and measuring the expression level of a gene or protein from Table 1 or a gene or protein from Table 2 in the biological sample. An increase in the expression level of the gene or protein from Table 1 relative to a control expression level or a decrease in the expression level of the gene or protein from Table 2 relative to a control expression level after administration of the αvβ6 integrin inhibitor, predicts that the human subject will have a clinical response, or has an increased likelihood of a clinical response, to treatment with the αvβ6-integrin inhibitor. In certain embodiments, the method further involves determining the phosphorylation status of SMAD2 protein in the biological sample. A decrease in the phosphorylation status of SMAD2 protein after administration of the αvβ6 integrin inhibitor compared to a control level is a further predictor that the human subject will have a clinical response, or has an increased likelihood of a clinical response, to treatment with the αvβ6-integrin inhibitor. The method may further involve determining the expression level (e.g., mRNA, protein) in peripheral blood or bronchoalveolar lavage of one or more (e.g., one, two, three, four, five, six, seven) serum biomarkers such as, but not limited to, tissue remodeling markers (e.g., metalloproteinase 7 (MMP-7), osteopontin (OPN)); TGF-β inducible proteins (e.g., tissue inhibitor of metalloproteinase 1 (TIMP-1), collagen type 1alpha1 (Col1A1)); and epithelial injury markers (e.g., surfactant A (SP-A), alpha defensins (DEFA1-3)). A decrease in the expression level (e.g., mRNA, protein) of one or more of the above serum biomarkers in peripheral blood is a further predictor that the human subject will have a clinical response, or has an increased likelihood of a clinical response, to treatment with the αvβ6-integrin inhibitor. In certain embodiments, the method comprises measuring any combination of at least 6 genes or proteins from Table 1, Table 2, or Tables 1 and 2. In some embodiments, a decrease in the expression level of at least one of: arachidonate 5-lipoxygenase 5 (ALOX5), fibronectin (FN1), oxidized low density lipoprotein receptor 1 (OLR1), plasminogen activator inhibitor-1 (PAI-1 also known as SERPINE1), transglutaminase 2 (TGM2), or triggering receptor expressed on myeloid cells 1 (TREM1) after administration of the αvβ6 integrin inhibitor in the biological sample is measured and predicts that the human subject will have a clinical response, or has an increased likelihood of a clinical response, to treatment with the αvβ6-integrin inhibitor.

The disclosure provides a method for predicting whether a human subject who has an αvβ6-mediated disorder will respond to treatment with an αvβ6-integrin inhibitor. The method involves providing a biological sample obtained from the human subject before treatment with an αvβ6-integrin inhibitor and measuring the expression level of a gene or protein from Table 1 or Table 2 relative to a predicted control level (e.g., compare the expression level to a predicted normal value or range of values). Subjects with decreased expression of the gene or the protein from Table 1, or increased expression of the gene or the protein from Table 2, relative to a predicted control level are predicted to have a clinical response, or have an increased likelihood of a clinical response, to treatment with the αvβ6-integrin inhibitor. The method may further involve determining the phosphorylation status of SMAD2 protein in the biological sample. An increase in the phosphorylation status of SMAD2 protein relative a control level is a further predictor that the human subject will have a clinical response, or has an increased likelihood of a clinical response, to treatment with the αvβ6-integrin inhibitor. In certain embodiments, the method further involves determining the expression level (e.g., mRNA, protein) in peripheral blood or bronchoalveolar lavage of one or more (e.g., one, two, three, four, five, six, seven) serum biomarkers such as, but not limited to, tissue remodeling markers (e.g., metalloproteinase 7 (MMP-7), osteopontin (OPN)); TGF-β inducible proteins (e.g., tissue inhibitor of metalloproteinase 1 (TIMP-1), collagen type 1alpha1 (Col1A1)); and epithelial injury markers (e.g., surfactant A (SP-A), alpha defensins (DEFA1-3)). An increase in the expression level (e.g., mRNA, protein) of one or more of the above serum biomarkers in peripheral blood is a further predictor that the human subject will have a clinical response, or has an increased likelihood of a clinical response, to treatment with the αvβ6-integrin inhibitor. The method may comprise measuring any combination of at least 6 genes or proteins from Table 1, Table 2, or Tables 1 and 2. In some embodiments, an increase in the expression level (e.g., mRNA or protein) of at least one of: arachidonate 5-lipoxygenase 5 (ALOX5), fibronectin (FN1), oxidized low density lipoprotein receptor 1 (OLR1), plasminogen activator inhibitor-1 (PAI-1 also known as SERPINE1), transglutaminase 2 (TGM2), or triggering receptor expressed on myeloid cells 1 (TREM 1) in the biological sample is measured and predicts that the human subject will have a clinical response, or has an increased likelihood of a clinical response, to treatment with the αvβ6-integrin inhibitor.

The disclosure features a method for predicting responsiveness of a human subject to treatment with an inhibitor of a TGF-β-signaling pathway. The method involves measuring the expression level of a gene or protein from Table 1 or a gene or protein from Table 2 in a first biological sample obtained from the human subject, then administering the inhibitor of a TGF-β-signaling pathway to the human subject, and finally measuring the expression level of the gene or protein from Table 1 or the gene or protein from Table 2 in a second biological sample obtained from the human subject. An increase in the level of expression of the gene or protein from Table 1 or a decrease in the level of expression of the gene or protein from Table 2 in the second biological sample compared to the level of expression of the gene or protein measured in the first biological sample predicts that the human subject will have a clinical response, or has an increased likelihood of having a clinical response, to treatment with the inhibitor of the TGF-β-signaling pathway. The method may comprise measuring any combination of at least 6 genes or proteins from Table 1, Table 2, or Tables 1 and 2. In some embodiments, a decrease in the expression level (e.g., mRNA or protein) of at least one of: arachidonate 5-lipoxygenase 5 (ALOX5), fibronectin (FN1), oxidized low density lipoprotein receptor 1 (OLR1), plasminogen activator inhibitor-1 (PAI-1 or SERPlNE1), transglutaminase 2 (TGM2), or triggering receptor expressed on myeloid cells 1 (TREM1) in the biological sample is measured and predicts that the human subject will have a clinical response, or has an increased likelihood of having a clinical response, to treatment with the inhibitor of a TGF-β-signaling pathway. In certain embodiments, the method further involves determining the phosphorylation status of SMAD2 protein in the first and second biological samples. A decrease in the phosphorylation status of SMAD2 protein in the second biological sample compared to the first biological sample is a further predictor that the human subject will respond, or has an increased likelihood of responding, to treatment with the inhibitor of a TGF-β-signaling pathway. The method may further involve determining the expression level (e.g., mRNA, protein) in peripheral blood or bronchoalveolar lavage of one or more (e.g., one, two, three, four, five, six, seven) serum biomarkers such as, but not limited to, tissue remodeling markers (e.g., metalloproteinase 7 (MMP-7), osteopontin (OPN)); TGF-β inducible proteins (e.g., tissue inhibitor of metalloproteinase 1 (TIMP-1), collagen type 1alpha1 (Col1A1)); and epithelial injury markers (e.g., surfactant A (SP-A), alpha defensins (DEFA1-3)). A decrease in the expression level of one or more of the above serum biomarkers predicts that the human subject will have a clinical response, or has an increased likelihood of having a clinical response, to treatment with the inhibitor of a TGF-β-signaling pathway.

The invention provides anti αvβ6-antibodies for use in treating an αvβ6-mediated disorder in a human subject in need thereof. The use may comprise J administering to the human subject a therapeutically effective amount of an αvβ6 integrin inhibitor, wherein the human subject has been identified as having at least one of: (i) a decreased expression level of a gene or protein from Table 1 in a biological sample obtained from the human subject prior to administration of the αvβ6 integrin inhibitor, compared to a control expression level; or (ii) an increased expression level of a gene or protein from Table 2 in a biological sample obtained from the human subject prior to administration of the αvβ6 integrin inhibitor, compared to a control expression level. Alternatively, the use may comprise administering to the human subject a therapeutically effective amount of an αvβ6 integrin inhibitor, wherein the human subject has previously been administered the αvβ6 integrin inhibitor and has been identified as having at least one of: (i) an increased expression level of a gene or protein from Table 1 in a biological sample obtained from the human subject after the previous administration of the αvβ6 integrin inhibitor, compared to a control expression level; or (ii) a decreased expression level of a gene or protein from Table 2 in a biological sample obtained from the human subject after the previous administration of the αvβ6 integrin inhibitor, compared to a control expression level. The method involves measuring any combination of at least 6 genes or proteins from Table 1, Table 2, or Tables 1 and 2. The use may further involve identifying that the human subject has a decrease in the phosphorylation status of SMAD2 protein after administration of the αvβ6 integrin inhibitor and this is a further predictor that the human subject will respond, or has an increased likelihood of responding, to treatment with the αvβ6-integrin inhibitor. The use may involve determining the expression level (e.g., mRNA, protein) in peripheral blood or bronchoalveolar lavage of one or more (e.g., one, two, three, four, five, six, seven) serum biomarkers such as, but not limited to, tissue remodeling markers (e.g., metalloproteinase 7 (MMP-7), osteopontin (OPN)); TGF-β inducible proteins (e.g., tissue inhibitor of metalloproteinase 1 (TIMP-1), collagen type 1alpha1 (Col1A1)); and epithelial injury markers (e.g., surfactant A (SP-A), alpha defensins (DEFA1-3)) compared to a control expression level. In certain embodiments, the method comprises determining the expression level of at least one of: arachidonate 5-lipoxygenase 5 (ALOX5), fibronectin (FN1), oxidized low density lipoprotein receptor 1 (OLR1), plasminogen activator inhibitor-1 (PAI-1 also known as SERPINE1), transglutaminase 2 (TGM2), or triggering receptor expressed on myeloid cells 1 (TREM1) in the biological sample.

In certain embodiments, the mRNA level of the gene is measured. In other embodiments, the expression level of the protein is measured. The biological sample is a bronchoalveolar lavage fluid. In certain embodiments, the biological sample comprises bronchoalveolar lavage cells. The biological sample may be a tissue sample (e.g., lung tissue). The biological sample may be a bodily fluid sample

Also described herein, the αvβ6-mediated disorder is psoriasis, sclerosis, cancer, acute and chronic renal injury, acute and chronic liver injury, transplant, or Alports Syndrome. In some embodiments, the αvβ6-mediated disorder is lung fibrosis In one embodiment, the αvβ6-mediated disorder is interstitial lung disease with usual interstitial pneumonia (UIP). In certain embodiments, the αvβ6-mediated disorder is idiopathic pulmonary fibrosis, radiation induced fibrosis, bleomycin induced fibrosis, asbestos induced fibrosis, flu induced fibrosis, coagulation induced fibrosis, or vascular injury induced fibrosis. In one embodiment, the αvβ6-mediated disorder is acute lung injury. In some embodiments, the αvβ6-mediated disorder is lung cancer. In certain embodiments, the αvβ6-integrin inhibitor is an anti-αvβ6-integrin antibody. For example, the anti-αvβ6-integrin antibody can have the same CDRs as an antibody produced by a hybridoma selected from the group consisting of: 6.1A8 (ATCC accession number PTA-3647); hybridoma 6.3G9 (ATCC accession number PTA-3649); 6.8G6 (ATCC accession number PTA-3645); 6.2E5 (ATCC accession number PTA-3897); 6.2B1 (ATCC accession number PTA-3646); hybridoma 7.1G10 (ATCC accession number PTA-3898); 7.7G5 (ATCC accession number PTA-3899); and hybridoma 7.1C5 (ATCC accession number PTA-3900). In some embodiments, the anti-αvβ6-integrin antibody has the same CDRs as the antibody produced by the hybridoma deposited as 6.3G9 (ATCC accession number PTA-3649), except that the light chain CDR 1 contains an asparagine to serine substitution such that the light chain CDR 1 sequence is the sequence of SASSSVSSSYLY (SEQ ID NO:1196). In certain embodiments, the anti-αvβ6-integrin antibody comprises a heavy chain variable region comprising the amino acid sequence set forth in SEQ ID NO: 1210. In a specific embodiment, the anti-αvβ6-integrin antibody further comprises a light chain variable region comprising the amino acid sequence set forth in SEQ ID NO: 1211.

The "control expression level" is the expression level of the gene or protein of interest prior to administration of the anti-αvβ6-integrin inhibitor, or a pre-determined cut-off value. A cut-off value is typically an expression level of a gene (or protein), or ratio of the expression level of a gene (or protein) with the expression level of another gene (or protein) (e.g., an internal control such as a housekeeping gene), above or below which is considered predictive of responsiveness of a subject to a treatment comprising anti-αvβ6-integrin inhibitor or a TGF-β pathway inhibitor. Thus, in accordance with the methods described herein, a reference expression level of a gene (e.g., a gene depicted in Table 1 or 2) is identified as a cut-off value, above or below of which is predictive of responsiveness to a therapy comprising anti-αvβ6-integrin inhibitor (or a TGF-β pathway inhibitor). Some cut-off values are not absolute in that clinical correlations can still remain significant over a range of values on either side of the cutoff; however, it is possible to select an optimal cut-off value (e.g. varying H-scores) of expression levels of genes for particular sample types. Cut-off values determined for use in the methods described herein can be compared with, e.g., published ranges of expression levels but can be individualized to the methodology used and patient population. It is understood that improvements in optimal cut-off values could be determined depending on the sophistication of statistical methods used and on the number and source of samples used to determine reference level values for the different genes and sample types. Therefore, established cut-off values can be adjusted up or down, on the basis of periodic re-evaluations or changes in methodology or population distribution. The reference expression level of one or more genes (or proteins) can be determined by a variety of methods. The reference level can be determined by comparison of the expression level of a gene (or protein) of interest in, e.g., populations of subjects (e.g., patients) that are responsive to a therapy comprising anti-αvβ6-integrin inhibitor (or a TGF-β pathway inhibitor), or not responsive to this therapy. This can be accomplished, for example, by histogram analysis, in which an entire cohort of patients are graphically presented, wherein a first axis represents the expression level of a gene (or protein) and a second axis represents the number of subjects in the cohort whose sample contain one or more expression levels at a given amount. Determination of the reference expression level of a gene (or protein) can then be made based on an amount which best distinguishes these separate groups. The reference level can be a single number, equally applicable to every subject, or the reference level can vary, according to specific subpopulations of subjects. For example, older subjects can have a different reference level than younger subjects for the same αvβ6-mediated disorder. In addition, a subject with more advanced disease (e.g., a more advanced form of an αvβ6-mediated disorder) can have a different reference value than one with a milder form of the disease.

In a fifth aspect, the disclosure features a biomarker panel comprising a probe for each of ALOX5, FN1, OLR1, SERPINE1, TGM2, and TREM1 and no additional genes other than one or more of the genes listed in Table 1 and Table 2. In certain embodiments, the probe is a nucleotide probe. In some embodiments, the probe is a protein probe. In some embodiments, the probe is an antibody or an antigen-binding fragment thereof.

Herein disclosed is a method for predicting clinical responsiveness to an anti-αvβ6-integrin antibody, a small molecule inhibitor of αvβ6-integrin, or a micrRNA or siRNA of αvβ6-integrin, in a mammal having a disease, wherein the method comprises:
a) measuring the mRNA expression or protein level of the biomarkers from Table 1 in a tissue, bodily fluid or cell sample from said mammal, wherein an increase in the expression of mRNA or protein level of said biomarkers from Table 1 relative to a predetermined expression mRNA or protein level of said biomarkers in such a tissue, bodily fluid, or cell sample in response to treatment with said antibody, small molecule, microRNA or siRNA predicts an increased likelihood the mammal will respond clinically to said method of treatment with said antibody, small molecule inhibitor, microRNA or siRNA; and/or
b) measuring the mRNA expression or protein level of biomarkers from Table 2 in a tissue, bodily fluid, or cell sample from said mammal, wherein a decrease in the expression of mRNA or protein level of said biomarker relative to a predetermined expression of mRNA or protein level of said biomarker in such a tissue, bodily fluid or cell sample in response to treatment with said antibody, small molecule, microRNA or siRNA predicts an increased likelihood the mammal will respond clinically to said method of treatment with said antibody, small molecule inhibitor, microRNA, or siRNA.

More specifically, the method may comprise measuring any combination of at least 6 genes from Tables 1 and 2.

In addition, it may also be desirable to include the further step of determining the phosphorylation status of SMAD2 protein in said sample, wherein a decrease in that phosphorylation status in response to administration of an αvβ6-integrin inhibitor is indicative of inhibition of TGFβ activity.

The methods may further comprise the step of measuring the expression or protein level of at least one additional biomarker selected from Table 1, wherein an increase in expression of mRNA or protein level of said biomarker in cells, tissue, or bodily fluid in response to administering said antibody is indicative of an increased likelihood that the mammal will respond clinically to said therapy.

More particularly, the methods described herein at least comprise the determination of a decrease in expression of mRNA or protein level of at least one of ALOX5, FN1, OLR1, SERPINE1, TGM2, TREM1, ENPP1, IGSF2, or GPR82 in cells, tissue, or bodily fluid as being predictive of said mammal's response to said therapy. In one embodiment, the method comprises determining a decrease in expression of an mRNA or a protein level of at least one, at least two, at least three, at least four, at least five, or six of the following: ALOX5, FN1, OLR1, SERPINE1, TGM2, and, TREM1.

Also described are methods of selecting a test subject as a candidate to receive treatment with an anti-αvβ6-integrin antibody or a small molecule inhibitor of αvβ6-integrin wherein the method comprises:
a) determining the expression of mRNA or protein level of a plurality of biomarkers from Table 1 and Table 2 in a cell, bodily fluid or tissue sample from a test subject to be treated with said anti-αvβ6-integrin antibody or a small molecule inhibitor of αvβ6-integrin and
b) comparing the expression of mRNA or protein level of the biomarkers from Table 1 and Table 2 obtained in step (a) with either (i) the expression of mRNA or protein level of those biomarkers in a healthy subject and/or (ii) the expression of mRNA or protein level of said biomarkers from a subject known to be responsive to said therapy; and
c) selecting the test subject as a candidate to receive said treatment if either (i) said subject has a decreased expression of mRNA or protein level of the biomarkers of Table 1 or an increased expression of mRNA or protein level of the biomarkers in Table 2 as compared to a healthy subject, or (ii) if said subject has expression of mRNA or protein level of the biomarkers comparable to the levels of those biomarkers in subjects known to have fibrosis that is responsive to said therapy.

Additional embodiments of methods of selecting a subject for the therapies as described herein may comprise determining the phosphorylation status of SMAD2 protein in said sample, wherein an elevated level of SMAD2 phosphorylation status as compared to the SMAD2 phosphorylation status of healthy subjects is indicative that said subject will be responsive to said therapy.

The methods describe herein may comprise measuring any combination of at least 6 biomarkers from Tables 1 and 2. The methods may further comprise the step of measuring the expression of mRNA or protein level of at least one additional biomarker selected from Table 1, wherein a decrease in expression of mRNA or protein level of said biomarker in cells, tissue, or bodily fluid of said test subject as compared to a healthy subject is indicative that said subject will be responsive to said therapy. More specifically, in such methods of selecting a candidate for therapy, the methods involve monitoring the increase in expression of mRNA or protein level of at least one of ALOX5, FN1, OLR1, SERPINE1, TGM2, TREM1, ENPP1, IGSF2, or GPR82 in cells, tissue, or bodily fluid, which if measured as compared to the level of expression of said genes in said subject is indicative that said subject will be responsive to said therapy. In one embodiment, in such methods of selecting a candidate for therapy, the methods involve monitoring the increase in expression of an mRNA or a protein level of at least one, at least two, at least three, at least four, at least five, or all six of: ALOX5, FN1, OLR1, SERPINE1, TGM2, or TREM1.

In the methods described herein a particularly preferred sample is bronchoalveolar lavage sample. The sample may be tissue sample.

The sample may be a bodily fluid.

The subject being treated or selected described herein is a subject having or suspected of having a disorder selected from the group consisting of psoriasis, sclerosis, cancer, acute and chronic renal injury, acute and chronic liver injury, transplant or Alports Syndrome. In one embodiment, the subject has or is suspected of having lung fibrosis. In a particular embodiment, the subject has or is suspected of having idiopathic pulmonary fibrosis (IPF). In another embodiment, the subject has or is suspected of having flu induced fibrosis, coagulation induced fibrosis, or vascular injury induced fibrosis. In one embodiment, the subject has or is suspected of having acute lung injury. In one embodiment, the subject has or is suspected of having a lung cancer.

For predicting the responsiveness of a subject to therapy, the methods may involve the step of measuring at least one additional biomarker selected from Table 2, wherein a decrease in expression of mRNA or protein level of said biomarker in cells, tissue, or bodily fluid in response to administering said -αvβ6 inhibitor is indicative of an increased likelihood that the mammal will respond clinically to said therapy. Alternatively, or in addition, the method may further comprise the step of measuring at least one additional biomarker selected from Table 1, wherein an increase in expression of mRNA or protein level of said biomarker in cells, tissue, or bodily fluid in response to administering said antibody is indicative of an increased likelihood that the mammal will respond clinically to said therapy.

The methods may further comprise the step of measuring at least one additional biomarker selected from Table 2, wherein an increase in expression of mRNA or protein level of said biomarker in cells, tissue, or bodily fluid as compared to the expression of said biomarker in a healthy subject is indicative of an increased likelihood that the subject will respond clinically to said therapy. Alternatively, or in addition, the methods may further comprise the step of measuring at least one additional biomarker selected from Table 1, wherein a decrease in expression of mRNA or protein level of said biomarker in cells, tissue, or bodily fluid as compared to the expression of said biomarker in a healthy subject is indicative of an increased likelihood that the subject will respond clinically to said therapy.

In embodiments in which the subject has lung fibrosis, the disease may be selected from the group consisting of idiopathic pulmonary fibrosis, radiation induced fibrosis, chronic obstructive pulmonary disease (COPD), scleroderma, bleomycin induced fibrosis, chronic asthma, silicosis, asbestos induced fibrosis, acute lung injury, and acute respiratory distress.

In embodiments, wherein the subject has acute respiratory distress, the disease is selected from the group consisting of bacterial pneumonia induced acute respiratory distress, trauma induced acute respiratory distress, viral pneumonia induced acute respiratory distress, ventilator induced acute respiratory distress, non-pulmonary sepsis induced acute respiratory distress, aspiration induced acute respiratory distress, and interstitial lung disease with usual interstitial pneumonia (UIP).

The subject may be any mammal including, e.g., a mammal selected from the group consisting of: human, rat, mouse, dog, rabbit, pig, sheep, cow, horse, cat, primate, and monkey.

In the uses described herein the therapy may be with an antibody, wherein said antibody is selected from the group consisting of a monoclonal, polyclonal or single chain antibody.

In exemplary embodiments, the antibody has the same CDRs as a murine antibody produced by hybridoma 6.1A8 (ATCC accession number PTA-3647); hybridoma 6.3G9 (ATCC accession number PTA-3649); 6.8G6 (ATCC accession number PTA-3645); hybridoma 6.2B1 (ATCC accession number PTA-3646); hybridoma 7.1G10 (ATCC accession number PTA-3898); 7.7G5 (ATCC accession number PTA-3899); hybridoma 2E5 (ATCC accession number PTA-3897); or hybridoma 7.1C5 (ATCC accession number PTA-3900).

More specifically, the antibody has the same CDRs as murine antibody deposited as hybridoma 6.3G9 (ATCC accession number PTA-3649).

In some embodiments, the antibody has CDRs having three or fewer, two or fewer, or one amino acid substitution(s) in CDR 1 and/or CDR2, and/or CDR3 of a murine antibody produced by hybridoma 6.1A8 (ATCC accession number PTA-3647); hybridoma 6.3G9 (ATCC accession number PTA-3649); 6.8G6 (ATCC accession number PTA-3645); hybridoma 6.2B1 (ATCC accession number PTA-3646); hybridoma 7.1G10 (ATCC accession number PTA-3898); 7.7G5 (ATCC accession number PTA-3899); hybridoma 2E5 (ATCC accession number PTA-3897); or hybridoma 7.1C5 (ATCC accession number PTA-3900). In certain embodiments, the amino acid substitution(s) are conservative amino acid substitution(s).

In other embodiments, the antibody has the same CDRs as the murine antibody produced by hybridoma 6.3G9 (ATCC accession number PTA-3649) except that the light chain CDR 1 contains an asparagine to serine substitution such that the light chain CDR 1 sequence is the sequence of SASSSVSSSYLY (SEQ ID NO: 1196).

In still other embodiments, the antibody has:
a) a heavy chain sequence comprising the sequence of GFTFSRYVMS (SEQ ID NO: 1178) as heavy chain CDR 1, SISSGGRMYYPDTVKG (SEQ ID NO:1185) as heavy chain CDR 2, and GSIYDGYYVFPY (SEQ ID NO: 1191) as heavy chain CDR 3;
b) a light chain sequence comprising the sequence of SANSSVSSSYLY (SEQ ID NO: 1197) or SASSSVSSSYLY (SEQ ID NO:1196) as light chain CDR 1, STSNLAS (SEQ ID NO:1202) as light chain CDR 2 and HQWSTYPPT (SEQ ID NO:1206) as light chain CDR 3.

In still further embodiments, the antibody is a humanized antibody, a chimeric antibody, a single chain antibody or an antibody construct that is capable of binding to and blocking αvβ6-integrin and comprises at least one heavy chain variable domain and one light chain variable domain comprising each of CDR 1, CDR 2, and CDR 3 from the light chain and heavy chain as follows:
a) a heavy chain sequence comprising the sequence of GFTFSRYVMS (SEQ ID NO: 1178) as heavy chain CDR 1, SISSGGRMYYPDTVKG (SEQ ID NO:1185) as heavy chain CDR 2, and GSIYDGYYVFPY (SEQ ID NO: 1191) as heavy chain CDR 3;
b) a light chain sequence comprising the sequence of SANSSVSSSYLY (SEQ ID NO: 1197) or SASSSVSSSYLY (SEQ ID NO: 1196) as light chain CDR 1, STSNLAS (SEQ ID NO:1202) as light chain CDR 2 and HQWSTYPPT (SEQ ID NO:1206) as light chain CDR 3.

In another embodiment, the antibody can bind to and/or block αvβ6-integrin and comprises a heavy chain variable region comprising an amino acid sequence having 85%, 86%, 87%, 88%, 89% 90%, 91%, 92%, 93%, 94%, 95%, 96%, 97%, 98%, 99% or 100% identity to the amino acid sequence set forth in SEQ ID NO: 1210; and a light chain variable region comprising an amino acid sequence having 85%, 86%, 87%, 88%, 89% 90%, 91%, 92%, 93%, 94%, 95%, 96%, 97%, 98%, 99% or 100% identity to the amino acid sequence set forth in SEQ ID NO: 1211. In a specific embodiment, the antibody comprises a heavy chain variable region comprising the amino acid sequence set forth in SEQ ID NO: 1210 and a light chain variable region comprising the amino acid sequence set forth in SEQ ID NO: 1211.

In another embodiment, the antibody is a humanized antibody, a chimeric antibody, a single chain antibody or an antibody construct that is capable of binding to and blocking αvβ6-integrin and comprises at least one heavy chain variable domain and one light chain variable domain comprising each of CDR 1, CDR 2, and CDR 3 from the light chain and heavy chain as follows:
a) a heavy chain sequence comprising the sequence of SYTFTDYAMH (SEQ ID NO: 1176) as heavy chain CDR 1, VISTYYGNTNYNQKFKG (SEQ ID NO:1182) as heavy chain CDR 2, and GGLRRGDRPSLRYAMDY (SEQ ID NO: 1188) as heavy chain CDR 3;
b) a light chain sequence comprising the sequence of RASQSVSTSSYSYMY (SEQ ID NO: 1194) as light chain CDR 1, YASNLES (SEQ ID NO:1200) as light chain CDR 2 and QHNWEIPFT (SEQ ID NO:1203) as light chain CDR 3.In another embodiment, the antibody is a humanized antibody, a chimeric antibody, a single chain antibody or an antibody construct that is capable of binding to and blocking αvβ6-integrin and comprises at least one heavy chain variable domain and one light chain variable domain comprising each of CDR 1, CDR 2, and CDR 3 from the light chain and heavy chain of the antibody produced by hybridoma clone 2E5 (ATCC accession number PTA-3897).

The antibody therapy may be administered at a dose of between 0.015 mg/kg/week to 10 mg/kg/week. More particularly, the dose is between 0.5 mg/kg/week and 5 mg/kg/week.

The mammal may have a serum concentration of level of at least 2500µg/ml of said antibody.

Also described is a method of predicting responsiveness to an αvβ6-integrin inhibitor in a mammal that has a fibrosis, wherein the method comprises:
(a) measuring the mRNA expression or protein level of biomarkers from Table 1 and Table 2 in a bronchoalveolar lavage (BAL) sample of said mammal;
(b) administering an anti-αvβ6-integrin antibody, a small molecule inhibitor of αvβ6-integrin, a siRNA inhibitor of β6-integrin expression, a miRNA inhibitor to inhibit β6-integrin expression, or a miRNA mimetic to inhibit β6-integrin expression to said mammal;
(c) following the administering step (b), measuring in a BAL sample of said mammal the mRNA expression or protein level of said biomarkers from Table 1 and Table 2,
wherein an increase in the expression of mRNA or protein level of the biomarkers of Table 1 and/or a decrease in the expression of mRNA or protein level of the biomarkers of Table 2 measured in step (c) compared to the level of said biomarkers measured in step (a) predicts an increased likelihood the mammal will respond clinically to said method of treating fibrosis. In certain embodiments, the anti-αvβ6-integrin antibody is an antibody comprising the heavy chain variable domain sequence set forth in SEQ ID NO:1210 and the light chain variable domain sequence set forth in SEQ ID NO:1211. In one embodiment, the fibrosis is lung fibrosis. In a specific embodiment, the fibrosis is IPF.

Another method described herein is for selecting a subject that has fibrosis as a candidate for therapy with an αvβ6-integrin inhibitor wherein the method comprises:
(a) measuring the mRNA expression or protein level of the biomarkers from Table 1 and Table 2 in a bronchoalveolar lavage (BAL) sample of said subject;
(b) comparing the mRNA expression or protein levels measured in step (a) with either (i) the expression of mRNA or protein level of those biomarkers in a healthy subject and/or (ii) with the expression of mRNA or protein level of those biomarkers from subjects known to have fibrosis that is responsive to said therapy; and
c) selecting the test subject as a candidate to receive said treatment if said subject has either (i) a decreased expression mRNA or protein level of the biomarkers of Table 1 or an increased expression of mRNA or protein level of the biomarkers in Table 2 as compared to a healthy subject or (ii) if said subject has expression mRNA or protein level of the biomarkers comparable to the levels of those biomarkers in subjects known to have fibrosis that is responsive to said therapy.

Also herein disclosed is a method for predicting responsiveness to an inhibitor of a TGFβ-signaling pathway in a mammal, wherein the method comprises:
(a) measuring the mRNA expression or protein level of biomarkers from Table 1 and Table 2 in a tissue, bodily fluid or cell sample of said mammal;
(b) administering said inhibitor of TGFβ signaling pathway;
(c) following the administering step (b), measuring in said sample of said mammal the mRNA expression or protein level of said biomarkers from Table 1 and Table 2,
wherein an increase in the level of expression of the markers of Table 1 and/or a decrease in the level of expression of the markers of Table 2 measured in step (c) compared to the level of said biomarkers measured in step (a) predicts an increased likelihood the mammal will respond clinically to said inhibitor of TGFβ-signaling pathway.

Also disclosed herein is a method for selecting a treatment regimen for therapy with a αvβ6 integrin inhibitor in a subject, the method comprising:
a) assaying the subject for expression of mRNA or protein level of one or more biomarkers predictive of responsiveness to a αvβ6 integrin inhibitor for treatment of the disorder; and
b) selecting a treatment regimen with a αvβ6 integrin inhibitor based upon expression of mRNA or protein level of the one or more biomarkers in the subject.

Herein described is the treatment of a subject having a disorder with a αvβ6 integrin inhibitor, the method comprising:
a) assaying the subject for expression of mRNA or protein level of one or more biomarkers predictive of responsiveness to a αvβ6 integrin inhibitor for treatment of the disorder;
b) selecting a treatment regimen with a αvβ6 integrin inhibitor based upon expression of mRNA or protein level of the one or more biomarkers in the subject; and
c) administering the αvβ6 integrin inhibitor according to the treatment regimen such that the subject is treated for the disorder.

A further aspect of the invention describes a biomarker panel specifically for use in predicting the responsiveness of a subject to a particular therapeutic regimen, said biomarker panel comprising of at least ALOX5, FN1, OLR1, SERPINE1, TGM2, TREM1, ENPP1, IGSF2, and GPR82 and not more than the genes listed in Table 1 and Table 2 collectively. In one embodiment, the biomarker panel for use in predicting the responsiveness of a subject to a particular therapeutic regimen, comprises at least ALOX5, FN1, OLR1, SERPINE1, TGM2, and TREM1.

### BRIEF DESCRIPTION OF THE DRAWINGS

Figure 1 is a graphical representation of the expression of the ratio of phosphorylated SMAD2 (pSMAD2) protein relative to total SMAD2 protein in BAL cells isolated from cynomolgus monkeys after 8-weekly doses of STX-100 treatment relative to circulating levels of STX-100 in serum after the last (8th) weekly dose of antibody (area under the curve (AUC) ug*hr/ml). Data are shown for individual animals in vehicle and 0.1, 0.3, 1.0, 3.0, and 10 mg/kg STX-100 treatment groups. pSMAD2 and total SMAD2 levels were determined by ELISA analysis.
Figure 2A is a graphical representation of the expression of ALOX5 mRNA in BAL cells isolated from cynomolgus monkeys after 8-weekly doses of STX-100 treatment relative to circulating levels of STX-100 in serum after the last (8th) weekly dose of antibody (area under the curve (AUC) ug*hr/ml). Data are shown for individual animals in vehicle and 0.1, 0.3, 1.0, 3.0, and 10 mg/kg STX-100 treatment groups. Gene expression was determined by Taqman® gene expression analysis.
Figure 2B is a graphical representation of the expression of OLR1 mRNA in BAL cells isolated from cynomolgus monkeys after 8-weekly doses of STX-100 treatment relative to circulating levels of STX-100 in serum after the last (8th) weekly dose of antibody (area under the curve (AUC) ug*hr/ml). Data are shown for individual animals in vehicle and 0.1, 0.3, 1.0, 3.0, and 10 mg/kg STX-100 treatment groups. Gene expression was determined by Taqman® gene expression analysis.
Figure 2C is a graphical representation of the expression of Serpine1 mRNA in BAL cells isolated from cynomolgus monkeys after 8-weekly doses of STX-100 treatment relative to circulating levels of STX-100 in serum after the last (8th) weekly dose of antibody (area under the curve (AUC) ug*hr/ml). Data are shown for individual animals in vehicle and 0.1, 0.3, 1.0, 3.0, and 10 mg/kg STX-100 treatment groups. Gene expression was determined by Taqman® gene expression analysis.
Figure 2D is a graphical representation of the expression of TGM2 mRNA in BAL cells isolated from cynomolgus monkeys after 8-weekly doses of STX-100 treatment relative to circulating levels of STX-100 in serum after the last (8th) weekly dose of antibody (area under the curve (AUC) ug*hr/ml). Data are shown for individual animals in vehicle and 0.1, 0.3, 1.0, 3.0, and 10 mg/kg STX-100 treatment groups. Gene expression was determined by Taqman® gene expression analysis.
Figure 3 is a bar graph showing the expression level of Cathepsin L mRNA in mouse BAL macrophage cells following treatment with 3G9.
Figure 4 is a bar graph showing the expression level of Legumain mRNA in mouse BAL macrophage cells following treatment with 3G9.
Figure 5 is a bar graph showing the expression level of PAI-1 (also known as Serpine1) mRNA in mouse BAL macrophage cells following treatment with 3G9.
Figure 6 is a bar graph showing the expression level of Osteopontin mRNA in mouse BAL macrophage cells following treatment with 3G9.
Figure 7 is a bar graph showing the expression level of TREM-1 mRNA in mouse BAL macrophage cells following treatment with 3G9.
Figure 8 is a bar graph showing the expression level of MMP-19 mRNA in mouse BAL macrophage cells following treatment with 3G9.
Figure 9 is a bar graph showing the expression level of ALCAM mRNA in mouse BAL macrophage cells following treatment with 3G9.

### DETAILED DESCRIPTION OF THE INVENTION

The identification of biomarkers that will provide rapid and accessible readouts of efficacy, drug exposure, or clinical response is increasingly important in the clinical development of drug candidates. In the present invention, the inventors have identified specific biomarkers that can be used to tailor therapy with an anti αvβ6-inhibitor such as an anti αvβ6-integrin antibody in the treatment of, for example lung injury. Embodiments of the invention include measuring changes in the expression levels of specific biomarkers that are responsive to treatment with an anti αvβ6-inhibitor such as an αvβ6-integrin antibody. In one aspect, bronchoalveolar lavage samples from subjects that are to be treated with the antibody are used for biomarker analysis.

This invention provides methods for predicting responsiveness to a αvβ6-integrin inhibitor in a subject suffering from a disorder, and methods for selecting a treatment regimen with an inhibitor of αvβ6-integrin, based on expression of particular biomarkers in the subject to be treated. The invention is based, at least in part, on the observation that altered expression of particular biomarkers in a subject suffering from lung fibrosis is associated with increased or decreased responsiveness to therapy with an anti-αvβ6-integrin antibody. Microarray analysis, and other nucleic acid analyses were used to examine normal subjects and subjects suffering from fibrosis, who were categorized as being responsive to treatment with an antibody (responders) or nonresponsive to treatment with an anti-αvβ6-integrin antibody (nonresponders). While the initial determination is made based on lung fibrosis models, the markers may be useful in the treatment of other diseases, including but not limited to fibrosis, psoriasis, sclerosis, cancer, acute lung injury, renal injury, liver injury, scleroderma, transplant or Alports Syndrome, and the like. A list of additional diseases that may be treated include those listed in US Patent US 7,465,449, 7,943,742, 8,153,126, and 7,927,590

A panel of genes were identified whose expression was altered (up-regulated (Table 1) or downregulated (Table 2)) in animals treated with the antibody, demonstrating the ability of these genes to act as biomarkers for predicting responsiveness to αvβ6-integrin inhibitor treatment. In particular, ALOX5, FN1, OLR1, SERPINE1, TGM2, TREM1, ENPP1, IGSF2, and GPR82 which are each down-regulated by administration of an anti-αvβ6 integrin specific antibody were identified as particularly useful in predicting the future response to αvβ6-integrin treatment. Accordingly, in specific embodiments, the expression pattern of one or more biomarkers which particularly include one or more, two, three, four, five, six, seven, eight, or nine of the above 9 genes (e.g., ALOX5, FN1, OLR1, SERPINE1, TGM2, and TREM1) can be assessed in subjects for which αvβ6-inhibitor therapy is being considered, or subjects suffering from other disorders amenable to modulation with αvβ6-integrin inhibition therapy, to thereby predict responsiveness of the subject to such therapy and/or to aid in the selection of an appropriate treatment regimen.

As used herein, the term "treatment regimen" is intended to refer to one or more parameters selected for the treatment of a subject, e.g., with a αvβ6 integrin inhibitor, which parameters can include, but are not necessarily limited to, the type of agent chosen for administration, the dosage, the formulation, the route of administration and the frequency of administration.

Using such tissue, cell, or fluid samples to assess gene expression before and after treatment with the anti-αvβ6-integrin antibody or small molecule inhibitor therapy, the inventors identified specific biomarkers that respond to anti-αvβ6-integrin therapy or small molecule inhibitor of -αvβ6-integrin activity. These biomarkers can be employed for predicting response to one or more αvβ6-integrin modulators and indeed for modulating the effects of modulators of the TGFβ signaling pathway. In one aspect, the biomarkers of the invention are those provided in Table 1 and Table 2 and the Sequence Listing, including both polynucleotide and polypeptide sequences. Also described are nucleotide sequences that hybridize to the polynucleotides provided in Table 1 and Table 2. The biomarkers in Table 1 are those that were found to be up-regulated, or increased in response to administration of an anti-αvβ6-integrin antibody, and the biomarkers shown in Table 2 are downregulated or decreased in response to administration of an αvβ6-antibody.

The biomarkers have expression levels in cells that are highly correlated with sensitivity to anti-αvβ6-antibody exhibited by the cells. Hence, these biomarkers serve as useful molecular tools for predicting the likelihood of a response to inhibition of αvβ6-integrin activity, preferably with anti-αvβ6-integrin antibodies but may also be predictive of efficacy of small molecule inhibitors of αvβ6-integrin activity. As αvβ6-integrin activity has been shown to influence TGFβ signaling pathway, the biomarkers identified herein also will be useful in predicting efficacy of modulators of the TGFβ signaling pathway.

Furthermore, the biomarker expression patterns described herein also can be used in monitoring a disorder in a subject, e.g., monitoring the responsiveness of the subject to a particular therapy or assisting in the diagnosis or prognosis of the disorder (e.g., fibrosis) in the subject.

The term "predicting responsiveness to a αvβ6 integrin inhibitor", as used herein, is intended to refer to an ability to assess the likelihood that treatment of a subject with a αvβ6 integrin inhibitor will or will not be effective in (e.g., provide a measurable benefit to) the subject. In particular, such an ability to assess the likelihood that treatment will or will not be effective typically is exercised before treatment with the αvβ6 integrin inhibitor is begun in the subject. However, it is also possible that such an ability to assess the likelihood that treatment will or will not be effective can be exercised after treatment has begun but before an indicator of effectiveness (e.g., an indicator of measurable benefit) has been observed in the subject. Thus, genes identified herein in Table 1 and Table 2 and their alterations (i.e., up-regulation or down-regulation) in response to such therapy will be useful as surrogate biomarkers for clinical efficacy of such therapy.

The term "αvβ6 integrin inhibitor" as used herein is intended to encompass agents including proteins, antibodies, antibody fragments, fusion proteins (e.g., Ig fusion proteins or Fc fusion proteins), small molecule αvβ6 integrin antagonists and similar naturally- or nonnaturally-occurring molecules, and/or recombinant and/or engineered forms thereof, that, directly or indirectly, inhibit αvβ6 integrin activity, such as by inhibiting interaction of αvβ6 integrin with a cell surface receptor for αvβ6 integrin, inhibiting αvβ6 integrin protein production, inhibiting αvβ6 integrin gene expression, inhibiting αvβ6 integrin secretion from cells, inhibiting αvβ6 integrin receptor signaling or any other means resulting in decreased αvβ6 integrin activity in a subject. The term "αvβ6 integrin inhibitor" also includes agents which interfere with αvβ6 integrin activity or expression. For example, particular αvβ6 integrin inhibitors may include nucleic acid or chemical based inhibitors of expression, such as for example, RNAi molecules, siRNA molecules, micro-RNA molecules (e.g., inhibitors of RNAs and mimetics of microRNA), as well as longer antisense nucleic acid molecules. Examples of αvβ6 integrin inhibitors include the antibodies described and disclosed in e.g., US Patent No. 7,465,449, 7,943,742, 8,153,126,, and 7,927,590 each incorporated herein by reference in its entirety for the disclosure therein of specific antibodies and variants thereof, modulators of αvβ6 integrin activity, and related methods of production.

The term "antibody" as referred to herein includes whole antibodies and any antigen binding fragment (i.e., "antigen-binding portion") or single chains thereof. An "antibody" refers to a glycoprotein comprising at least two heavy (H) chains and two light (L) chains inter-connected by disulfide bonds, or an antigen binding portion thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. Preferred sequences for the CDRs of the antibodies for use in the present invention include those described in US Patent No. 7,465,449. For example, the CDR sequences are as follows:

**TABLE A**

| **Antibody** | **Amino Acid Sequence** | **SEQ ID NO** |
|---|---|---|
| Heavy Chain CDR1 | | |
| 8G6 | SYTFTDYAMH | 1176 |
| 1A8 | SYTFTDYTMH | 1177 |
| 2BI | GFTFSRYVMS | 1178 |
| 3G9 | GFTFSRYVMS | 1178 |
| 2AI | GYDFNNDLIE | 1180 |
| 2G2 | GYAFTNYLIE | 1181 |

| Heavy Chain CDR2 | | |
|---|---|---|
| 8G6 | VISTYYGNTNYNQKFKG | 1182 |
| 1A8 | VIDTYYGKTNYNQKFEG | 1183 |
| 2BI | SISSG-GSTYYPDSVKG | 1184 |
| 3G9 | SISSG-GRMYYPDTVKG | 1185 |
| 2AI | VINPGSGRTNYNEKFKG | 1186 |
| 2G2 | VISPGSGHNYNEKFKG | 1187 |
| | | |

| Heavy Chain CDR3 | | |
|---|---|---|
| 8G6 | GGLRRGDRPSLRYAMDY | 1188 |
| 1A8 | GGFRRGDRPSLRYAMDS | 1189 |
| 2BI | GAIYDG ----- YYVFAY | 1190 |
| 3G9 | GSIYDG ----- YYVFPY | 1191 |
| 2AI | IYYGPH ----- SYAMDY | 1192 |
| 2G2 | ID-YSG-PYAVDD | 1193 |

| Light Chain CDR 1 | | |
|---|---|---|
| 8G6 | RASQSVSTSS-YSYMY | 1194 |
| 1A8 | RASQSVSIST-YSYIH | 1195 |
| 2BI | SASSSVSSS ----- YLY | 1196 |
| 3G9 | SANSSVSSS ----- YLY | 1197 |
| 2AI | KASLDVRTAVA | 1198 |
| 2G2 | KASQAVNTAVA | 1199 |

| Light Chain CDR 2 | | |
|---|---|---|
| 8G6 | YASNLES | 1200 |
| 1A8 | YASNLES | 1200 |
| 2BI | STSNLAS | 1202 |
| 3G9 | STSNLAS | 1202 |
| 2AI | SASYRYT | 1179 |
| 2G2 | SASYQYT | 1201 |

| Light Chain CDR 3 | | |
|---|---|---|
| 8G6 | QHNWEIPFT | 1203 |
| 1A8 | QHSWEIPYT | 1204 |
| 2BI | HQWSSYPPT | 1205 |
| 3G9 | HQWSTYPPT | 1206 |
| 2AI | QQHYGIPWT | 1207 |
| 2G2 | QHHYGVPWT | 1208 |

In the above sequences, for the 8G6 heavy chain CDR 3 sequence, the sequence also may be modified in that the "R" in position 12 can for example be Q, such that the sequence is: GGLRRGDRPSLQYAMDY (SEQ ID NO:1209). Further it may be desirable in certain embodiments that the light chain CDR 1 sequence of the 3G9 antibody be modified to SASSSVSSSYLY (SEQ ID NO:1196).

The term "antigen-binding portion" of an antibody (or simply "antibody portion"), as used herein, refers to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab') 2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv). Such single chain antibodies are also intended to be encompassed within the term "antigen-binding portion" of an antibody. Also encompassed within the term "antigen-binding portion" of an antibody are sc(Fv)2 and diabodies. These antibody fragments are obtained using conventional techniques known to those with skill in the art, and the fragments are screened for utility in the same manner as are intact antibodies.

The terms "monoclonal antibody" or "monoclonal antibody composition" as used herein refer to a preparation of antibody molecules of single molecular composition. A monoclonal antibody composition displays a single binding specificity and affinity for a particular epitope.

The terms "chimeric antibody" or "chimeric monoclonal antibody" are intended to refer to antibodies in which the variable region sequences are derived from one species and the constant region sequences are derived from another species, such as an antibody in which the variable region sequences are derived from a mouse antibody and the constant region sequences are derived from a human antibody. Such "chimeric antibodies" can be prepared by standard recombinant technology well established in the art. For example, a nucleic acid encoding a VH region from a mouse antibody can be operatively linked to a nucleic acid encoding the heavy chain constant regions from a human antibody and, likewise, a nucleic acid encoding a VL region from a mouse antibody can be operatively linked to a nucleic acid encoding the light chain constant region from a human antibody.

The terms "humanized antibody" or "humanized monoclonal antibody" are intended to refer to antibodies in which CDR sequences derived from the germline of a non-human mammalian species, such as a mouse, have been grafted onto human framework sequences. Additional framework region modifications may be made within the human framework sequences. Such "humanized antibodies" can be prepared by standard recombinant technology well established in the art. For example, nucleic acids encoding the CDR1, CD2 and CDR3 regions from a VH region of a mouse antibody can be operatively linked to nucleic acids encoding the FR1, FR2, FR3 and FR4 regions of a human VH region, and the entire "CDR-grafted" VH region can be operatively linked to nucleic acid encoding the heavy chain constant regions from a human antibody. Likewise, nucleic acids encoding the CDR1, CD2 and CDR3 regions from a VL region of a mouse antibody can be operatively linked to nucleic acids encoding the FR1, FR2, FR3 and FR4 regions of a human VL region, and the entire "CDR-grafted" VL region can be operatively linked to nucleic acid encoding the light chain constant region from a human antibody. Preferred humanized antibodies for use in the present invention are described in US Patent 7,943,742. Preferably, the humanized antibody used in the methods of the invention is one that comprises the heavy and light chain CDRs 1, 2, and 3 from murine antibody 3G9. More preferably, the light chain CDR1 of murine antibody 3G9 is employed in the humanized antibody, wherein the humanized 3G9 antibody contains a light chain variable domain wherein the CDR1 region contains an asparagine (N) to serine (S) substitution at residue 3 of CDR 1 of the light chain (SEQ ID NO:1197 showing wild-type sequence of light chain CDR 1, whereas the humanized sequence would be: SASSSVSSSYLY (SEQ ID NO:1196). An exemplary such humanized antibody (referred to herein as STX-100) for use in the present invention is an antibody that has a heavy chain sequence of: 
EVQLVESGGGLVQPGGSLRLSCAASGFTFSRYWMSWVRQAPGK
GLEWVASISSGGRMYYPDTVKGRFTISRDNAKNSLYLQMNSLRAED
TAVYYCARGSIYDGYYVFPYWGQGTLVTVSS (SEQ ID NO:1210)
 and a light chain sequence of: 
EIVLTQSPATLSLSPGERATLSCSASSSVSSSYLYWYQQKPGQAPRL
LIY
STSNLASGIPARFSGSGSGTDFTLTISSLEPEDFAVYYCHQWSTYPPT
FG
GGTKVEIK (SEQ ID NO:1211)
 The biomarker profiles identified herein also may be used to identify mammals that will be responsive to small molecule inhibitors of αvβ6-integrin. It should be understood that small molecules can have any molecular weight. They are merely called small molecules because they typically have molecular weights less than 450 daltons. Small molecules include compounds that are found in nature as well as synthetic compounds. In one embodiment, the αvβ6-integrin -modulator or modulator of TGFβ signaling (see e.g., Akhurst, Curr. Opin. Investig.Drugs, 7(6):513-21(2006); Hawinkels, Growth Factors, 29(4): 140-52 (2011) is a small molecule that inhibits the growth of tumor cells that express αvβ6-integrin. In another embodiment, the small molecule is one that inhibits the growth of refractory tumor cells that express αvβ6-integrin.

As used herein, the term "biomarker" is intended to encompass a substance that is used as an indicator of a biologic state and includes genes (and nucleotide sequences of such genes), mRNAs (and nucleotide sequences of such mRNAs) and proteins (and amino acid sequences of such proteins) and post-translationally modified forms of proteins (i.e. phosphorylated and non-phosphorylated forms). A "biomarker expression pattern" is intended to refer to a quantitative or qualitative summary of the expression of one or more biomarkers in a subject, such as in comparison to a standard or a control.

The terms "increased" or "increased expression" and "decreased" or "decreased expression", with respect to the expression pattern of a biomarker(s), are used herein as meaning that the level of expression is increased or decreased relative to a constant basal level of expression of a household, or housekeeping, gene, whose expression level does not significantly vary under different conditions. A non-limiting example of such a household, or housekeeping, gene is GAPDH. Other suitable household, or housekeeping, genes are well-established in the art.

The invention includes individual biomarkers and biomarker sets having both diagnostic and prognostic value in disease areas which are amenable to treatment with an agent that inhibits αvβ6-integrin activity, an anti-αvβ6-integrin antibody or a modulator of TGFβ signaling. The biomarker sets comprise a plurality of biomarkers such as, for example, a plurality of the biomarkers provided in Table 1 and Table 2 that are highly correlated with sensitivity or efficacy to one or more αvβ6-integrin modulators, such as αvβ6-integrin -specific antibodies.

The biomarkers and biomarker sets of the invention can be used to predict or provide a prognosis of the likely effect of one or more αvβ6-integrin modulators in different biological systems or for cellular responses. The biomarkers and biomarker sets can be used in in vitro assays of αvβ6 antibodies response by test cells to predict in vivo outcome. In accordance with the invention, the various biomarkers and biomarker sets described herein, or the combination of these biomarker sets with other biomarkers or markers, can be used, for example, to predict how patients with an αvβ6-integrin related disease might respond to therapeutic intervention with one or more αvβ6-integrin -specific antibodies.

A biomarker and biomarker set of cellular gene expression patterns correlating with sensitivity or resistance of cells following exposure of the cells to one or more αvβ6-specific antibodies provides a useful tool for screening one or more tissue or cell samples from a subject before treatment with the αvβ6-integrin specific antibodies. The screening allows a prediction of cells of a patient's sample exposed to one or more αvβ6-integrin -specific antibodies, based on the expression results of the biomarker and biomarker set, as to whether or not the biological sample, and hence a patient harboring a disease such as, e.g., fibrosis (e.g., IPF), psoriasis, sclerosis, cancer, acute lung injury, liver injury, scleroderma, transplant, or Alports Syndrome, will or will not respond to treatment with the αvβ6-integrin -specific antibodies.

The biomarker or biomarker set can also be used to monitor the progress of disease treatment or therapy in those patients undergoing treatment for a disease involving an αvβ6-integrin -specific antibody.

The biomarkers also serve as targets for the development of therapies for disease treatment. Such targets may be particularly applicable to treatment of lung fibrosis. Indeed, because these biomarkers are differentially expressed in samples that are sensitive and resistant to therapy, the expression patterns of these biomarkers are correlated with relative intrinsic sensitivity of cells to treatment with αvβ6-integrin -specific antibodies.

The level of biomarker protein and/or mRNA can be determined using methods well known to those skilled in the art. For example, quantification of protein can be carried out using methods such as ELISA, 2-dimensional SDS PAGE, Western blot, immunopreciptation, immunohistochemistry, fluorescence activated cell sorting (FACS), or flow cytometry. Quantification of mRNA can be carried out using methods such as PCR, array hybridization, sequencing, Northern blot, in-situ hybridization, dot-blot, Taqman, or RNAse protection assay.

**Microarrays.** Also described are specialized microarrays, e.g., oligonucleotide microarrays or cDNA microarrays, comprising one or more biomarkers of Table 1 and Table 2, showing expression profiles that correlate with increased or decreased expression in response to αvβ6-integrin-specific antibodies. Such microarrays can be employed in in vitro assays for assessing the expression level of the biomarkers in the test cells from patients before and after treatment, and determining whether the expression of the biomarkers has been changed as a result of the treatment such that where expression of the biomarkers in Table 1 is increased and/or where there is a decrease in the expression of the biomarkers of Table 2 there is an indication of therapeutic efficacy of the antibody in the subject from whom the sample is isolated.

For example, a specialized microarray can be prepared using all the biomarkers, or subsets thereof (e.g., ALOX5, FN1, OLR1, PAI-1, TGM2, TREM1), as described herein and shown in Table 1 and Table 2. Cells from a tissue or organ biopsy can be isolated and exposed to one or more of αvβ6-integrin -specific antibodies. In one aspect, following application of nucleic acids isolated from both untreated and treated cells to one or more of the specialized microarrays, the pattern of gene expression of the tested cells can be determined and compared with that of the biomarker pattern from the control panel of cells used to create the biomarker set on the microarray. Based upon the gene expression pattern results from the cells that underwent testing, it can be determined if the biological sample is taken from a subject that will be responsive to the therapy using that profile of gene expression. Whether or not the tested cells from a tissue or organ biopsy will respond to one or more of the αvβ6-integrin -specific antibodies and the course of treatment or therapy can then be determined or evaluated based on the information gleaned from the results of the specialized microarray analysis.

***Antibodies.*** The invention also includes antibodies, including polyclonal or monoclonal, directed against one or more of the polypeptide biomarkers. Such antibodies can be used in a variety of ways, for example, to purify, detect, and target the biomarkers of the invention, including both in vitro and in vivo diagnostic, detection, screening, and/or therapeutic methods.

**Kits.** Also described are kits for determining or predicting whether a patient would be susceptible or resistant to a treatment that comprises one or more αvβ6-integrin -specific antibodies. The patient may have a disorder such as, for example, fibrosis, psoriasis, sclerosis, cancer, acute lung injury, renal injury, liver injury, scleroderma, transplant, or Alports Syndrome. Such kits would be useful in a clinical setting for use in testing a patient's biopsied samples, for example, to determine or predict if the patient will be resistant or sensitive to a given treatment or therapy with αvβ6-integrin -specific antibodies. The kit comprises a suitable container that comprises: one or more microarrays, e.g., oligonucleotide microarrays or cDNA microarrays, that comprise those biomarkers that correlate with increased or decreased expression in response to αvβ6-integrin -specific antibodies; one or more αvβ6-integrin -specific antibodies for use in testing cells from patient tissue specimens or patient samples; and instructions for use. In addition, kits can further include, for example, reagents or materials for monitoring the expression of biomarkers of the invention at the level of mRNA or protein, using other techniques and systems practiced in the art such as, for example, RT-PCR assays, which employ primers designed on the basis of one or more of the biomarkers described herein, immunoassays, such as enzyme linked immunosorbent assays (ELISAs), immunoblotting, e.g., Western blots, or in situ hybridization, mRNA sequencing, and the like.

**Application of Biomarkers and Biomarker Sets.** The biomarkers and biomarker sets may be used in different applications. A "biomarker set" can be built from any combination of biomarkers listed in Table 1 and/or Table 2 and used to make predictions about the effect of αvβ6-integrin -specific antibodies in different biological systems, for efficacy and responsiveness of lung injury to such antibodies. The various biomarkers and biomarkers sets described herein can be used, for example, as diagnostic or prognostic indicators in lung disease management, to predict how patients with such diseases might respond to therapeutic intervention with αvβ6-integrin -specific antibodies, and to predict how patients might respond to therapeutic intervention that modulates signaling through the entire TGFβ pathway.

The biomarkers have both diagnostic and prognostic value in diseases areas in which signaling through TGFβ is of importance.

Herein described is a method for predicting responsiveness to a αvβ6 integrin inhibitor in a subject having disease that would be amenable to treatment with the an anti-αvβ6 integrin antibody. Typically, the method comprises (i) assaying the subject for the expression of one or more biomarkers predictive of responsiveness to a αvβ6 integrin inhibitor in a disorder, and (ii) predicting responsiveness of the subject to the αvβ6 integrin inhibitor based on expression of the one or more biomarkers in the subject. As used herein, the term "one or more biomarkers" is intended to mean that at least one biomarker in a disclosed list of biomarkers is assayed and, in various embodiments, more than one biomarker set forth in the list may be assayed, such as two, three, four, five, ten, twenty, thirty, forty, fifty, more than fifty, or all the biomarkers in the list may be assayed. Further the diagnostic methods of the invention may be combined with assays that determine the phosphorylation status of SMAD2. Such assays would involve determination of SMAD2 phosphorylation status in response to administration of an αvβ6 integrin inhibitor wherein phosphorylation of SMAD2 is indicative of TGFβ activation and a decrease in that phosphorylation status in response to administration of an αvβ6 integrin inhibitor is indicative of inhibition of TGFβ activity. The methods described herein can further be combined with assays that determine the expression level (e.g., mRNA, protein) in peripheral blood or BAL of one or more (e.g., one, two, three, four, five, six, seven) serum biomarker such as, but not limited to, tissue remodeling markers (e.g., metalloproteinase 7 (MMP-7), osteopontin (OPN)); TGF-β inducible proteins (e.g., tissue inhibitor of metalloproteinase 1 (TIMP-1), collagen type 1alpha1 (Col1A1)); and epithelial injury markers (e.g., surfactant A (SP-A), alpha defensins (DEFA1-3)). Blood samples for serum biomarkers can be collected prior to (baseline) and after administration (post-treatment) of the αvβ6 inhibitor (e.g., STX-100). In one embodiment, differential expression of the serum biomarkers can be measured as the difference from baseline to post-treatment value for serum biomarkers compared between αvβ6 inhibitor treated patients and placebo treated patients. A decrease in the expression of these biomarkers post-treatment is indicative that the subject will, or is highly likely to, respond to the αvβ6 inhibitor (e.g., STX-100) therapy or anti-TGFβ treatment.

In one aspect, cells or fluid from a patient, from bronchoalveolar lavage, or tissue or even a tissue biopsy, can be assayed to determine the expression pattern of one or more biomarkers prior to treatment with one or more αvβ6-integrin - specific antibodies, small molecule inhibitors of αvβ6-integrin activity, or indeed modulators of TGFβ signaling. As described herein the disease is for example, fibrosis (e.g., IPF), psoriasis, sclerosis, cancer (e.g., a pancreatic cancer, a lung cancer, a breast cancer, a colorectal cancer, a head and neck cancer, an esophageal cancer, a skin cancer, and an endometrial cancer), acute lung injury, renal injury, liver injury, scleroderma, transplant, or Alports Syndrome. Success or failure of a treatment can be determined based on the biomarker expression pattern of the sample from the test tissue (test cells or fluid), e.g., sample from a bronchoalveolar lavage, as being relatively similar or different from the expression pattern of a control set of the one or more biomarkers. Thus, if the test sample shows a biomarker expression profile which corresponds to that of the biomarkers in the control panel but which are increased or decreased as a result of the treatment with αvβ6-integrin specific antibody or small molecule inhibitor of αvβ6-integrin activity, it is highly likely or predicted that the individual's disease will respond favorably to treatment with the αvβ6-integrin -specific antibodies, thereby allowing the clinician to identify the subjects that are likely to be responders to the therapeutic regiment. By contrast, if the test cells show a biomarker expression pattern in which the biomarkers of Table 1 are not increased or the biomarkers or Table 2 are not decreased in response to the αvβ6-integrin antibody or small molecule inhibitor of αvβ6-integrin activity, it is highly likely or predicted that the individual will not respond to treatment with agents that are TGFβ modulators, anti-αvβ6-integrin antibodies, or small molecules that inhibit the activity of αvβ6-integrin.

Also described herein is a method of monitoring the treatment of a patient having a disease treatable by one or more αvβ6-integrin antibodies, a small molecule inhibitor of αvβ6-integrin activity or a TGFβ modulator. The isolated test cells from the patient's tissue sample, e.g., tissue or cell sample from any of the disease states mentioned herein and in specific embodiments, e.g., a BAL sample, can be assayed to determine the expression pattern of one or more biomarkers before and after exposure to the αvβ6-integrin inhibiting agent wherein, preferably, the agent is an anti-αvβ6-integrin antibody. The resulting biomarker expression profile of the test sample before and after treatment is compared with that of one or more biomarkers as described in Table 1 and whose expression is shown herein to be up-regulated in response to treatment with an anti-αvβ6-integrin antibody, and/or one or more biomarkers of Table 2 whose expression is shown to be down-regulated in response to treatment with an anti-αvβ6-integrin antibody. Thus, if a patient's response is sensitive to treatment by an anti-αvβ6-integrin antibody, based on correlation of the expression profile of the one or biomarkers up-regulated (i.e., biomarkers in Table 1) or down-regulated (i.e., biomarkers in Table 2), the patient's treatment prognosis can be qualified as favorable and treatment can continue. Also, if, after treatment with an anti-αvβ6-integrin antibody, the test sample fails to show a change in the biomarker expression profile as compared to the expression profile prior to treatment, it can serve as an indicator that the current treatment should be modified, changed, or even discontinued. This monitoring process can indicate success or failure of a patient's treatment with an anti-αvβ6-integrin antibody and such monitoring processes can be repeated as necessary or desired.

Additionally or alternatively, in certain situations it may be possible to assay for the expression of one or more biomarkers at the protein level or the post-translationally modified form of a protein (e.g. phosphorylation or non-phosphorylation), using a detection reagent that detects the protein product or phosphorylated form of a protein encoded by the mRNA of the biomarker(s). For example, if an antibody reagent is available that binds specifically to the biomarker protein product or a phosphorylated form of a protein product to be detected, and not to other proteins, then such an antibody reagent can be used to detect the expression of the biomarker of interest in a cellular, tissue, or fluid sample from the subject, or a preparation derived from the test sample, using standard antibody-based techniques known in the art, such as FACS analysis, ELISA, mass-spectrometry and the like.

As used herein, the term "subject" includes humans, and non-human animals amenable to αvβ6 integrin inhibitor therapy, e.g., preferably mammals, such as non-human primates, sheep, dogs, cats, horses and cows.

Given the observation that the expression pattern of particular biomarkers is associated with responsiveness of the subject to a αvβ6 integrin inhibitor, one can select an appropriate treatment regimen for the subject based on the expression of one or more biomarkers in the subject. Accordingly, in one embodiment, the above-described method for predicting the responsiveness to a αvβ6 integrin inhibitor in a subject further comprises selecting a treatment regimen with the αvβ6 integrin inhibitor based upon expression of the one or more biomarkers in the subject

Described herein is a method for selecting a treatment regimen for therapy with a αvβ6 integrin inhibitor in a subject, the method comprising:
assaying the subject for expression of one or more biomarkers predictive of responsiveness to a αvβ6 integrin inhibitor for treatment of the disorder; and
selecting a treatment regimen with a αvβ6 integrin inhibitor based upon expression of the one or more biomarkers in the subject.

Also described herein is the treatment of a subject having a disorder with a αvβ6 integrin inhibitor (e.g., an αvβ6 antibody or a small molecule inhibitor), the method comprising:
assaying the subject for expression of one or more biomarkers predictive of responsiveness to a αvβ6 integrin inhibitor for treatment of the disorder;
selecting a treatment regimen with a αvβ6 integrin inhibitor based upon expression of the one or more biomarkers in the subject; and
administering the αvβ6 integrin inhibitor according to the treatment regimen such that the subject is treated for the disorder.

The treatment regimen that is selected typically includes at least one of the following parameters and more typically includes many or all of the following parameters: the type of agent chosen for administration, the dosage, the formulation, the route of administration and/or the frequency of administration.

In one embodiment, the αvβ6 integrin inhibitor is an anti-αvβ6 integrin antibody, or antigen-binding portion thereof. For example, the anti-αvβ6 integrin antibody, or antigen-binding portion thereof, can be a humanized antibody, a chimeric antibody or a multivalent antibody.

It is well known in the art that antibody heavy and light chain CDR3 domains play an important role in the binding specificity/affinity of an antibody for an antigen. Accordingly, in another aspect, the αvβ6 integrin inhibitor used in the treatment is a human anti-αvβ6 integrin antibody that has slow dissociation kinetics for association with αvβ6 integrin and that has light and heavy chain CDR3 domains that structurally are identical to or related to those of STX-100 (humanized 3G9) whose sequences are shown herein.

The αvβ6 integrin antibody of the invention can be modified. In some embodiments, the αvβ6 integrin antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factors 3:4-10 (1992); EP 0 154 316; and EP 0 401 384 Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (C1-C10) alkoxy- or aryloxy-polyethylene glycol.

Methods for preparing pegylated antibodies and antibody fragments of the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

In yet another embodiment of the invention, αvβ6 integrin antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see e.g., Canfield, S. M. and S. L. Morrison (1991) J. Exp. Med. 173:1483-1491; and Lund, J. et al. (1991) J. Immunol. 147:2657-2662). Reduction in FcR binding ability of the antibody may also reduce other effector functions which rely on FcR interactions, such as opsonization and phagocytosis and antigen-dependent cellular cytotoxicity.

The biomarkers of the invention will be particularly useful in predicting the responsiveness of diseases mediated by αᵥβ₆. For example, these humanized antibodies can be used to treat fibrosis (e.g., lung fibrosis, acute lung injury, kidney fibrosis, liver fibrosis, Alport's Syndrome, transplant and scleroderma), and other diseases and disorders described elsewhere herein, by blocking the activation of TGF-β through blockade of binding to the latency associated peptide (LAP) portion of TGF-β or blocking the binding of αᵥβ₆ to any other ligands, such as fibronectin, vitronectin, and tenascin. In particular, the humanized antibodies of this invention can be used to treat lung diseases associated with injury/fibrosis such as, but not limited to, idiopathic pulmonary fibrosis, radiation induced fibrosis, flu induced fibrosis, coagulation induced fibrosis, vascular injury induced fibrosis, chronic obstructive pulmonary disease (COPD), scleroderma, bleomycin induced fibrosis, chronic asthma, silicosis, asbestos induced fibrosis, acute lung injury, transplant, and acute respiratory distress, (including bacterial pneumonia induced, trauma induced, viral pneumonia induced, ventilator induced, non-pulmonary sepsis induced and aspiration induced). The biomarkers will also be useful in predicting treatment of chronic nephropathies associated with injury/fibrosis such as, but not limited to, lupus, diabetes, scleroderma, glomerular nephritis, focal segmental glomerular sclerosis, IgA nephropathy, hypertension, allograft, Alport's disease, and acute kidney injury. The humanized antibodies may also be useful to treat gut fibrosis, scleroderma, radiation-induced fibrosis. In addition, the biomarkers may be used for determining the responsiveness and predicting therapy for liver fibrosis such as, but not limited to, biliary duct injury induced fibrosis. Other indications include head and neck fibrosis, radiation induced fibrosis, corneal scarring, LASIX, corneal transplant, trabeculectomy, hypertrophic scarring, burn induced fibrosis, surgical fibrosis, sarcoidosis, psoriasis and spinal cord injury/fibrosis.

In addition to fibrotic diseases and conditions, the biomarkers may also be useful for predicting responsiveness of a subject with cancer or cancer metastasis (including tumor growth and invasion), particularly epithelial cancers, to therapy with an anti-αvβ6 or a small molecule inhibitor of αvβ6. A subset of epithelial cancers is squamous cell carcinoma, e.g., head and neck (including oral, laryngeal, pharyngeal, esophageal), breast, lung, prostate, cervical, colon, pancreatic, skin (basal cell carcinomas), prostate, and ovarian cancers. Studies have shown that αᵥβ₆ is highly expressed in many epithelial cancers, especially on the leading edge of the tumors. The biomarkers of the invention can be used to assess whether such cancers are responsive to the αᵥβ₆ therapies. The biomarkers also could be used for predicting responsiveness of a subject with psoriasis to therapy with an anti-αvβ6 or a small molecule inhibitor of αvβ6.

### EXAMPLES

### Example 1: Experimental Determination of Gene Expression Profiles and Phosphorylated SMAD2 Levels in BAL Cells from an 8 Week Study of Subcutaneous Injection of STX-100 (humanized 3G9 antibody) in Cynomolgus Monkeys.

The TGF-β cytokine is central to the initiation and maintenance of fibrosis, a pathological process that is marked by the replacement of diseased tissue with excess extracellular matrix (ECM) and ultimately leads to organ scarring and failure. TGF-β promotes fibroblast proliferation and activation, leading to excess secretion of ECM and progression of the fibrotic process. TGF-β plays a well-regulated role in tissue remodeling events that take place during wound healing; however, in many diseases the process of tissue remodeling becomes aberrant and is characterized by prolonged upregulated TGF-β signaling, excess fibroblast accumulation, ECM deposition, and scarring. Elevated expression of TGF-β is a hallmark of fibrotic human tissues, and the functional importance of TGF-β in promoting tissue fibrosis has been documented *in vitro* and *in vivo* in animal disease models. Overexpression of TGF-β is sufficient to induce fibroblast activation and angiogenesis *in vivo* and to activate excessive production of ECM in organotypic and cell cultures. Conversely, genetic or pharmacological disruption of the TGF-β pathway protects from fibrosis in models of tissue fibrosis. Consequently, TGF-β has been identified as a therapeutic target for treatment of diseases associated with the pathology of fibrosis. This includes idiopathic pulmonary fibrosis (IPF), an interstitial lung disease characterized by chronic progressive fibrosis, where there is an increased TGF-β transcriptional signature in affected lung tissue and increased TGF-β protein levels in focal areas of fibrosis.

Monitoring TGF-β activity in bronchoalveolar lavage (BAL) cells is a means to monitor activation TGF-β activity in the lung since these cells can make intimate contact with the epithelium of the lung. BAL cells, which are largely composed of macrophages, show tonic activation of TGF-β activation as monitored by phosphorylated SMAD2 (pSMAD2) levels and the expression of TGF-β regulated genes. For instance, there is no meaningful difference in pSMAD2 levels in BAL cells isolated from wild type mice treated with TGF-β1 or from wild type mice versus bleomycin treated mice. Likewise, there is no meaningful difference in TGF-β regulated genes or pSMAD2 levels in BAL cells from healthy volunteers or IPF patients with or without TGF-β1 treatment.

Thus, evaluating changes in pSMAD2 levels and gene expression in anti-αvβ6 antibody-treated BAL cells from healthy primates provides a measure of monitoring inhibition of the TGF-β pathway. These experiments are described in greater detail below.

Cynomolgus monkeys (2 males/group and 3 females/group) received a bolus subcutaneous injection of 0 (0.9% Sodium Chloride for Injection, USP), 0.1, 0.3, 1, 3, or 10 mg/kg/dose STX-100 once weekly for 8 consecutive weeks. The dose volume was 1 mL/kg/dose for all dose groups. At the end of the treatment period, all animals were euthanized and tissues collected for possible analysis. Animals were bled prestudy and on Days 1, 7, 35, 42, 49, and on Day 50, blood samples were drawn at 12, 24, 36, 48, 72, 96, 120, 144 and 168 hours after the administration of the 8th (last) dose for pharmacokinetic analysis. Animals were also bled pretest and on Days 35, 53 and 57 for RNA isolation and gene expression analysis, and pretest and on Days 7, 35, 53 and 57 for possible serum biomarker and plasma biomarker analysis. Bronchoalveolar lavage was performed pretest and on Days 53 and 57 (termination) for RNA and gene expression analysis as well as total and differential cell counts. Parameters evaluated during the study included: viability, clinical observations and body weights.

All animals survived to termination of the study. Weekly dosing with STX-100 up to 10 mg/kg/week did not result in any effects on animal viability, clinical observations or body weight. There were noticeable variations in the trough concentrations in the 0.1 and 0.3 mg/kg dose groups. In the 0.1 mg/kg dose group, all animals had detectable concentrations of STX-100 on Day 7; however, trough concentrations following subsequent doses diminished to BQL in 3 out of 5 animals. For the 2 animals that had measurable STX-100 level in subsequent trough sampling time points, female Animal No. 8899 showed no serum exposure at trough following the 7th dose and no exposure at all was noted following the 8th dose, whereas, male animal 9281 showed serum exposure of STX-100 through the 8th dose.

Overall, serum exposures of STX-100 appeared to be higher in female than in male monkeys at 0.1, 0.3 and 1 mg/kg, but were similar at 3 and 10 mg/kg. Overall, the serum concentration profile of STX-100 in monkeys (males and females combined) increased with increasing dose.

**BAL Collection:** During the study, Bronchoalveolar lavage fluid (BALF) samples were harvested under anesthesia. Animals were sedated with ketamine (5 to 10 mg/kg IM) and anesthetized with Propofol (7 mg/kg IV). Additional Propofol was administered as needed. The entire procedure took less than 30 minutes per animal. At necropsy, the side of the lung that was last lavaged (right side) was separated and the apical lobe only was lavaged with two washes of 10mL sterile saline.

The BAL procedure was performed by guiding a bronchoscope into the left or right main (1st and 2nd collection respectively) bronchus and advancing it into a terminal bronchus until it became 'wedged'. Two washes of sterile phosphate buffered saline (PBS) (10 mL total) was instilled and aspirated for collection into a cryotube and placed immediately on wet ice. For the pre-test collection only, the bronchoscope was repositioned to the opposite side to the initial BALF collection and the procedure was repeated. Post-dose BAL was performed on one lung lobe only and the lavaged lung side was noted. The total harvested volume was estimated and recorded. All BALF cells were processed and frozen within two hours of BALF collection. Post collection, animals were placed in lateral recumbency on the side opposite to that lavaged. Animals lavaged on both sides were switched from side to side every 5 to 10 minutes to ensure adequate recovery and excess fluid absorption in both lung lobes.

Samples were spun at 500g for 5 minutes at 2 to 8°C. The supernatant was divided into four equal aliquots and frozen at approximately -80°C (±10°C). The cell pellet were resuspended in 10.5 mL of ice cold 1XPBS and then aliquoted into samples for preparing cell lysates to analyze total SMAD2 and phosphorylated SMAD2 levels by ELISA and for preparing total RNA to monitor gene expression by affymetrix gene chip analysis (Affymetrix GeneChip® Human Gene 1.0 ST arrays) or Taqman® gene expression assays.

Table 1 shows the list of genes that showed a statistically significant increase in expression in BAL cells isolated from cynomolgus monkeys after 8-weekly doses of STX-100 treatment relative to vehicle treated control animals. Gene expression was determined by affymetrix gene chip analysis (Affymetrix GeneChip® Human Gene 1.0 ST arrays).

Table 2 shows the list of genes showing a statistically significant decrease in expression in BAL cells isolated from cynomolgus monkeys after 8-weekly doses of STX-100 treatment relative to vehicle treated control animals. Gene expression was determined by affymetrix gene chip analysis (Affymetrix GeneChip® Human Gene 1.0 ST arrays).

**Table 1: Genes Up-regulated Upon STX-100 Administration**

| **gene_assignment** | **Genbank No.** | **Nucleic acid Seqeunce** | **Protein Sequence** |
|---|---|---|---|
| RHOU // ras homolog gene family, member U | NM_021205 | SEQ ID NO:1 | SEQ ID NO:2 |
| ASTN2 // astrotactin 2 | NM_198186 | SEQ ID NO: 3 | SEQ ID NO: 4 |
| // ITM2C // integral membrane protein 2C | NM_030926 | SEQ ID NO: 5 | SEQ ID NO: 6 |
| // PCOLCE2 // procollagen C-endopeptidase enhancer 2 | NM_013363 | SEQ ID NO: 7 | SEQ ID NO: 8 |
| // SLC39A8 // solute carrier family 39 (zinc transporter), member 8 | NM_022154 | SEQ ID NO: 9 | SEQ ID NO: 10 |
| NM_015028 // TNIK // TRAF2 and NCK interacting kinase | NM_015028 | SEQ ID NO: 11 | SEQ ID NO:12 |
| // FUCA1 // fucosidase, alpha-L- 1, tissue | NM_000147 | SEQ ID NO: 13 | SEQ ID NO: 14 |
| NM_ 033104 // STON2 // stonin 2 | NM_033104 | SEQ ID NO: 15 | SEQ ID NO: 16 |
| // GATA3 // GATA binding protein 3 | NM_001002295 | SEQ ID NO: 17 | SEQ ID NO: 18 |
| // GRK5 // G protein-coupled receptor kinase 5 | NM_005308 | SEQ ID NO: 19 | SEQ ID NO: 20 |
| // PLA1A // phospholipase A1 member A | NM_015900 | SEQ ID NO: 21 | SEQ ID NO: 22 |
| // ATP2B4 // ATPase, Ca++ transporting, plasma membrane 4 | NM_001001396 | SEQ ID NO: 23 | SEQ ID NO: 24 |
| // TEC // tec protein tyrosine kinase | NM_003215 | SEQ ID NO: 25 | SEQ ID NO: 26 |
| // LAMC1 // laminin, gamma 1 (formerly LAMB2) | NM_002293 | SEQ ID NO: 27 | SEQ ID NO: 28 |
| // TAGAP // T-cell activation RhoGTPase activating protein | NM_054114 | SEQ ID NO: 29 | SEQ ID NO: 30 |
| // CD3E // CD3e molecule, epsilon (CD3-TCR complex) | NM_000733 | SEQ ID NO: 31 | SEQ ID NO: 32 |
| // BACE2 // beta-site APP-cleaving enzyme 2 | NM_012105 | SEQ ID NO: 33 | SEQ ID NO: 34 |
| // TRERF1 // transcriptional regulating factor 1 | NM_033502 | SEQ ID NO: 35 | SEQ ID NO: 36 |
| // CCL5 // chemokine (C-C motif) ligand 5 | NM_002985 | SEQ ID NO: 37 | SEQ ID NO: 38 |
| // PLA2G2D // phospholipase A2, group IID | NM_012400 | SEQ ID NO: 39 | SEQ ID NO: 40 |
| // S1 PR1 // sphingosine-1-phosphate receptor 1 | NM_001400 | SEQ ID NO: 41 | SEQ ID NO: 42 |
| // PLXNA1 // plexin A1 | NM_032242 | SEQ ID NO: 43 | SEQ ID NO:44 |
| // MTSS1 // metastasis suppressor 1 | NM_014751 | SEQ ID NO: 45 | SEQ ID NO: 46 |
| // SLAMF7 // SLAM family member 7 | NM_021181 | SEQ ID NO: 47 | SEQ ID NO: 48 |
| // C11 orf49 // chromosome 11 open reading frame 49 | NM_001003676 | SEQ ID NO: 49 | SEQ ID NO: 50 |
| // CCDC103 // coiled-coil domain containing 103 | NM_213607 | SEQ ID NO: 51 | SEQ ID NO: 52 |
| // PTPN22 // protein tyrosine phosphatase, non-receptor type 22 (lymphoid) | NM_015967 | SEQ ID NO: 53 | SEQ ID NO: 54 |
| // PIM2 // pim-2 oncogene | NM_006875 | SEQ ID NO: 55 | SEQ ID NO: 56 |
| // SLAMF8 // SLAM family member 8 | NM_020125 | SEQ ID NO: 57 | SEQ ID NO: 58 |
| // IQGAP2 // IQ motif containing GTPase activating protein 2 | NM_006633 | SEQ ID NO: 59 | SEQ ID NO: 60 |
| // CHST15 // carbohydrate (N-acetylgalactosamine 4-sulfate 6-O) sulfotransferase 15 | NM_015892 | SEQ ID NO: 61 | SEQ ID NO: 62 |
| // MAP2K6 // mitogen-activated protein kinase kinase 6 | NM_002758 | SEQ ID NO: 63 | SEQ ID NO: 64 |
| // ALOX15 // arachidonate 15-lipoxygenase | NM_001140 | SEQ ID NO: 65 | SEQ ID NO:66 |
| NM_ 020859 // SHROOM3 // shroom family member 3 | NM_020859 | SEQ ID NO: 67 | SEQ ID NO:68 |
| // DNM1 // dynamin 1 | NM_004408 | SEQ ID NO: 69 | SEQ ID NO: 70 |
| // NT5DC2 // 5'-nucleotidase domain containing 2 | NM_022908 | SEQ ID NO: 71 | SEQ ID NO:72 |
| // IFITM1 // interferon induced transmembrane protein 1 (9-27) | NM_003641 | SEQ ID NO: 73 | SEQ ID NO: 74 |
| // E2F5 // E2F transcription factor 5, p130-bindinq | NM_001951 | SEQ ID NO: 75 | SEQ ID NO: 76 |
| // AES // amino-terminal enhancer of split | NM_198969 | SEQ ID NO: 77 | SEQ ID NO: 78 |
| // USP2 // ubiquitin specific peptidase 2 | NM_004205 | SEQ ID NO: 79 | SEQ ID NO: 80 |
| // CD8A // CD8a molecule | NR_027353 | SEQ ID NO: 81 | N/A |
| // MYO1E // myosin IE | NM_004998 | SEQ ID NO: 82 | SEQ ID NO: 83 |
| // KREMEN1 // kringle containing transmembrane protein 1 | NM_001039570 | SEQ ID NO: 84 | SEQ ID NO: 85 |
| // VLDLR // very low density lipoprotein receptor | NM_003383 | SEQ ID NO: 86 | SEQ ID NO: 87 |
| // TIAM1 // T-cell lymphoma invasion and metastasis 1 | NM_003253 | SEQ ID NO: 88 | SEQ ID NO: 89 |
| // ABLIM1 // actin binding LIM protein 1 | NM_002313 | SEQ ID NO: 90 | SEQ ID NO: 91 |
| // TSPAN4 // tetraspanin 4 | NM_001025237 | SEQ ID NO: 92 | SEQ ID NO: 93 |
| // PLTP // phospholipid transfer protein | NM_006227 | SEQ ID NO: 94 | SEQ ID NO: 95 |
| // PSCA // prostate stem cell antigen | NM_005672 | SEQ ID NO: 96 | SEQ ID NO: 97 |
| // GRAP2 // GRB2-related adaptor protein 2 | NM_004810 | SEQ ID NO: 98 | SEQ ID NO: 99 |
| // P2RY10 // purinergic receptor P2Y, G-protein coupled, 10 | NM_014499 | SEQ ID NO: 100 | SEQ ID NO:101 |
| // MAP1A // microtubule-associated protein 1A | NM_002373 | SEQ ID NO: 102 | SEQ ID NO: 103 |
| // THEMIS // thymocyte selection associated | NM_001010923 | SEQ ID NO: 104 | SEQ ID NO: 105 |
| // IL18RAP // interleukin 18 receptor accessory protein | NM_003853 | SEQ ID NO: 106 | SEQ ID NO: 107 |
| // CHN2 // chimerin (chimaerin) 2 | NM_004067 | SEQ ID NO: 108 | SEQ ID NO: 109 |
| // PPM1E // protein phosphatase 1E (PP2C domain containing) | NM_014906 | SEQ ID NO: 110 | SEQ ID NO:111 |
| // TMEM154 // transmembrane protein 154 | NM_152680 | SEQ ID NO: 112 | SEQ ID NO: 113 |
| // PLBD1 // phospholipase B domain containing 1 | NM_024829 | SEQ ID NO: 114 | SEQ ID NO: 115 |
| // SCN1 B // sodium channel, voltage-gated, type I, beta | NM_001037 | SEQ ID NO: 116 | SEQ ID NO: 117 |
| // UST // uronyl-2-sulfotransferase | NM_005715 | SEQ ID NO: 118 | SEQ ID NO: 119 |
| // IL23R // interleukin 23 receptor | NM_144701 | SEQ ID NO: 120 | SEQ ID NO: 121 |
| // KLRK1 // killer cell lectin-like receptor subfamily K, member 1 | NM_007360 | SEQ ID NO: 122 | SEQ ID NO: 123 |
| // NFATC2 // nuclear factor of activated T-cells, cytoplasmic, calcineurin-dependent 2 | NM_012340 | SEQ ID NO: 124 | SEQ ID NO: 125 |
| // FYB // FYN binding protein (FYB-120/130) | NM_001465 | SEQ ID NO: 126 | SEQ ID NO: 127 |
| // KMO // kynurenine 3-monooxygenase (kynurenine 3-hydroxylase) | NM_003679 | SEQ ID NO: 128 | SEQ ID NO: 129 |
| // MCOLN2 // mucolipin 2 | NM_153259 | SEQ ID NO: 130 | SEQ ID NO: 131 |
| // TMEM37 // transmembrane protein 37 | NM_183240 | SEQ ID NO: 132 | SEQ ID NO: 133 |
| // STARD13 // StAR-related lipid transfer (START) domain containing 13 | NM_178006 | SEQ ID NO: 134 | SEQ ID NO: 135 |
| // CD80 // CD80 molecule | NM_005191 | SEQ ID NO: 136 | SEQ ID NO: 137 |
| // LOC100289528 // hypothetical protein LOC100289528 | ENST00000438157 | SEQ ID NO: 998 | SEQ ID NO: 138 |
| // ARID5B // AT rich interactive domain 5B (MRF1-like) | NM_032199 | SEQ ID NO:139 | SEQ ID NO: 140 |
| // ADD3 // adducin 3 (gamma) | NM_016824 | SEQ ID NO: 141 | SEQ ID NO:142 |
| // PLCG1 // phospholipase C, gamma 1 | NM_002660 | SEQ ID NO: 143 | SEQ ID NO: 144 |
| // SNORD115-31 // small nucleolar RNA, C/D box 115-31 | NR_003346 | SEQ ID NO:145 | N/A |
| // PYROXD2 // pyridine nucleotide-disulphide oxidoreductase domain 2 | NM_032709 | SEQ ID NO: 146 | SEQ ID NO: 147 |
| // AMICA1 // adhesion molecule, interacts with CXADR antigen 1 | NM_001098526 | SEQ ID NO: 148 | SEQ ID NO: 149 |
| // TGFBI // transforming growth factor, beta-induced, 68kDa | NM_000358 | SEQ ID NO: 150 | SEQ ID NO: 151 |
| // ABI2 // abl-interactor 2 | NM_005759 | SEQ ID NO: 152 | SEQ ID NO: 153 |
| // SLC16A7 // solute carrier family 16, member 7 (monocarboxylic acid transporter 2) | NM_004731 | SEQ ID NO: 154 | SEQ ID NO: 155 |
| // CD3D // CD3d molecule, delta (CD3-TCR complex) | NM_000732 | SEQ ID NO: 156 | SEQ ID NO: 157 |
| // OSBPL3 // oxysterol binding protein-like 3 | NM_ 015550 | SEQ ID NO: 158 | SEQ ID NO: 159 |
| // TPCN1 // two pore segment channel 1 | NM_ 001143819 | SEQ ID NO: 160 | SEQ ID NO: 161 |
| // ECE1 // endothelin converting enzyme 1 | NM_001397 | SEQ ID NO: 162 | SEQ ID NO: 163 |
| // TGFA // transforming growth factor, alpha | NM_003236 | SEQ ID NO: 164 | SEQ ID NO: 165 |
| // ZEB1 // zinc finger E-box binding homeobox 1 | NR_024285 | SEQ ID NO: 166 | N/A |
| - | | | |
| // CTDSPL // CTD (carboxy-terminal domain, RNA polymerase II, polypeptide A) small phosphatase-like | NM_001008392 | SEQ ID NO: 167 | SEQ ID NO: 168 |
| // PPFIBP2 // PTPRF interacting protein, binding protein 2 (liprin beta 2) | NM_003621 | SEQ ID NO: 169 | SEQ ID NO: 170 |
| // KLRD1 // killer cell lectin-like receptor subfamily D, member 1 | NM_002262 | SEQ ID NO: 171 | SEQ ID NO: 172 |
| // GM2A // GM2 ganglioside activator | NM_000405 | SEQ ID NO: 173 | SEQ ID NO: 174 |
| // EIF4G3 // eukaryotic translation initiation factor 4 gamma, 3 | NM_003760 | SEQ ID NO: 175 | SEQ ID NO: 176 |
| // PADI2 // peptidyl arginine deiminase, type II | NM_007365 | SEQ ID NO: 177 | SEQ ID NO: 178 |
| // TSPAN33 // tetraspanin 33 | NM_178562 | SEQ ID NO: 179 | SEQ ID NO: 180 |
| // SDC3 // syndecan 3 | NM_014654 | SEQ ID NO: 181 | SEQ ID NO: 182 |
| // ZFP36L1 // zinc finger protein 36, C3H type-like 1 | NM_004926 | SEQ ID NO: 183 | SEQ ID NO: 184 |
| // CPM // carboxypeptidase M | NM_001874 | SEQ ID NO: 185 | SEQ ID NO: 186 |
| // SPP1 // secreted phosphoprotein 1 | NM_001040058 | SEQ ID NO: 187 | SEQ ID NO: 188 |
| // CYFIP2 // cytoplasmic FMR1 interacting protein 2 | NM_001037332 | SEQ ID NO: 189 | SEQ ID NO: 190 |
| // FLNB // filamin B, beta | NM_001457 | SEQ ID NO: 191 | SEQ ID NO: 192 |
| // C6orf192 // chromosome 6 open reading frame 192 | NM_052831 | SEQ ID NO: 193 | SEQ ID NO: 194 |
| // CD226 // CD226 molecule | NM_006566 | SEQ ID NO: 195 | SEQ ID NO: 196 |
| // RASA3 // RAS p21 protein activator 3 | NM_007368 | SEQ ID NO: 197 | SEQ ID NO: 198 |
| // CD274 // CD274 molecule | NM_014143 | SEQ ID NO: 199 | SEQ ID NO: 200 |
| // CD28 // CD28 molecule | NM_006139 | SEQ ID NO: 201 | SEQ ID NO: 202 |
| // KCNMA1 // potassium large conductance calcium-activated channel, subfamily M, alpha member 1 | NM_001014797 | SEQ ID NO: 203 | SEQ ID NO: 204 |
| NM_001259 // CDK6 // cyclin-dependent kinase 6 | NM_001259 | SEQ ID NO: 205 | SEQ ID NO: 206 |
| // ADAM19 // ADAM metallopeptidase domain 19 (meltrin beta) | NM_033274 | SEQ ID NO: 207 | SEQ ID NO: 208 |
| // CD3G // CD3g molecule, gamma (CD3-TCR complex) | NM_000073 | SEQ ID NO: 209 | SEQ ID NO: 210 |
| // ADORA3 // adenosine A3 receptor | NM_020683 | SEQ ID NO: 211 | SEQ ID NO: 212 |
| // STAT4 // signal transducer and activator of transcription 4 | NM_003151 | SEQ ID NO: 213 | SEQ ID NO: 214 |
| // CD86 // CD86 molecule | NM_175862 | SEQ ID NO: 215 | SEQ ID NO: 216 |
| // TM7SF4 // transmembrane 7 superfamily member 4 | NM_030788 | SEQ ID NO: 217 | SEQ ID NO: 218 |
| // CCL3L1 // chemokine (C-C motif) ligand 3-like 1 | NM_021006 | SEQ ID NO: 219 | SEQ ID NO: 220 |
| // CCL3L1 // chemokine (C-C motif) ligand 3-like 1 | NM_021006 | SEQ ID NO: 221 | SEQ ID NO: 222 |
| // CCL3L1 // chemokine (C-C motif) ligand 3-like 1 | NM_021006 | SEQ ID NO: 223 | SEQ ID NO: 224 |
| // TOX // thymocyte selection-associated high mobility group box | NM_014729 | SEQ ID NO: 225 | SEQ ID NO: 226 |
| // ITK // IL2-inducible T-cell kinase | NM_005546 | SEQ ID NO: 227 | SEQ ID NO: 228 |
| // SORBS2 // sorbin and SH3 domain containing 2 | NM_021069 | SEQ ID NO: 229 | SEQ ID NO: 230 |
| // PLEKHA5 // pleckstrin homology domain containing, family A member 5 | NM_019012 | SEQ ID NO: 231 | SEQ ID NO: 232 |
| // FAT1 // FAT tumor suppressor homolog 1 (Drosophila) | NM_005245 | SEQ ID NO: 233 | SEQ ID NO: 234 |
| // TGFBR3 // transforming growth factor, beta receptor III | NM_003243 | SEQ ID NO: 235 | SEQ ID NO: 236 |
| // SMAD3 // SMAD family member 3 | NM_005902 | SEQ ID NO: 237 | SEQ ID NO: 238 |
| // TUBA4A // tubulin, alpha 4a | NM_006000 | SEQ ID NO: 230 | SEQ ID NO: 240 |
| // SLC40A1 // solute carrier family 40 (iron-regulated transporter), member 1 | NM_014585 | SEQ ID NO: 241 | SEQ ID NO: 242 |
| // LEF1 // lymphoid enhancer-binding factor 1 | NM_016269 | SEQ ID NO: 243 | SEQ ID NO: 244 |
| // CCL8 // chemokine (C-C motif) ligand 8 | NM_005623 | SEQ ID NO: 245 | SEQ ID NO: 246 |
| // CCR4 // chemokine (C-C motif) receptor 4 | NM_005508 | SEQ ID NO: 247 | SEQ ID NO: 248 |
| // CDC14A // CDC14 cell division cycle 14 homolog A (S. cerevisiae) | NM_003672 | SEQ ID NO: 240 | SEQ ID NO: 250 |
| // PRKCQ // protein kinase C, theta | NM_006257 | SEQ ID NO: 251 | SEQ ID NO: 252 |
| // STK39 // serine threonine kinase 39 (STE20/SPS1 homolog, yeast) | NM_013233 | SEQ ID NO: 253 | SEQ ID NO: 254 |
| // TSPAN3 // tetraspanin 3 | NM_005724 | SEQ ID NO: 255 | SEQ ID NO: 256 |
| // CCDC88C // coiled-coil domain containing 88C | NM_001080414 | SEQ ID NO: 257 | SEQ ID NO: 258 |
| // PDE4D // phosphodiesterase 4D, cAMP-specific (phosphodiesterase E3 dunce homolog, Drosophila) | NM_001104631 | SEQ ID NO: 259 | SEQ ID NO: 260 |
| // DFNA5 // deafness, autosomal dominant 5 | NM_004403 | SEQ ID NO: 261 | SEQ ID NO: 262 |
| // MMP2 // matrix metallopeptidase 2 (gelatinase A, 72kDa gelatinase, 72kDa type IV collagenase) | NM_004530 | SEQ ID NO: 263 | SEQ ID NO: 264 |
| // GNG2 // guanine nucleotide binding protein (G protein), gamma 2 | NM_053064 | SEQ ID NO: 265 | SEQ ID NO: 266 |
| // ETS1 // v-ets erythroblastosis virus E26 oncogene homolog 1 (avian) | NM_001143820 | SEQ ID NO: 267 | SEQ ID NO: 268 |
| // P2RY12 // purinergic receptor P2Y, G-protein coupled, 12 | NM_022788 | SEQ ID NO: 269 | SEQ ID NO: 270 |
| // SLC9A9 // solute carrier family 9 (sodium/hydrogen exchanger), member 9 | NM_173653 | SEQ ID NO: 271 | SEQ ID NO: 272 |
| // CD180 // CD180 molecule | NM_005582 | SEQ ID NO: 273 | SEQ ID NO: 274 |
| // TRERF1 // transcriptional regulating factor 1 | NM_033502 | SEQ ID NO: 275 | SEQ ID NO: 276 |
| // CD1C // CD1c molecule | NM_001765 | SEQ ID NO: 277 | SEQ ID NO: 278 |
| // INPP4B // inositol polyphosphate-4-phosphatase, type II, 105kDa | NM_003866 | SEQ ID NO: 279 | SEQ ID NO: 280 |
| // DKFZP56400823 // prostatic androgen-repressed messaqe-1 | NM_015393 | SEQ ID NO: 281 | SEQ ID NO: 282 |
| // STAMBPL1 // STAM binding protein-like 1 | NM_020799 | SEQ ID NO: 283 | SEQ ID NO: 284 |
| // ADAM23 // ADAM metallopeptidase domain 23 | NM_003812 | SEQ ID NO: 285 | SEQ ID NO: 286 |
| // SRGAP1 // SLIT-ROBO Rho GTPase activating protein 1 | NM_020762 | SEQ ID NO: 287 | SEQ ID NO: 288 |
| // TUBB2C // tubulin, beta 2C | NM_006088 | SEQ ID NO: 289 | SEQ ID NO: 290 |
| // CES1 // carboxylesterase 1 (monocyte/macrophag e serine esterase 1) | NM_001025195 | SEQ ID NO: 291 | SEQ ID NO: 292 |
| // DPP4 // dipeptidyl-peptidase 4 | NM_001935 | SEQ ID NO: 293 | SEQ ID NO: 294 |
| // SKAP1 // src kinase associated phosphoprotein 1 | NM_003726 | SEQ ID NO: 295 | SEQ ID NO: 296 |
| // SEPP1 // selenoprotein P, plasma, 1 | NM_005410 | SEQ ID NO: 297 | SEQ ID NO: 298 |
| // KIAA0746 // KIAA0746 protein | NM_015187 | SEQ ID NO: 299 | SEQ ID NO: 300 |
| // CD200R1 // CD200 receptor 1 | NM_138806 | SEQ ID NO: 301 | SEQ ID NO: 302 |
| // ANGPTL2 // angiopoietin-like 2 | NM_012098 | SEQ ID NO: 303 | SEQ ID NO: 304 |
| // GZMK // granzyme K (granzyme 3; tryptase II) | NM_002104 | SEQ ID NO: 305 | SEQ ID NO: 306 |
| // SLC16A 10 // solute carrier family 16, member 10 (aromatic amino acid transporter) | NM_018593 | SEQ ID NO: 307 | SEQ ID NO: 308 |
| // MARCKS // myristoylated alanine-rich protein kinase C substrate | NM_002356 | SEQ ID NO: 309 | SEQ ID NO: 310 |
| // C6orf105 // chromosome 6 open reading frame 105 | NM_001143948 | SEQ ID NO: 311 | SEQ ID NO: 312 |
| // CCND2 // cyclin D2 | NM_ 001759 | SEQ ID NO: 313 | SEQ ID NO: 314 |
| // GDPD1 // glycerophosphodiester phosphodiesterase domain containing 1 | NM_182569 | SEQ ID NO: 315 | SEQ ID NO: 316 |
| // CD38 // CD38 molecule | NM_001775 | SEQ ID NO: 317 | SEQ ID NO: 318 |
| // TGFB2 // transforming growth factor, beta 2 | NM_001135599 | SEQ ID NO: 319 | SEQ ID NO: 320 |
| // ARRDC4 // arrestin domain containing 4 | NM_183376 | SEQ ID NO: 321 | SEQ ID NO: 322 |
| // ITM2A // integral membrane protein 2A | NM_004867 | SEQ ID NO: 323 | SEQ ID NO: 324 |
| // SLC44A3 // solute carrier family 44, member 3 | NM_001114106 | SEQ ID NO: 325 | SEQ ID NO: 326 |
| // FGD6 // FYVE, RhoGEF and PH domain containing 6 | NM_018351 | SEQ ID NO: 327 | SEQ ID NO: 328 |
| // BIRC3 // baculoviral IAP repeat-containing 3 | NM_001165 | SEQ ID NO: 329 | SEQ ID NO: 330 |
| // GUCY1A3 // guanylate cyclase 1, soluble, alpha 3 | NM_000856 | SEQ ID NO: 331 | SEQ ID NO: 332 |
| // PAPSS2 // 3'-phosphoadenosine 5'-phosphosulfate synthase 2 | NM_004670 | SEQ ID NO: 333 | SEQ ID NO: 334 |
| // RAB6B // RAB6B, member RAS oncogene family | NM_016577 | SEQ ID NO: 335 | SEQ ID NO: 336 |
| // SLC38A1 // solute carrier family 38, member 1 | NM_030674 | SEQ ID NO: 337 | SEQ ID NO: 338 |
| // ST8SIA4 // ST8 alpha-N-acetyl-neuraminide alpha-2,8-sialyltransferase 4 | NM_005668 | SEQ ID NO: 339 | SEQ ID NO: 340 |
| // WDR17 // WD repeat domain 17 | NM_170710 | SEQ ID NO: 341 | SEQ ID NO: 342 |
| // C4orf18 // chromosome 4 open reading frame 18 | NM_001128424 | SEQ ID NO: 343 | SEQ ID NO: 344 |
| // KLRG1 // killer cell lectin-like receptor subfamily G, member 1 | NM_005810 | SEQ ID NO: 345 | SEQ ID NO: 346 |
| // TES // testis derived transcript (3 LIM domains) | NM_015641 | SEQ ID NO: 347 | SEQ ID NO: 348 |
| // ABCA6 // ATP-binding cassette, sub-family A (ABC1), member 6 | NM_080284 | SEQ ID NO: 349 | SEQ ID NO: 350 |
| // CD96 // CD96 molecule | NM_198196 | SEQ ID NO: 351 | SEQ ID NO: 352 |
| // C5orf13 // chromosome 5 open reading frame 13 | NM_004772 | SEQ ID NO: 353 | SEQ ID NO: 354 |
| // ITGB3 // integrin, beta 3 (platelet glycoprotein IIIa, antigen CD61) | NM_000212 | SEQ ID NO: 355 | SEQ ID NO: 356 |
| // PLXNC1 // plexin C1 | NM_005761 | SEQ ID NO: 357 | SEQ ID NO: 358 |
| // NEDD4L // neural precursor cell expressed, developmentally down-regulated 4-like | NM_001144967 | SEQ ID NO: 359 | SEQ ID NO: 360 |
| // LGMN // legumain | NM_005606 | SEQ ID NO: 361 | SEQ ID NO: 362 |
| // SCIN // scinderin | NM_001112706 | SEQ ID NO: 363 | SEQ ID NO: 364 |
| // TRAT1 // T cell receptor associated transmembrane adaptor 1 | NM_016388 | SEQ ID NO: 365 | SEQ ID NO: 366 |
| // ANTXR2 // anthrax toxin receptor 2 | NM_058172 | SEQ ID NO: 367 | SEQ ID NO: 368 |
| // CCL4L1 // chemokine (C-C motif) ligand 4-like 1 | NM_001001435 | SEQ ID NO: 369 | SEQ ID NO: 370 |
| // KIAA0040 // KIAA0040 | NM_014656 | SEQ ID NO: 371 | SEQ ID NO: 372 |
| // MYO1 D // myosin ID | NM_015194 | SEQ ID NO: 373 | SEQ ID NO: 374 |
| - | | | |
| // RARRES1 // retinoic acid receptor responder (tazarotene induced) 1 | NM_206963 | SEQ ID NO: 375 | SEQ ID NO: 376 |
| // LYVE1 // lymphatic vessel endothelial hyaluronan receptor 1 | NM_006691 | SEQ ID NO: 377 | SEQ ID NO: 378 |
| // GPR174 // G protein-coupled receptor 174 | NM_032553 | SEQ ID NO: 379 | SEQ ID NO: 380 |
| // GABRG3 // gamma-aminobutyric acid (GABA) A receptor, gamma 3 | NM_033223 | SEQ ID NO: 381 | SEQ ID NO: 382 |
| // FOLR2 // folate receptor 2 (fetal) | NM_000803 | SEQ ID NO: 383 | SEQ ID NO: 384 |
| --- | | | |
| --- | | | |
| // GPR171 // G protein-coupled receptor 171 | NM_013308 | SEQ ID NO: 385 | SEQ ID NO: 386 |
| // PMP22 // peripheral myelin protein 22 | NM_000304 | SEQ ID NO: 387 | SEQ ID NO: 388 |
| // PLD3 // phospholipase D family, member 3 | NM_012268 | SEQ ID NO: 389 | SEQ ID NO: 390 |
| // VSIG4 // V-set and immunoglobulin domain containing 4 | NM_007268 | SEQ ID NO: 391 | SEQ ID NO: 392 |
| // PTPRB // protein tyrosine phosphatase, receptor type, B | NM_001109754 | SEQ ID NO: 393 | SEQ ID NO: 394 |
| // RNF125 // ring finger protein 125 | NM_017831 | SEQ ID NO: 395 | SEQ ID NO: 396 |
| // TCN2 // transcobalamin II; macrocytic anemia | NM_000355 | SEQ ID NO: 397 | SEQ ID NO: 398 |
| // EPS8 // epidermal growth factor receptor pathway substrate 8 | NM_004447 | SEQ ID NO: 399 | SEQ ID NO: 400 |
| // CD84 // CD84 molecule | NM_003874 | SEQ ID NO: 401 | SEQ ID NO: 402 |
| // F13A1 // coagulation factor XIII, A1 polypeptide | NM_000129 | SEQ ID NO: 403 | SEQ ID NO: 404 |
| // IKZF3 // IKAROS family zinc finger 3 (Aiolos) | NM_012481 | SEQ ID NO: 405 | SEQ ID NO: 406 |
| // SLFN5 // schlafen family member 5 | NM_144975 | SEQ ID NO: 407 | SEQ ID NO: 408 |
| // CAMK4 // calcium/calmodulin-dependent protein kinase IV | NM_001744 | SEQ ID NO: 409 | SEQ ID NO: 410 |
| // PLA2G7 // phospholipase A2, group VII (platelet-activating factor acetyl hydrolase, plasma) | NM_005084 | SEQ ID NO: 411 | SEQ ID NO: 412 |
| // TNS1 // tensin 1 | NM_022648 | SEQ ID NO: 413 | SEQ ID NO: 414 |
| // HBD // hemoglobin, delta | NM_000519 | SEQ ID NO: 415 | SEQ ID NO: 416 |
| // CTSL1 // cathepsin L1 | NM_001912 | SEQ ID NO: 417 | SEQ ID NO: 418 |
| // SELL // selectin L | NM_000655 | SEQ ID NO: 419 | SEQ ID NO: 420 |
| // TNFSF10 // tumor necrosis factor (ligand) superfamily, member 10 | NM_003810 | SEQ ID NO: 421 | SEQ ID NO: 422 |
| // CLIC2 // chloride intracellular channel 2 | NM_001289 | SEQ ID NO: 423 | SEQ ID NO: 424 |
| // RGL1 // ral guanine nucleotide dissociation stimulator-like 1 | NM_015149 | SEQ ID NO: 425 | SEQ ID NO: 426 |
| // TM4SF19 // transmembrane 4 L six family member 19 | NM_138461 | SEQ ID NO: 427 | SEQ ID NO: 428 |
| // TFCP2L1 // transcription factor CP2-like 1 | NM_014553 | SEQ ID NO: 429 | SEQ ID NO: 430 |
| // STEAP4 // STEAP family member 4 | NM_024636 | SEQ ID NO: 431 | SEQ ID NO: 432 |
| // GPR15 // G protein-coupled receptor 15 | NM_005290 | SEQ ID NO: 433 | SEQ ID NO: 434 |
| // CYBRD1 // cytochrome b reductase 1 | NM_024843 | SEQ ID NO: 435 | SEQ ID NO: 436 |
| // SFRP4 // secreted frizzled-related protein 4 | NM_003014 | SEQ ID NO: 437 | SEQ ID NO: 438 |
| // CMKLR1 // chemokine-like receptor 1 | NM_001142343 | SEQ ID NO: 439 | SEQ ID NO: 440 |
| // HGF // hepatocyte growth factor (hepapoietin A; scatter factor) | NM_000601 | SEQ ID NO: 441 | SEQ ID NO: 442 |
| // CCL2 // chemokine (C-C motif) ligand 2 | NM_002982 | SEQ ID NO: 443 | SEQ ID NO: 444 |
| // THSD7A // thrombospondin, type I, domain containing 7A | NM_015204 | SEQ ID NO: 445 | SEQ ID NO: 446 |
| // SULT1C2 // sulfotransferase family, cytosolic, 1C, member 2 | NM_001056 | SEQ ID NO: 447 | SEQ ID NO: 448 |
| // MMP9 // matrix metallopeptidase 9 (gelatinase B, 92kDa gelatinase, 92kDa type IV collagenase) | NM_004994 | SEQ ID NO: 449 | SEQ ID NO: 450 |
| // LRRC39 // leucine rich repeat containing 39 | NM_144620 | SEQ ID NO: 451 | SEQ ID NO: 452 |
| // HBB // hemoglobin, beta | NM_000518 | SEQ ID NO: 453 | SEQ ID NO: 454 |
| // CTSK // cathepsin K | NM_000396 | SEQ ID NO: 455 | SEQ ID NO: 456 |
| // FABP3 // fatty acid binding protein 3, muscle and heart (mammary-derived growth inhibitor) | NM_004102 | SEQ ID NO: 457 | SEQ ID NO: 458 |
| // ENPP2 // ectonucleotide pyrophosphatase/phos phodiesterase 2 | NM_006209 | SEQ ID NO: 459 | SEQ ID NO: 460 |
| // MLANA // melan-A | NM_005511 | SEQ ID NO: 461 | SEQ ID NO: 462 |
| ABCD2// ATP-binding cassette, sub-family D (ALD), member 2 | NM_005164 | SEQ ID NO: 999 | SEQ ID NO: 1000 |
| C12orf35//chromosom e 12 open reading frame 35 | NM_018169 | SEQ ID NO: 1001 | SEQ ID NO: 1002 |
| CD207//CD207 molecule, langerin | NM_015717 | SEQ ID NO: 1003 | SEQ ID NO: 1004 |
| CD247//CD247 molecule | NM_198053 | SEQ ID NO: 1005 | SEQ ID NO: 1006 |
| CD27//CD27 molecule | NM_001242 | SEQ ID NO: 1007 | SEQ ID NO: 1008 |
| CD5L //CD5 molecule-like | NM_005894 | SEQ ID NO: 1009 | SEQ ID NO: 1010 |
| CHIT1//chitinase 1 (chitotriosidase) | NM_003465 | SEQ ID NO: 1011 | SEQ ID NO: 1012 |
| CLEC2D //C-type lectin domain family 2, member D | NM_001004419 | SEQ ID NO: 1013 | SEQ ID NO: 1014 |
| CST7//cystatin F (leukocystatin) | NM_003650 | SEQ ID NO: 1015 | SEQ ID NO: 1016 |
| DTNA //dystrobrevin, alpha | NM_001390 | SEQ ID NO: 1017 | SEQ ID NO: 1018 |
| ENTPD1//ectonucleosi de triphosphate diphosphohydrolase 1 | NM_001776 | SEQ ID NO: 1019 | SEQ ID NO: 1020 |
| EPHB1 //EPH receptor B1 | NM_004441 | SEQ ID NO: 1021 | SEQ ID NO: 1022 |
| ESPNP //espin pseudogene | NR_026567 | SEQ ID NO: 1023 | N/A |
| FAM40B //family with sequence similarity 40, member B | NM_020704 | SEQ ID NO: 1024 | SEQ ID NO: 1025 |
| FAM87A //family with sequence similarity 87, member A | BC037297 | SEQ ID NO: 1026 | N/A |
| FBXO40//F-box protein 40 | NM_016298 | SEQ ID NO: 1027 | SEQ ID NO: 1028 |
| FCGR2A// Fc fragment of IgG, low affinity Ila, receptor (CD32) | NM_001136219 | SEQ ID NO: 1029 | SEQ ID NO: 1030 |
| FCGR2B //Fc fragment of IgG, low affinity IIb, receptor (CD32) | NM_004001 | SEQ ID NO: 1031 | SEQ ID NO: 1032 |
| FCGR2C //Fc fragment of IgG, low affinity IIc, receptor for (CD32) | NM_201563 | SEQ ID NO: 1033 | SEQ ID NO: 1034 |
| FMN1// formin 1 | ENST00000414268 | SEQ ID NO: 1035 | SEQ ID NO: 1036 |
| FMO1 //flavin containing monooxygenase 1 | NM_002021 | SEQ ID NO: 1037 | SEQ ID NO: 1038 |
| FOLH1 //folate hydrolase (prostate-specific membrane antigen) 1 | NM_153696 | SEQ ID NO: 1039 | SEQ ID NO: 1040 |
| FOLH1B //folate hydrolase 1B | NM_153696 | SEQ ID NO: 1041 | SEQ ID NO: 1042 |
| FYN //FYN oncogene related to SRC, FGR, YES | NM_002037 | SEQ ID NO: 1043 | SEQ ID NO: 1044 |
| GAST //gastrin | NM_000805 | SEQ ID NO: 1045 | SEQ ID NO: 1046 |
| GlMAP1//GTPase, IMAP family member 1 | NM_130759 | SEQ ID NO: 1047 | SEQ ID NO: 1048 |
| GIMAP5 //GTPase, IMAP family member 5 | NM_018384 | SEQ ID NO: 1049 | SEQ ID NO: 1050 |
| GIMAP8//GTPase, IMAP family member 8 | NM_175571 | SEQ ID NO: 1051 | SEQ ID NO: 1052 |
| GPX3//glutathione peroxidase 3 (plasma) | NM_002084 | SEQ ID NO: 1053 | SEQ ID NO: 1054 |
| GUSBL1//glucuronida se, beta-like 1 | NR_003504 | SEQ ID NO: 1055 | N/A |
| HAVCR2//hepatitis A virus cellular receptor 2 | NM_032782 | SEQ ID NO: 1056 | SEQ ID NO: 1057 |
| IGJ //immunoglobulin J polypeptide, linker protein for immunoglobulin alpha and mu polypeptides | NM_144646 | SEQ ID NO: 1058 | SEQ ID NO: 1059 |
| IGKV3D-11 // immunoglobulin kappa variable 3D-11 | ENST00000390250 | SEQ ID NO: 1060 | SEQ ID NO: 1061 |
| IL1R2// interleukin 1 receptor, type II | NM_004633 | SEQ ID NO: 1062 | SEQ ID NO: 1063 |
| JAKMIP2// janus kinase and microtubule interacting protein 2 | NM_ 014790 | SEQ ID NO: 1064 | SEQ ID NO: 1065 |
| KLHL38//kelch-like 38 (Drosophila) | NM_001081675 | SEQ ID NO: 1066 | SEQ ID NO: 1067 |
| LAT2 //linker for activation of T cells family, member 2 | NM_032464 | SEQ ID NO: 1068 | SEQ ID NO: 1069 |
| LIPA// lipase A, lysosomal acid, cholesterol esterase | NM_001127605 | SEQ ID NO: 1070 | SEQ ID NO: 1071 |
| LOC100128751//INM0 4 | A Y194294 | SEQ ID NO: 1072 | SEQ ID NO: 1073 |
| LOC91316//glucuronid ase, beta | NR_024448 | SEQ ID NO: 1074 | N/A |
| LPL //lipoprotein lipase | NM_000237 | SEQ ID NO: 1075 | SEQ ID NO: 1076 |
| LY9// lymphocyte antigen 9 | NM_002348 | SEQ ID NO: 1077 | SEQ ID NO: 1078 |
| MAF// v-maf musculoaponeurotic fibrosarcoma oncogene homolog (avian) | NM_001031804 | SEQ ID NO: 1079 | SEQ ID NO: 1080 |
| MATK //megakaryocyte-associated tyrosine kinase | NM_139355 | SEQ ID NO: 1081 | SEQ ID NO: 1082 |
| MS4A4A //membrane-spanning 4-domains, subfamily A, member 4 | NM_024021 | SEQ ID NO: 1083 | SEQ ID NO: 1084 |
| MX2 //myxovirus (influenza virus) resistance 2 (mouse) | NM_002463 | SEQ ID NO: 1085 | SEQ ID NO: 1086 |
| NCALD //neurocalcin delta | NM_001040624 | SEQ ID NO: 1087 | SEQ ID NO: 1088 |
| OBFC2A //oligonucleotide | NM_001031716 | SEQ ID NO: 1089 | SEQ ID NO: 1090 |
| OR14C36// olfactory receptor, family 14, subfamily C, member 36 | NM_001001918 | SEQ ID NO: 1091 | SEQ ID NO: 1092 |
| OR5L1//olfactory receptor, family 5, subfamily L, member 1 | NM_001004738 | SEQ ID NO: 1093 | SEQ ID NO: 1094 |
| PDZRN3//PDZ domain containing ring finger 3 | NM_015009 | SEQ ID NO: 1095 | SEQ ID NO: 1096 |
| PLXDC1//plexin domain containing 1 | NM_ 020405 | SEQ ID NO: 1097 | SEQ ID NO: 1098 |
| PP13004//hypothetical LOC402481 | ENST00000381493 | SEQ ID NO: 1099 | SEQ ID NO: 1100 |
| PSAT1 //phosphoserine aminotransferase 1 | NM_058179 | SEQ ID NO: 1101 | SEQ ID NO: 1102 |
| PTPRE //protein tyrosine phosphatase, receptor type, E | NM_006504 | SEQ ID NO: 1103 | SEQ ID NO: 1104 |
| RASSF2 //Ras association (RaIGDS | NM_014737 | SEQ ID NO: 1105 | SEQ ID NO: 1106 |
| RCAN3// RCAN family member 3 | NM_013441 | SEQ ID NO: 1107 | SEQ ID NO: 1108 |
| RCSD1 //RCSD domain containing 1 | NM_052862 | SEQ ID NO: 1109 | SEQ ID NO: 1110 |
| RGS10// regulator of G-protein signaling | NM_001005339 | SEQ ID NO: 1111 | SEQ ID NO: 1112 |
| RGS9// regulator of G-protein signaling 9 | NM_003835 | SEQ ID NO: 1113 | SEQ ID NO: 1114 |
| RHOBTB1 //Rho-related BTB domain containing 1 | NM_001242359, | SEQ ID NO: 1115 | SEQ ID NO: 1116 |
| RNASE3// ribonuclease, RNase A family, 3 (eosinophil cationic protein) | NM_002935 | SEQ ID NO: 1117 | SEQ ID NO: 1118 |
| RTKN2 //rhotekin 2 | NM_145307 | SEQ ID NO: 1119 | SEQ ID NO: 1120 |
| RUNX2 //runt-related transcription factor 2 | NM_001024630 | SEQ ID NO: 1121 | SEQ ID NO: 1122 |
| SCML4 //sex comb on midleg-like 4 (Drosophila) | NM_198081 | SEQ ID NO: 1123 | SEQ ID NO: 1124 |
| SEPT3// septin 3 | NM_019106 | SEQ ID NO: 1125 | SEQ ID NO: 1126 |
| SH2D1A //SH2 domain protein 1A | NM_002351 | SEQ ID NO: 1127 | SEQ ID NO: 1128 |
| SLC16A9// solute carrier family 16, member 9 (monocarboxylic acid transporter 9) | NM_194298 | SEQ ID NO: 1129 | SEQ ID NO: 1130 |
| SLCO4A1// solute carrier organic anion transporter family, member 4A1 | NM_016354 | SEQ ID NO: 1131 | SEQ ID NO: 1132 |
| SMA5 //glucuronidase, beta pseudogene | AK289851 | SEQ ID NO: 1133 | SEQ ID NO: 1134 |
| ST3GAL5 //ST3 beta-galactoside alpha-2,3-sialyltransferase 5 | NM_003896 | SEQ ID NO: 1135 | SEQ ID NO: 1136 |
| SULF2 //sulfatase 2 | NM_018837 | SEQ ID NO: 1137 | SEQ ID NO: 1138 |
| TCF7 //transcription factor 7 (T-cell specific, HMG-box) | NM_201634 | SEQ ID NO: 1139 | SEQ ID NO: 1140 |
| TMEM176A// transmembrane protein 176A | NM_018487 | SEQ ID NO: 1141 | SEQ ID NO: 1142 |
| TMEM45A// transmembrane protein 45A | NM_018004 | SEQ ID NO: 1143 | SEQ ID NO: 1144 |
| TRAF3IP3// TRAF3 interacting protein 3 | NM_025228 | SEQ ID NO: 1145 | SEQ ID NO: 1146 |
| TREM2// triggering receptor expressed on myeloid cells 2 | NM_018965 | SEQ ID NO: 1147 | SEQ ID NO: 1148 |
| TYR //tyrosinase (oculocutaneous albinism IA) | NM_000372 | SEQ ID NO: 1149 | SEQ ID NO: 1150 |
| UBASH3A //ubiquitin associated and SH3 domain containing, A | NM_ 018961 | SEQ ID NO: 1151 | SEQ ID NO: 1152 |

**Table 2: Genes Down-regulated Upon STX-100 Administration**

| **gene_assignment** | **Genbank No.** | **Nucleotide Sequence** | **Protein Sequence** |
|---|---|---|---|
| // GPR82 // G protein-coupled receptor 82 | NM_080817 | SEQ ID NO: 463 | SEQ ID NO: 464 |
| // ENPP1 // ectonucleotide pyrophosphatase/phos phodiesterase 1 | NM_006208 | SEQ ID NO: 465 | SEQ ID NO: 466 |
| // THBS1 // thrombospondin 1 | NM_003246 | SEQ ID NO: 467 | SEQ ID NO: 468 |
| // SYDE2 // synapse defective 1, Rho GTPase, homolog 2 (C. elegans) | NM_032184 | SEQ ID NO: 469 | SEQ ID NO: 470 |
| // IGSF2 // immunoglobulin superfamily, member 2 | NM_004258 | SEQ ID NO: 471 | SEQ ID NO: 472 |
| // RETN // resistin | NM_020415 | SEQ ID NO: 473 | SEQ ID NO: 474 |
| // GPR116 // G protein-coupled receptor 116 | NM_015234 | SEQ ID NO: 475 | SEQ ID NO: 476 |
| // TRHDE // thyrotropin-releasing hormone degrading enzyme | NM_013381 | SEQ ID NO: 477 | SEQ ID NO: 478 |
| // CACNB4 // calcium channel, voltage-dependent, beta 4 subunit | NM_000726 | SEQ ID NO: 479 | SEQ ID NO: 480 |
| // PLXDC2 // plexin domain containing 2 | NM_032812 | SEQ ID NO: 481 | SEQ ID NO: 482 |
| // SMC6 // structural maintenance of chromosomes 6 | NM_001142286 | SEQ ID NO: 483 | SEQ ID NO: 484 |
| // OLR1 // oxidized low density lipoprotein (lectin-like) receptor 1 | NM_002543 | SEQ ID NO: 485 | SEQ ID NO: 486 |
| // SERPINE1 // serpin peptidase inhibitor, clade E (nexin, plasminogen activator inhibitor type 1), member 1 | NM_000602 | SEQ ID NO: 487 | SEQ ID NO: 488 |
| // MEST // mesoderm specific transcript homolog (mouse) | NM_002402 | SEQ ID NO: 489 | SEQ ID NO: 490 |
| // LY75 // lymphocyte antigen 75 | NM_002349 | SEQ ID NO: 491 | SEQ ID NO: 492 |
| // PRKAR2B // protein kinase, cAMP-dependent, regulatory, type II, beta | NM_002736 | SEQ ID NO: 493 | SEQ ID NO: 494 |
| // TCF7L2 // transcription factor 7-like 2 (T-cell specific, HMG-box) | NM_001146274 | SEQ ID NO: 495 | SEQ ID NO: 496 |
| // CLEC5A // C-type lectin domain family 5, member A | NM_013252 | SEQ ID NO: 497 | SEQ ID NO: 498 |
| // AWAT2 // acyl-CoA wax alcohol acyltransferase 2 | NM_001002254 | SEQ ID NO: 499 | SEQ ID NO: 500 |
| // B3GNT5 // UDP-GlcNAc:betaGal beta-1,3-N-acetylglucosaminyltran sferase 5 | NM_032047 | SEQ ID NO: 501 | SEQ ID NO: 502 |
| // MICAL3 // microtubule associated monoxygenase, calponin and LIM domain containing 3 | NM_015241 | SEQ ID NO: 503 | SEQ ID NO: 504 |
| // PLAC8 // placenta-specific 8 | NM_016619 | SEQ ID NO: 505 | SEQ ID NO: 506 |
| // SLC11A1 // solute carrier family 11 (proton-cou pled divalent metal ion transporters), member 1 | NM_000578 | SEQ ID NO: 507 | SEQ ID NO: 508 |
| // HSF5 // heat shock transcription factor family member 5 // 17q22 // 124535 /// ENST00000323777 // HSF5 // heat shock transcription factor family member 5 | NM_001080439 | SEQ ID NO: 509 | SEQ ID NO: 510 |
| // EDIL3 // EGF-like repeats and discoidin I-like domains 3 | NM_005711 | SEQ ID NO: 511 | SEQ ID NO: 512 |
| // GLRB // glycine receptor, beta | NM_000824 | SEQ ID NO: 513 | SEQ ID NO: 514 |
| // GPR120 // G protein-coupled receptor 120 | NM_1181745 | SEQ ID NO: 515 | SEQ ID NO: 516 |
| // EMR1 // egf-like module containing, mucin-like, hormone receptor-like 1 | NM_001974 | SEQ ID NO: 517 | SEQ ID NO: 518 |
| // CHI3L1 // chitinase 3-like 1 (cartilage glycoprotein-39) | NM_001276 | SEQ ID NO: 519 | SEQ ID NO: 520 |
| // RTN1 // reticulon 1 | NM_021136 | SEQ ID NO: 521 | SEQ ID NO: 522 |
| // GCA // grancalcin, EF-hand calcium binding protein | NM_012198 | SEQ ID NO: 523 | SEQ ID NO: 524 |
| // CLIP4 // CAP-GLY domain containing linker protein family, member 4 | NM_024692 | SEQ ID NO: 525 | SEQ ID NO: 526 |
| // SGMS1 // sphingomyelin synthase 1 | NM_147156 | SEQ ID NO: 527 | SEQ ID NO: 528 |
| // FN1 // fibronectin 1 | NM_212482 | SEQ ID NO: 529 | SEQ ID NO: 530 |
| // FAM9C // family with sequence similarity 9, member C | NM_174901 | SEQ ID NO: 531 | SEQ ID NO: 532 |
| // FER1L6 // fer-1-like 6 (C. elegans) | NM_001039112 | SEQ ID NO: 533 | SEQ ID NO: 534 |
| // PGM5P2 // phosphoglucomutase 5 pseudogene 2 | NR_002836 | SEQ ID NO: 535 | N/A |
| // MMD // monocyte to macrophage differentiation-associated | NM_012329 | SEQ ID NO: 536 | SEQ ID NO: 537 |
| // SEMA3D // sema domain, immunoglobulin domain (Ig), short basic domain, secreted, (semaphorin) 3D | NM_152754 | SEQ ID NO: 538 | SEQ ID NO: 539 |
| // GEN1 // Gen homolog 1, endonuclease (Drosophila) | NM_182625 | SEQ ID NO: 540 | SEQ ID NO: 541 |
| // PGM5P2 // phosphoglucomutase 5 pseudogene 2 | NR_002836 | SEQ ID NO: 542 | N/A |
| // ALOX5 // arachidonate 5-lipoxygenase | NM_000698 | SEQ ID NO: 543 | SEQ ID NO: 544 |
| // CARD17 // caspase recruitment domain family, member 17 | NM_001007232 | SEQ ID NO: 545 | SEQ ID NO: 546 |
| // ITGA6 // integrin, alpha 6 | NM_000210 | SEQ ID NO: 547 | SEQ ID NO: 548 |
| // CAMP // cathelicidin antimicrobial peptide | NM_004345 | SEQ ID NO: 549 | SEQ ID NO: 550 |
| // MTHFD1L // methylenetetrahydrofol ate dehydrogenase (NADP+ dependent) 1-like | NM_015440 | SEQ ID NO: 551 | SEQ ID NO: 552 |
| // MINK1 // misshapen-like kinase 1 (zebrafish) | NM_153827 | SEQ ID NO: 553 | SEQ ID NO: 554 |
| // FHL1 // four and a half LIM domains 1 | NM_001159702 | SEQ ID NO: 555 | SEQ ID NO: 556 |
| - | | | |
| // GPAM // glycerol-3-phosphate acyl transferase, mitochondrial | NM_020918 | SEQ ID NO: 557 | SEQ ID NO: 558 |
| // AMIGO2 // adhesion molecule with Ig-like domain 2 | NM_001143668 | SEQ ID NO: 559 | SEQ ID NO: 560 |
| // TREM1 // triggering receptor expressed on myeloid cells 1 | NM_018643 | SEQ ID NO: 561 | SEQ ID NO: 562 |
| // STAP1 // signal transducing adaptor family member 1 | NM_012108 | SEQ ID NO: 563 | SEQ ID NO: 564 |
| // ABCG1 // ATP-binding cassette, sub-family G (WHITE), member 1 | NM_207627 | SEQ ID NO: 565 | SEQ ID NO: 566 |
| // PRSS12 // protease, serine, 12 (neurotrypsin, motopsin) | NM_003619 | SEQ ID NO: 567 | SEQ ID NO: 568 |
| // NIACR2 // niacin receptor 2 | NM_006018 | SEQ ID NO: 569 | SEQ ID NO: 570 |
| // PPARG // peroxisome proliferator-activated receptor gamma | NM_138712 | SEQ ID NO: 571 | SEQ ID NO: 572 |
| // ENO3 // enolase 3 (beta, muscle) | NM_001976 | SEQ ID NO: 573 | SEQ ID NO: 574 |
| // DLEU2L // deleted in lymphocytic leukemia 2-like | NR_002771 | SEQ ID NO: 575 | N/A |
| // NOSTRIN // nitric oxide synthase trafficker | NM_001039724 | SEQ ID NO: 576 | SEQ ID NO: 577 |
| // KCNA3 // potassium voltage-gated channel, shaker-related subfamily, member 3 | NM_002232 | SEQ ID NO: 578 | SEQ ID NO: 579 |
| // CGNL1 // cingulin-like 1 | NM_032866 | SEQ ID NO: 580 | SEQ ID NO: 581 |
| // MATN2 // matrilin 2 | NM_002380 | SEQ ID NO: 582 | SEQ ID NO: 583 |
| // CLEC4D // C-type lectin domain family 4, member D | NM_080387 | SEQ ID NO: 584 | SEQ ID NO: 585 |
| // CENPV // centromere protein V | NM_181716 | SEQ ID NO: 586 | SEQ ID NO: 587 |
| // RAB13 // RAB13, member RAS oncogene family | NM_002870 | SEQ ID NO: 588 | SEQ ID NO: 589 |
| // OLFML3 // olfactomedin-like 3 | NM_020190 | SEQ ID NO: 590 | SEQ ID NO: 591 |
| // KCNMB1 // potassium large conductance calcium-activated channel, subfamily M, beta member 1 | NM_004137 | SEQ ID NO: 592 | SEQ ID NO: 593 |
| // FPR1 // formyl peptide receptor 1 | NM_002029 | SEQ ID NO: 594 | SEQ ID NO: 595 |
| // DST // dystonin | NM _001144769 | SEQ ID NO: 596 | SEQ ID NO: 597 |
| - | | | |
| // CD1D // CD1d molecule | NM_001766 | SEQ ID NO: 598 | SEQ ID NO: 599 |
| // PECAM1 // platelet/endothelial cell adhesion molecule | NM_000442 | SEQ ID NO: 600 | SEQ ID NO: 601 |
| // DDHD1 // DDHD domain containing 1 | NM_001160148 | SEQ ID NO: 602 | SEQ ID NO: 603 |
| // KLHDC8B // kelch domain containing 8B | NM_173546 | SEQ ID NO: 604 | SEQ ID NO: 605 |
| // ATP8B4 // ATPase, class I, type 8B, member 4 | NM_024837 | SEQ ID NO: 606 | SEQ ID NO: 607 |
| // GPD1 // glycerol-3-phosphate dehydrogenase 1 (soluble) | NM_005276 | SEQ ID NO: 608 | SEQ ID NO: 609 |
| // MCF2L2 // MCF.2 cell line derived transforming sequence-like 2 | NM_015078 | SEQ ID NO: 610 | SEQ ID NO: 611 |
| // SVIL // supervillin | NM_021738 | SEQ ID NO: 612 | SEQ ID NO: 613 |
| // ICAM3 // intercellular adhesion molecule 3 | NM_002162 | SEQ ID NO: 614 | SEQ ID NO: 615 |
| // NLRC4 // NLR family, CARD domain containing 4 | NM_021209 | SEQ ID NO: 616 | SEQ ID NO: 617 |
| // SLC25A16 // solute carrier family 25 (mitochondrial carrier; Graves disease autoantigen), member 16 | NM_152707 | SEQ ID NO: 618 | SEQ ID NO: 619 |
| // CD163 // CD163 molecule | NM_004244 | SEQ ID NO: 620 | SEQ ID NO: 621 |
| // GPR162 // G protein-coupled receptor 162 | NM_019858 | SEQ ID NO: 622 | SEQ ID NO: 623 |
| // RAB30 // RAB30, member RAS oncogene family | NM_014488 | SEQ ID NO: 624 | SEQ ID NO: 625 |
| // SMAD7 // SMAD family member 7 | NM_005904 | SEQ ID NO: 626 | SEQ ID NO: 627 |
| // HBEGF // heparin-binding EGF-like growth factor | NM_001945 | SEQ ID NO: 628 | SEQ ID NO: 629 |
| // RHBDD2 // rhomboid domain containing 2 | NM_001040457 | SEQ ID NO: 630 | SEQ ID NO: 631 |
| // GPR116 // G protein-coupled receptor 116 | NM_015234 | SEQ ID NO: 632 | SEQ ID NO: 633 |
| // ITPR1 // inositol 1,4,5-triphosphate receptor, type 1 | NM_001099952 | SEQ ID NO: 634 | SEQ ID NO: 635 |
| // PIWIL1 // piwi-like 1 (Drosophila) | NM_004764 | SEQ ID NO: 636 | SEQ ID NO: 637 |
| // TANC2 // tetratricopeptide repeat, ankyrin repeat and coiled-coil containing 2 | NM_025185 | SEQ ID NO: 638 | SEQ ID NO: 639 |
| // PHGDH // phosphoglycerate dehydrogenase | NM_006623 | SEQ ID NO: 640 | SEQ ID NO: 641 |
| // MTHFS // 5,10-methenyltetrahydrofola te synthetase (5-formyltetrahydrofolate cyclo-ligase) | NM_006441 | SEQ ID NO: 642 | SEQ ID NO: 643 |
| // PGM5 // phosphoglucomutase 5 | NM_021965 | SEQ ID NO: 644 | SEQ ID NO: 645 |
| - | | | |
| // CHI3L2 // chitinase 3-like 2 | NM_001025199 | SEQ ID NO: 646 | SEQ ID NO: 647 |
| // C19orf59 // chromosome 19 open reading frame 59 | NM_174918 | SEQ ID NO: 648 | SEQ ID NO: 649 |
| // CDH1 // cadherin 1, type 1, E-cadherin (epithelial) | NM_004360 | SEQ ID NO: 650 | SEQ ID NO: 651 |
| - | | | |
| // LDLRAP1 // low density lipoprotein receptor adaptor protein 1 | NM_015627 | SEQ ID NO: 652 | SEQ ID NO: 653 |
| // PANX1 // pannexin 1 | NM_ 015368 | SEQ ID NO: 654 | SEQ ID NO: 655 |
| // DGAT2 // diacylglycerol O-acyltransferase homolog 2 (mouse) | NM_032564 | SEQ ID NO: 656 | SEQ ID NO: 657 |
| // ABHD5 // abhydrolase domain containing 5 | NM_016006 | SEQ ID NO: 658 | SEQ ID NO: 659 |
| // STX6 // syntaxin 6 | NM_005819 | SEQ ID NO: 660 | SEQ ID NO: 661 |
| // MCTP1 // multiple C2 domains, transmembrane 1 | NM_024717 | SEQ ID NO: 662 | SEQ ID NO: 663 |
| // CCRL1 // chemokine (C-C motif) receptor-like 1 | NM_178445 | SEQ ID NO: 664 | SEQ ID NO: 665 |
| // FRMD4A // FERM domain containing 4A | NM_018027 | SEQ ID NO: 666 | SEQ ID NO: 667 |
| // CD300LD // CD300 molecule-like family member d | NM_001115152 | SEQ ID NO: 668 | SEQ ID NO: 669 |
| // SIRPB2 // signal-regulatory protein beta 2 | NM_001122962 | SEQ ID NO: 670 | SEQ ID NO: 671 |
| // C9orf150 // chromosome 9 open reading frame 150 | NM_203403 | SEQ ID NO: 672 | SEQ ID NO: 673 |
| // TMEM65 // transmembrane protein 65 | NM_194291 | SEQ ID NO: 674 | SEQ ID NO: 675 |
| // CDC42EP3 // CDC42 effector protein (Rho GTPase binding) 3 | NM_006449 | SEQ ID NO: 676 | SEQ ID NO: 677 |
| // UBASH3B // ubiquitin associated and SH3 domain containing, B | NM_032873 | SEQ ID NO: 678 | SEQ ID NO: 679 |
| // TTC39B // tetratricopeptide repeat domain 39B | NM_152574 | SEQ ID NO: 680 | SEQ ID NO: 681 |
| // TGM2 // transglutaminase 2 (C polypeptide, protein-glutamine-gammaglutamyltransferase) | NM_004613 | SEQ ID NO: 682 | SEQ ID NO: 683 |
| // KIAA1598 // KIAA1598 | NM_001127211 | SEQ ID NO: 684 | SEQ ID NO: 685 |
| // FCGR1 B // Fc fragment of IgG, high affinity lb, receptor (CD64) | NM_001017986 | SEQ ID NO: 686 | SEQ ID NO: 687 |
| // ALDH2 // aldehyde dehydrogenase 2 family (mitochondrial) | NM_000690 | SEQ ID NO: 688 | SEQ ID NO: 689 |
| // TECR // trans-2,3-enoyl-CoA reductase | NM_ 138501 | SEQ ID NO: 690 | SEQ ID NO: 691 |
| // LAPTM4B // lysosomal protein transmembrane 4 beta | NM_018407 | SEQ ID NO: 692 | SEQ ID NO: 693 |
| // DEPDC6 // DEP domain containing 6 | NM_022783 | SEQ ID NO: 694 | SEQ ID NO: 695 |
| // FCGR1A // Fc fragment of IgG, high affinity la, receptor (CD64) | NM_000566 | SEQ ID NO: 696 | SEQ ID NO: 697 |
| // CRYBG3 // betagamma crystallin domain containing 3 | NM_153605 | SEQ ID NO: 698 | SEQ ID NO: 699 |
| // LILRA5 // leukocyte immunoglobulin-like receptor, subfamily A (with TM domain), member 5 | NM_021250 | SEQ ID NO: 700 | SEQ ID NO: 701 |
| // FCGR1A // Fc fragment of IgG, high affinity la, receptor (CD64) | NM_000566 | SEQ ID NO: 702 | SEQ ID NO: 703 |
| // PDCL // phosducin-like | NM_005388 | SEQ ID NO: 704 | SEQ ID NO: 705 |
| // DGKH // diacylglycerol kinase, eta | NM_178009 | SEQ ID NO: 706 | SEQ ID NO: 707 |
| // FRK // fyn-related kinase | NM_002031 | SEQ ID NO: 708 | SEQ ID NO: 709 |
| ENST00000367321 // MTHFD1 L // methyle netetrahyd rofol ate dehydrogenase (NADP+ dependent) 1-like | ENST00000367321 | SEQ ID NO: 710 | SEQ ID NO: 711 |
| // POPDC3 // popeye domain containing 3 | NM_022361 | SEQ ID NO: 712 | SEQ ID NO: 713 |
| // AIM2 // absent in melanoma 2 | NM_004833 | SEQ ID NO: 714 | SEQ ID NO: 715 |
| // CYSLTR2 // cysteinyl leukotriene receptor 2 | NM_020377 | SEQ ID NO: 716 | SEQ ID NO: 717 |
| // RP5-1022P6.2 // hypothetical protein KIAA1434 | NM_019593 | SEQ ID NO: 718 | SEQ ID NO: 719 |
| // GPR124 // G protein-coupled receptor 124 | NM_032777 | SEQ ID NO: 720 | SEQ ID NO: 721 |
| // SVIP // small VCP/p97-interacting protein | NM_148893 | SEQ ID NO: 722 | SEQ ID NO: 723 |
| // GPHN // gephyrin | NM_ 020806 | SEQ ID NO: 724 | SEQ ID NO: 725 |
| // HK3 // hexokinase 3 (white cell) | NM_002115 | SEQ ID NO: 726 | SEQ ID NO: 727 |
| // ALOX5AP // arachidonate 5-lipoxygenase-activating protein | NM_001629 | SEQ ID NO: 728 | SEQ ID NO: 729 |
| // NCF1 // neutrophil cytosolic factor 1 | NM_000265 | SEQ ID NO: 730 | SEQ ID NO: 731 |
| // NGFRAP1 // nerve growth factor receptor (TNFRSF16) associated protein 1 | NM_206917 | SEQ ID NO: 732 | SEQ ID NO: 733 |
| // LGR4 // leucine-rich repeat-containing G protein-coupled receptor 4 | NM_018490 | SEQ ID NO: 734 | SEQ ID NO: 735 |
| // CABLES1 // Cdk5 and Abl enzyme substrate 1 | NM_138375 | SEQ ID NO: 736 | SEQ ID NO: 737 |
| // NCF1 // neutrophil cytosolic factor 1 | NM_000265 | SEQ ID NO: 738 | SEQ ID NO: 739 |
| // FLOT2 // flotillin 2 | NM_004475 | SEQ ID NO: 740 | SEQ ID NO: 741 |
| // CDC42BPB // CDC42 binding protein kinase beta (DMPK-like) | NM_006035 | SEQ ID NO: 742 | SEQ ID NO: 743 |
| // LRP1 // low density lipoprotein-related protein 1 (alpha-2-macroglobulin receptor) | NM_002332 | SEQ ID NO: 744 | SEQ ID NO: 745 |
| // C17orf76 // chromosome 17 open reading frame 76 | NM_001113567 | SEQ ID NO: 746 | SEQ ID NO: 747 |
| // TMEM150B // transmembrane protein 150B | NM_001085488 | SEQ ID NO: 748 | SEQ ID NO: 749 |
| // ENTPD3 // ectonucleoside triphosphate diphosphohydrolase 3 | NM_001248 | SEQ ID NO: 750 | SEQ ID NO: 751 |
| // HLCS // holocarboxylase synthetase (biotin-(proprionyl-Coenzyme A-carboxylase (ATP-hydrolysing)) ligase) | NM_000411 | SEQ ID NO: 752 | SEQ ID NO: 753 |
| // DENND1 B // DENN/MADD domain containing 1B | NM_001142795 | SEQ ID NO: 754 | SEQ ID NO: 755 |
| // MYO6 // myosin VI | NM_004999 | SEQ ID NO: 756 | SEQ ID NO: 757 |
| // TXNDC16 // thioredoxin domain containing 16 | NM_020784 | SEQ ID NO: 758 | SEQ ID NO: 759 |
| // FLVCR2 // feline leukemia virus subgroup C cellular receptor family, member 2 | NM_017791 | SEQ ID NO: 760 | SEQ ID NO: 761 |
| // ABCC4 // ATP-binding cassette, subfamily C (CFTR/MRP), member 4 | NM_005845 | SEQ ID NO: 762 | SEQ ID NO: 763 |
| // PFKFB3 // 6-phosphofructo-2-kinase/fructose-2,6-biphosphatase 3 | NM_004566 | SEQ ID NO:764 | SEQ ID NO: 765 |
| // SLC36A4 // solute carrier family 36 (proton/amino acid symporter), member 4 | NM_1152313 | SEQ ID NO: 766 | SEQ ID NO: 767 |
| // CLEC4E // C-type lectin domain family 4, member E | NM_014358 | SEQ ID NO:768 | SEQ ID NO: 769 |
| // CD302 // CD302 molecule | NM_014880 | SEQ ID NO: 770 | SEQ ID NO: 771 |
| // SFRP2 // secreted frizzled-related protein 2 | NM_003013 | SEQ ID NO: 772 | SEQ ID NO: 773 |
| // NCF1 // neutrophil cytosolic factor 1 | NM_000265 | SEQ ID NO: 774 | SEQ ID NO: 775 |
| // MC3R // melanocortin 3 receptor | NM_019888 | SEQ ID NO: 776 | SEQ ID NO: 777 |
| // NP // nucleoside phosphorylase | NM_000270 | SEQ ID NO: 778 | SEQ ID NO: 779 |
| // SLC9A7 // solute carrier family 9 (sodium/hydrogen exchanger), member 7 | NM_032591 | SEQ ID NO: 780 | SEQ ID NO: 781 |
| // NID1 // nidogen 1 | NM_002508 | SEQ ID NO: 782 | SEQ ID NO: 783 |
| // GPRIN3 // GPRIN family member 3 | NM_ 198281 | SEQ ID NO: 784 | SEQ ID NO: 785 |
| // GLIPR2 // GLI pathogenesis-related 2 | NM_022343 | SEQ ID NO: 786 | SEQ ID NO: 787 |
| // PLEKHH2 // pleckstrin homology domain containing, family H (with MyTH4 domain) member 2 | NM_172069 | SEQ ID NO: 788 | SEQ ID NO: 789 |
| // MERTK // c-mer proto-oncogene tyrosine kinase | NM_006343 | SEQ ID NO: 790 | SEQ ID NO: 791 |
| ENST00000367321 // MTHFD1 L // methyle netetrahyd rofol ate dehydrogenase (NADP+ dependent) 1-like | ENST00000367321 | SEQ ID NO: 792 | SEQ ID NO: 793 |
| // AK3L1 // adenylate kinase 3-like 1 | NM_001005353 | SEQ ID NO: 794 | SEQ ID NO: 795 |
| // RASA1 // RAS p21 protein activator (GTPase activating protein) 1 | NM_002890 | SEQ ID NO: 796 | SEQ ID NO: 797 |
| - | | | |
| // CXCL16 // chemokine (C-X-C motif) ligand 16 | NM_022059 | SEQ ID NO: 798 | SEQ ID NO: 799 |
| // AXL // AXL receptor tyrosine kinase | NM_021913 | SEQ ID NO: 800 | SEQ ID NO: 801 |
| // PDE3B // phosphodiesterase 3B, cGMP-inhibited | NM_000922 | SEQ ID NO: 802 | SEQ ID NO: 803 |
| ENST00000367321 // MTHFD1 L // methyle netetrahyd rofol ate dehydrogenase (NADP+ dependent) 1-like | ENST00000367321 | SEQ ID NO: 792 | SEQ ID NO: 793 |
| // LONRF3 // LON peptidase N-terminal domain and ring finger 3 | NM_001031855 | SEQ ID NO: 804 | SEQ ID NO: 805 |
| // PION // pigeon homolog (Drosophila) | NM_017439 | SEQ ID NO: 806 | SEQ ID NO: 807 |
| // BHLHE41 // basic helix-loop-helix family, member e41 | NM_030762 | SEQ ID NO: 808 | SEQ ID NO: 809 |
| // TLN2 // talin 2 | NM_015059 | SEQ ID NO: 810 | SEQ ID NO: 811 |
| // SPNS1 // spinster homolog 1 (Drosophila) | NM_032038 | SEQ ID NO: 812 | SEQ ID NO: 813 |
| // MEFV // Mediterranean fever | NM_000243 | SEQ ID NO: 814 | SEQ ID NO: 815 |
| // FAM69A // family with sequence similarity 69, member A | NM_001006605 | SEQ ID NO: 816 | SEQ ID NO: 817 |
| // LRRFIP1 // leucine rich repeat (in FLII) interacting protein 1 | NM_001137550 | SEQ ID NO: 818 | SEQ ID NO: 819 |
| // ATP10A // ATPase, class V, type 10A | NM_024490 | SEQ ID NO: 820 | SEQ ID NO: 821 |
| --- | | | |
| // EGLN3 // egl nine homolog 3 (C. elegans) | NM_022073 | SEQ ID NO: 822 | SEQ ID NO: 823 |
| // FGD2 // FYVE, RhoGEF and PH domain containing 2 | NM_173558 | SEQ ID NO: 824 | SEQ ID NO: 825 |
| // LSM6 // LSM6 homolog, U6 small nuclear RNA associated (S. cerevisiae) | NM_007080 | SEQ ID NO: 826 | SEQ ID NO: 827 |
| // MANBA // mannosidase, beta A, lysosomal | NM_005908 | SEQ ID NO: 828 | SEQ ID NO: 829 |
| // CD300LF // CD300 molecule-like family member f | NM_139018 | SEQ ID NO: 830 | SEQ ID NO: 831 |
| // C1 orf38 // chromosome 1 open reading frame 38 | NM_001105556 | SEQ ID NO: 832 | SEQ ID NO: 833 |
| // IRS2 // insulin receptor substrate 2 | NM_003749 | SEQ ID NO: 834 | SEQ ID NO: 835 |
| // CEBPB // CCAAT/enhancer binding protein (C/EBP), beta | NM_005194 | SEQ ID NO: 836 | SEQ ID NO: 837 |
| // RGL3 // ral guanine nucleotide dissociation stimulator-like 3 | NM_001161616 | SEQ ID NO: 838 | SEQ ID NO: 839 |
| // HIPK2 // homeodomain interacting protein kinase 2 | NM_022740 | SEQ ID NO: 840 | SEQ ID NO: 841 |
| // SLC25A37 // solute carrier family 25, member 37 | NM_016612 | SEQ ID NO: 842 | SEQ ID NO: 843 |
| // NRIP1 // nuclear receptor interacting protein 1 | NM_003489 | SEQ ID NO: 844 | SEQ ID NO: 845 |
| // PION // pigeon homolog (Drosophila) | NM_017439 | SEQ ID NO: 846 | SEQ ID NO: 847 |
| // TGFBR2 // transforming growth factor, beta receptor II (70/80kDa) | NM_001024847 | SEQ ID NO: 848 | SEQ ID NO: 849 |
| // UBE2CBP // ubiquitin-conjugating enzyme E2C binding protein | NM_198920 | SEQ ID NO: 850 | SEQ ID NO: 851 |
| // PCCA // propionyl Coenzyme A carboxylase, alpha polypeptide | NM_000282 | SEQ ID NO: 852 | SEQ ID NO: 853 |
| // TIMD4 // T-cell immunoglobulin and mucin domain containing 4 | NM_138379 | SEQ ID NO: 854 | SEQ ID NO: 855 |
| // NIACR1 // niacin receptor 1 | NM _ 177551 | SEQ ID NO: 856 | SEQ ID NO: 857 |
| - | | | |
| // IL28RA // interleukin 28 receptor, alpha (interferon, lambda receptor) | NM_170743 | SEQ ID NO: 858 | SEQ ID NO: 859 |
| // RARA // retinoic acid receptor, alpha | NM_000964 | SEQ ID NO: 860 | SEQ ID NO: 861 |
| // ACSL4 // acyl-CoA synthetase long-chain family member 4 | NM_022977 | SEQ ID NO: 862 | SEQ ID NO: 863 |
| // SGMS2 // sphingomyelin synthase 2 | NM_001136258 | SEQ ID NO: 864 | SEQ ID NO: 865 |
| // GMPR // guanosine monophosphate reductase | NM_006877 | SEQ ID NO: 866 | SEQ ID NO: 867 |
| // SKIL // SKI-like oncogene | NM_005414 | SEQ ID NO: 868 | SEQ ID NO: 869 |
| // HIP1 // huntingtin interacting protein 1 | NM_005338 | SEQ ID NO: 870 | SEQ ID NO: 871 |
| // EXOC5 // exocyst complex component 5 | NM_006544 | SEQ ID NO: 872 | SEQ ID NO: 873 |
| // ZC3H13 // zinc finger CCCH-type containing 13 | NM_015070 | SEQ ID NO: 874 | SEQ ID NO: 875 |
| // IMPAD1 // inositol monophosphatase domain containing 1 | NM_017813 | SEQ ID NO: 876 | SEQ ID NO: 877 |
| // SEPT4 // septin 4 | NM_080415 | SEQ ID NO: 879 | SEQ ID NO: 880 |
| // SLC1A5 // solute carrier family 1 (neutral amino acid transporter), member 5 | NM_005628 | SEQ ID NO: 881 | SEQ ID NO: 882 |
| // EML4 // echinoderm microtubule associated protein like 4 | NM_019063 | SEQ ID NO: 883 | SEQ ID NO: 884 |
| // ANPEP // alanyl (membrane) aminopeptidase | NM_001150 | SEQ ID NO: 885 | SEQ ID NO: 886 |
| // XG // Xg blood group | NM_ 001141919 | SEQ ID NO: 887 | SEQ ID NO: 888 |
| //PPP1R13B// protein phosphatase 1, regulatory (inhibitor) subunit 13B | NM_015316 | SEQ ID NO: 889 | SEQ ID NO:890 |
| // IL1 RAP // interleukin 1 receptor accessory protein | NM_002182 | SEQ ID NO: 891 | SEQ ID NO: 892 |
| // AR // androgen receptor | NM_000044 | SEQ ID NO: 893 | SEQ ID NO: 894 |
| // SLC25A33 // solute carrier family 25, member 33 | NM_032315 | SEQ ID NO: 895 | SEQ ID NO: 896 |
| // C11 orf59 // chromosome 11 open reading frame 59 | BC001706 | SEQ ID NO: 897 | SEQ ID NO: 898 |
| // ABHD2 // abhydrolase domain containing 2 | NM_007011 | SEQ ID NO: 899 | SEQ ID NO:900 |
| // DENND5A // DENN/MADD domain containing 5A | NM_015213 | SEQ ID NO: 901 | SEQ ID NO: 902 |
| // KCNJ15 // potassium inwardly-rectifying channel, subfamily J, member 15 | NM_002243 | SEQ ID NO: 903 | SEQ ID NO: 904 |
| // CHRNA5 // cholinergic receptor, nicotinic, alpha 5 | NM_000745 | SEQ ID NO: 905 | SEQ ID NO: 906 |
| // IRAK3 // interleukin-1 receptor-associated kinase 3 | NM_007199 | SEQ ID NO: 907 | SEQ ID NO: 908 |
| // SYTL4 // synaptotagmin-like 4 | NM_080737 | SEQ ID NO: 909 | SEQ ID NO: 910 |
| // SNORD38B // small nucleolar RNA, C/D box 38B | NR_001457 | SEQ ID NO: 911 | N/A |
| // LRRFIP1 // leucine rich repeat (in FLII) interacting protein 1 | NM_001137550 | SEQ ID NO: 912 | SEQ ID NO: 913 |
| // ZNF124 // zinc finger protein 124 | NM_003431 | SEQ ID NO: 914 | SEQ ID NO: 915 |
| // CLEC12A // C-type lectin domain family 12, member A | NM_138337 | SEQ ID NO: 916 | SEQ ID NO: 917 |
| // CBL // Cas-Br-M (murine) ecotropic retroviral transforming sequence | NM_005188 | SEQ ID NO: 918 | SEQ ID NO: 919 |
| // MMP14 // matrix metallopeptidase 14 (membrane-inserted) | NM_004995 | SEQ ID NO: 920 | SEQ ID NO: 921 |
| // CCDC23 // coiled-coil domain containing 23 | NM_199342 | SEQ ID NO: 922 | SEQ ID NO: 923 |
| // TBC1D2B // TBC1 domain family, member 2B | NM_144572 | SEQ ID NO: 924 | SEQ ID NO: 925 |
| // PAK1 // p21 protein (Cdc42/Rac)-activated kinase 1 | NM_001128620 | SEQ ID NO: 926 | SEQ ID NO: 927 |
| // PAQR5 // progestin and adipoQ receptor family member V | NM_001104554 | SEQ ID NO: 928 | SEQ ID NO: 929 |
| // BNC2 // basonuclin 2 | NM_017637 | SEQ ID NO: 930 | SEQ ID NO: 931 |
| // DENND1 B // DENN/MADD domain containing 1B | NM_144977 | SEQ ID NO: 932 | SEQ ID NO: 933 |
| // PPP2R3A // protein phosphatase 2 (formerly 2A), regulatory subunit B", alpha | NM_002718 | SEQ ID NO: 934 | SEQ ID NO: 935 |
| // ALDOC // aldolase C, fructose-bisphosphate | NM_005165 | SEQ ID NO: 936 | SEQ ID NO: 937 |
| // KCTD10 // potassium channel tetramerisation domain containing 10 | NM_031954 | SEQ ID NO: 938 | SEQ ID NO: 939 |
| // BIN2 // bridging integrator 2 | NM_016293 | SEQ ID NO: 940 | SEQ ID NO: 941 |
| // FAM82A2 // family with sequence similarity 82, member A2 | NM_018145 | SEQ ID NO: 942 | SEQ ID NO: 943 |
| // TNIP3 // TNFAIP3 interacting protein 3 | NM_024873 | SEQ ID NO: 944 | SEQ ID NO: 945 |
| // FGD4 // FYVE, RhoGEF and PH domain containing 4 | NM_139241 | SEQ ID NO: 946 | SEQ ID NO: 947 |
| // FAM89A // family with sequence similarity 89, member A | NM_198552 | SEQ ID NO: 948 | SEQ ID NO: 949 |
| // SNX10 // sorting nexin 10 | NM_013322 | SEQ ID NO: 950 | SEQ ID NO: 951 |
| // FBXO9 // F-box protein 9 | AK095307 | SEQ ID NO: 952 | N/A |
| // PLCB2 // phospholipase C, beta 2 | NM_004573 | SEQ ID NO: 953 | SEQ ID NO: 954 |
| // HACL1 // 2-hydroxyacyl-CoA lyase 1 | NM_012260 | SEQ ID NO: 955 | SEQ ID NO: 956 |
| // KIAA0564 // KIAA0564 | NM_015058 | SEQ ID NO: 957 | SEQ ID NO: 958 |
| // MNDA // myeloid cell nuclear differentiation antigen | NM_002432 | SEQ ID NO: 959 | SEQ ID NO: 960 |
| // ACOT11 // acyl-CoA thioesterase 11 | NM_147161 | SEQ ID NO: 961 | SEQ ID NO: 962 |
| // MAP3K8 // mitogen-activated protein kinase kinase kinase 8 | NM_005204 | SEQ ID NO: 963 | SEQ ID NO: 964 |
| // C5orf27 // chromosome 5 open reading frame 27 | NR_026936 | SEQ ID NO: 965 | N/A |
| //CD14//CD14 molecule | NM_000591 | SEQ ID NO: 966 | SEQ ID NO: 967 |
| // FMNL2 // formin-like 2 | NM_052905 | SEQ ID NO: 968 | SEQ ID NO: 969 |
| // FMNL3 // formin-like 3 | NM_175736 | SEQ ID NO: 970 | SEQ ID NO: 971 |
| // PLEK // pleckstrin | NM_ 002664 | SEQ ID NO: 972 | SEQ ID NO: 973 |
| // CXCR7 // chemokine (C-X-C motif) receptor 7 | NM_020311 | SEQ ID NO: 974 | SEQ ID NO: 975 |
| // PLAUR // plasminogen activator, urokinase receptor | NM_002659 | SEQ ID NO: 976 | SEQ ID NO: 977 |
| // BTK // Bruton agammaglobulinemia tyrosine kinase | NM_000061 | SEQ ID NO: 978 | SEQ ID NO: 979 |
| // VAMP4 // vesicle-associated membrane protein 4 | NM_003762 | SEQ ID NO: 980 | SEQ ID NO: 981 |
| // CCIN // calicin | NM_005893 | SEQ ID NO: 982 | SEQ ID NO: 983 |
| // ACTN1 // actinin, alpha 1 | NM_001130004 | SEQ ID NO: 984 | SEQ ID NO: 985 |
| - | | | |
| // DHCR7 // 7-dehydrocholesterol reductase | NM_001360 | SEQ ID NO: 986 | SEQ ID NO: 987 |
| // SYT17 // synaptotagmin XVII | NM_016524 | SEQ ID NO: 988 | SEQ ID NO: 989 |
| // TECR // trans-2,3-enoyl-CoA reductase | NM_ 138501 | SEQ ID NO: 990 | SEQ ID NO: 991 |
| // SLC7A7 // solute carrier family 7 (cationic amino acid transporter, y+ system), member 7 | NM_003982 | SEQ ID NO: 992 | SEQ ID NO: 993 |
| // APOC1 // apolipoprotein C-I | NM_001645 | SEQ ID NO: 994 | SEQ ID NO: 995 |
| // ECHDC1 // enoyl Coenzyme A hydratase domain containing 1 | NM_001002030 | SEQ ID NO: 996 | SEQ ID NO: 997 |
| ACVRL1//activin A receptor type II-like 1 | NM_000020 | SEQ ID NO: 1153 | SEQ ID NO: 1154 |
| CASP9//caspase 9, apoptosis-related cysteine peptidase | NM_001229 | SEQ ID NO: 1155 | SEQ ID NO: 1156 |
| EGR2 //early growth response 2 | NM_000399 | SEQ ID NO: 1157 | SEQ ID NO: 1158 |
| FBN1//fibrillin 1 | NM_000138 | SEQ ID NO: 1159 | SEQ ID NO: 1160 |
| FFAR2//free fatty acid receptor 2 | NM_005306 | SEQ ID NO: 1161 | SEQ ID NO: 1162 |
| GPR82//G protein-coupled receptor 82 | NM_080817 | SEQ ID NO: 1163 | SEQ ID NO: 1164 |
| HCK //hemopoietic cell kinase | NM_002110 | SEQ ID NO: 1165 | SEQ ID NO: 1166 |
| SCARNA7//small Cajal body-specific RNA 7 | NR_003001 | SEQ ID NO: 1167 | N/A |
| SNORD34 //small nucleolar RNA, C | NR_000019 | SEQ ID NO: 1168 | N/A |
| SPN //sialophorin | NM_001030288 | SEQ ID NO: 1169 | SEQ ID NO: 1170 |
| TMEM163// transmembrane protein 163 | NM_030923 | SEQ ID NO: 1171 | SEQ ID NO: 1172 |
| TSPAN7//tetraspanin 7 | NM_004615 | SEQ ID NO: 1173 | SEQ ID NO: 1174 |
| ZMYND17// zinc finger, MYND-type containing 17 | NM_001024593 | SEQ ID NO: 1175 | SEQ ID NO: 878 |

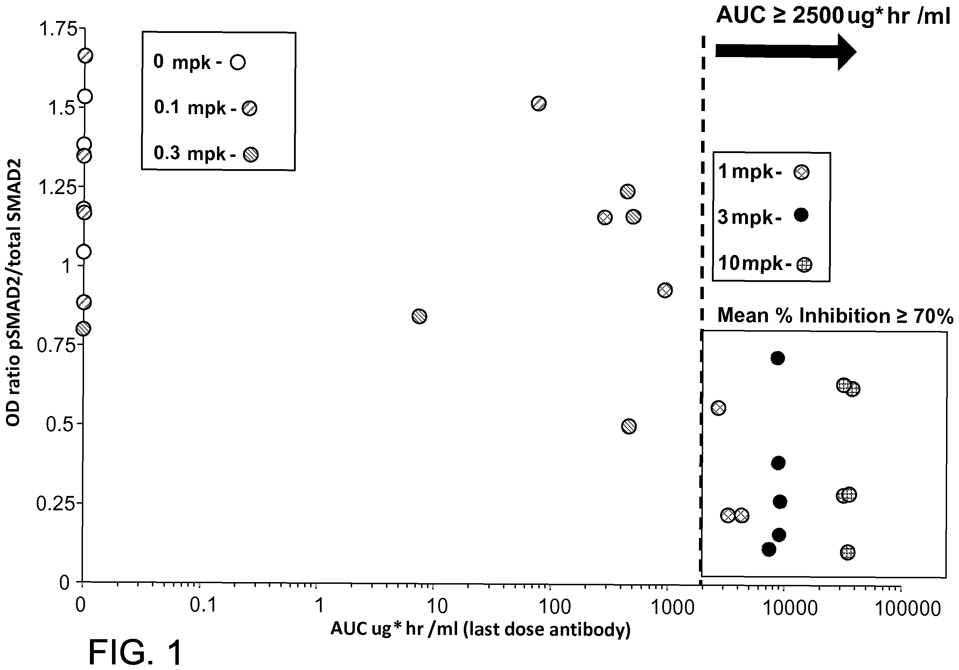

In Figure 1, the data show expression of the ratio of phosphorylated SMAD2 (pSMAD2) protein relative to total SMAD2 protein in BAL cells isolated from cynomolgus monkeys after 8-weekly doses of STX-100 treatment relative to circulating levels of STX-100 in serum after the last (8^{th}) weekly dose of antibody (area under the curve (AUC) ug*hr/ml). Data are shown for individual animals in vehicle and 0.1, 0.3, 1.0, 3.0, and 10 mg/kg STX-100 treatment groups. pSMAD2 and total SMAD2 levels were determined by ELISA analysis.

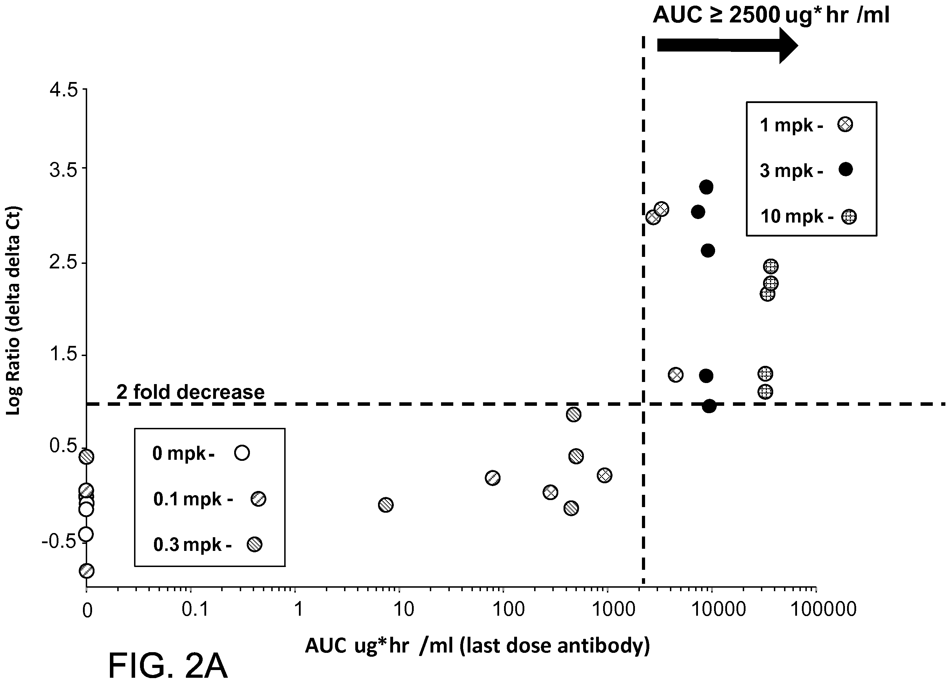

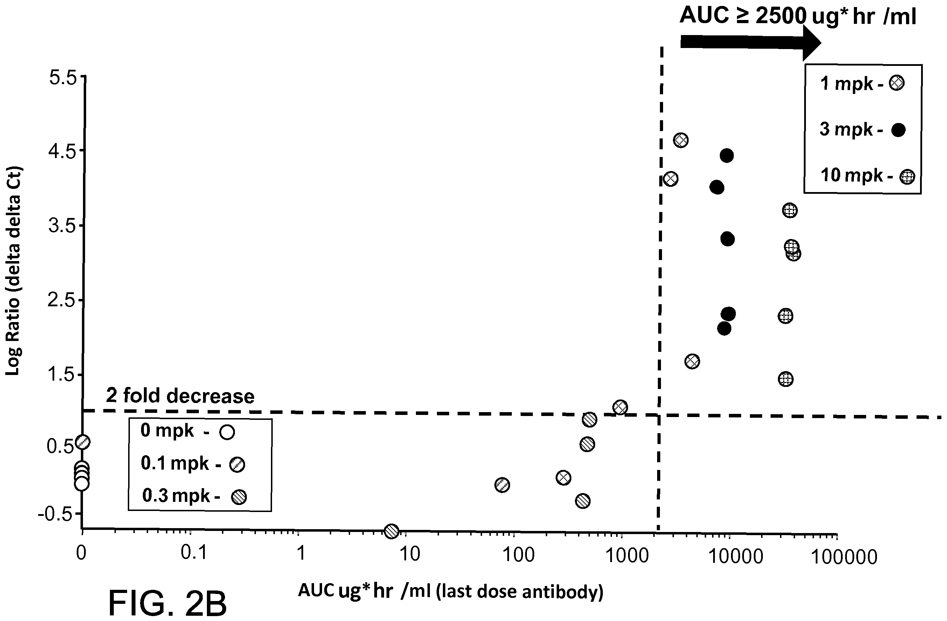

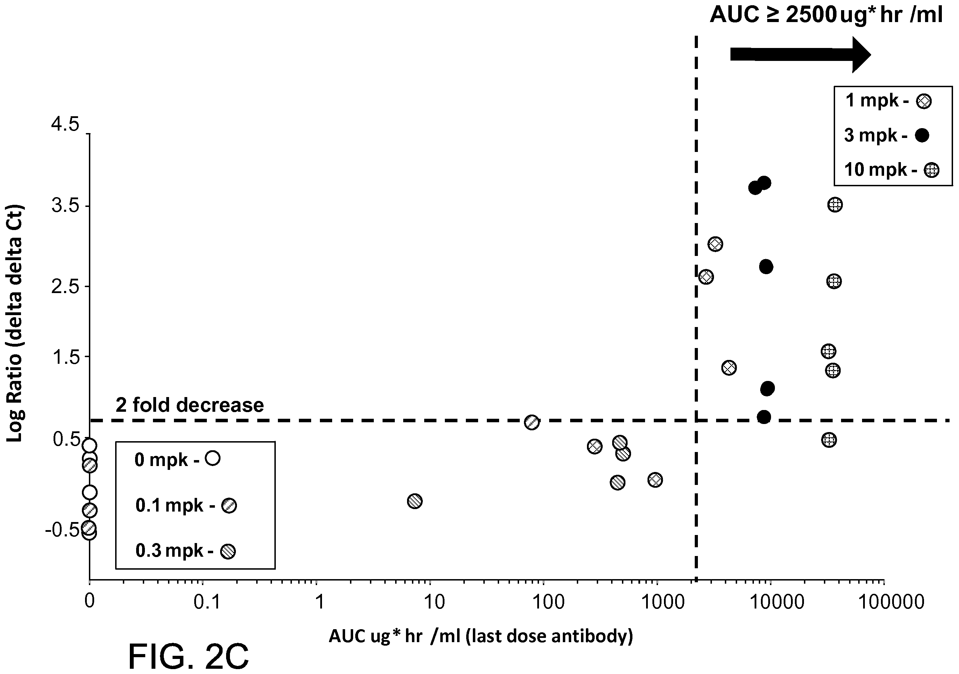

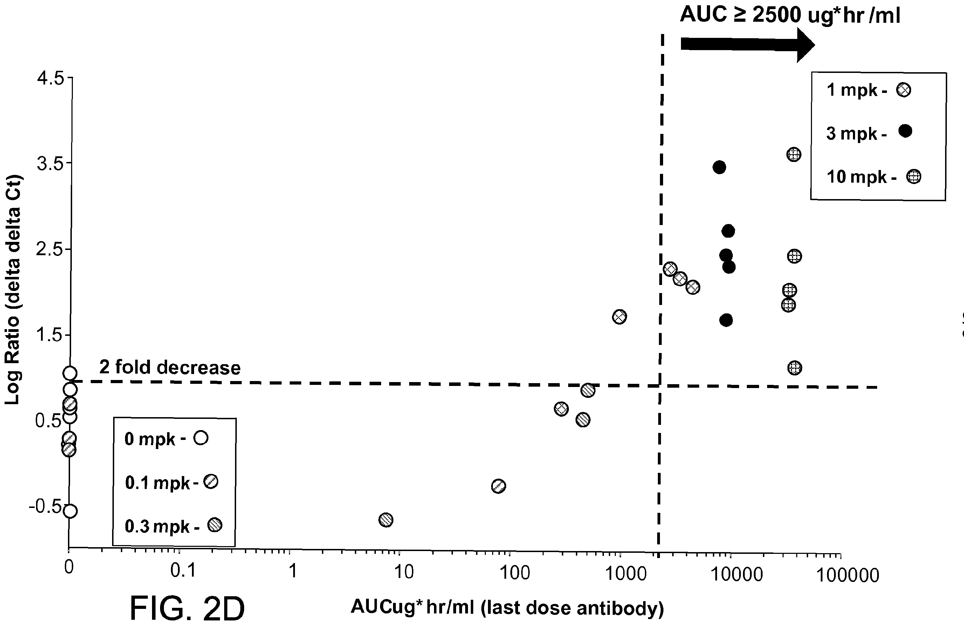

In Figure 2, the data show expression of example genes a) ALOX5, b) OLR1, c) Serpine1, and d) TGM2 in BAL cells isolated from cynomolgus monkeys after 8-weekly doses of STX-100 treatment relative to circulating levels of STX-100 in serum after the last (8^{th}) weekly dose of antibody (area under the curve (AUC) ug*hr/ml). Data are shown for individual animals in vehicle and 0.1, 0.3, 1.0, 3.0, and 10 mg/kg STX-100 treatment groups. Gene expression was determined by Taqman® gene expression analysis.

### Example 2: Quantitative Polymerase Chain Reaction Studies on Gene Expression Levels in BAL Macrophage Cells Following Injection of 3G9 antibody in Mice.

Wild type mice were either treated with 2 doses of 3G9 antibody at three different concentrations (i.e., 0.3 mg/kg, 1 mg/kg, or 3 mg/kg) 7 days apart or not treated (control). BAL macrophages were isolated from the mice 24 hours after the second dose and quantitative polymerase chain reaction was used to determine the expression level of Cathepsin L, Legumain, PAI-1 (also known as Serpine1), Osteopontin, TREM-1, MMP-19, and ALCAM.

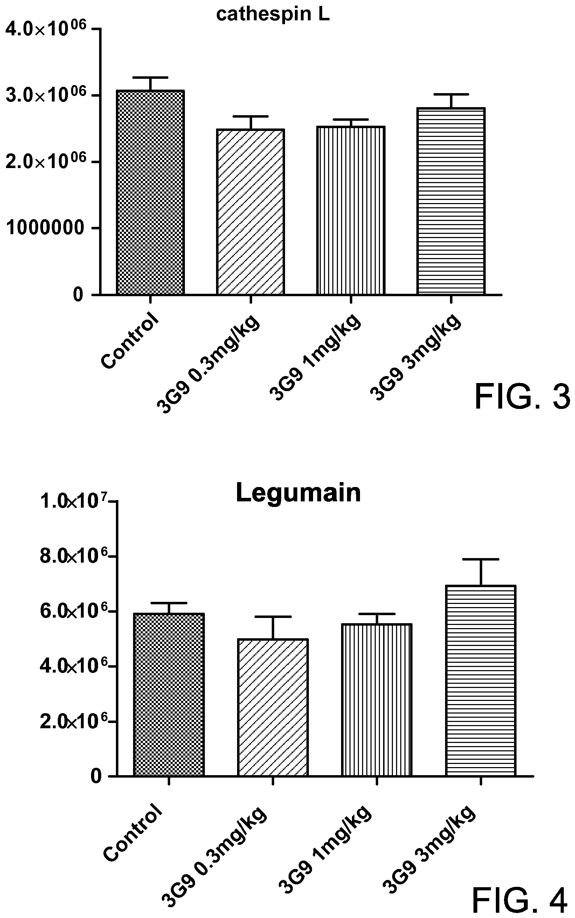

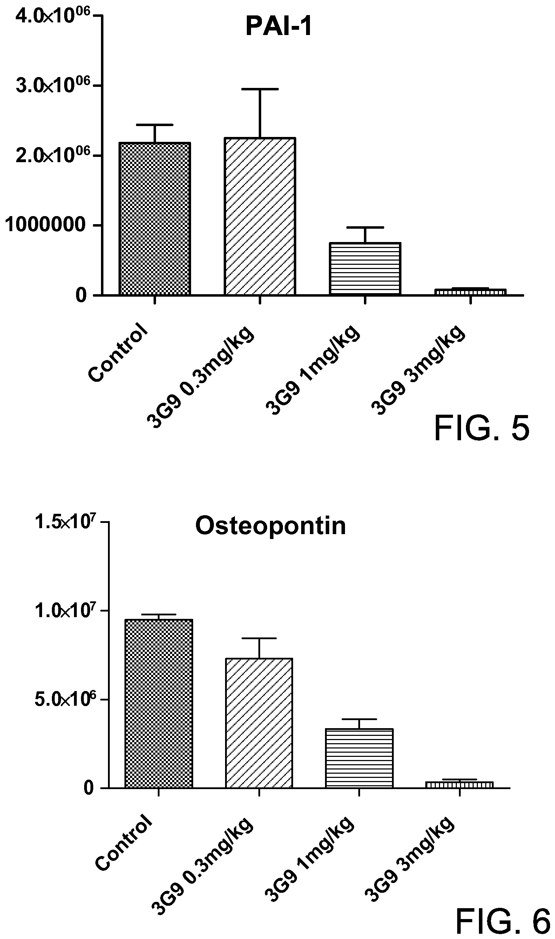

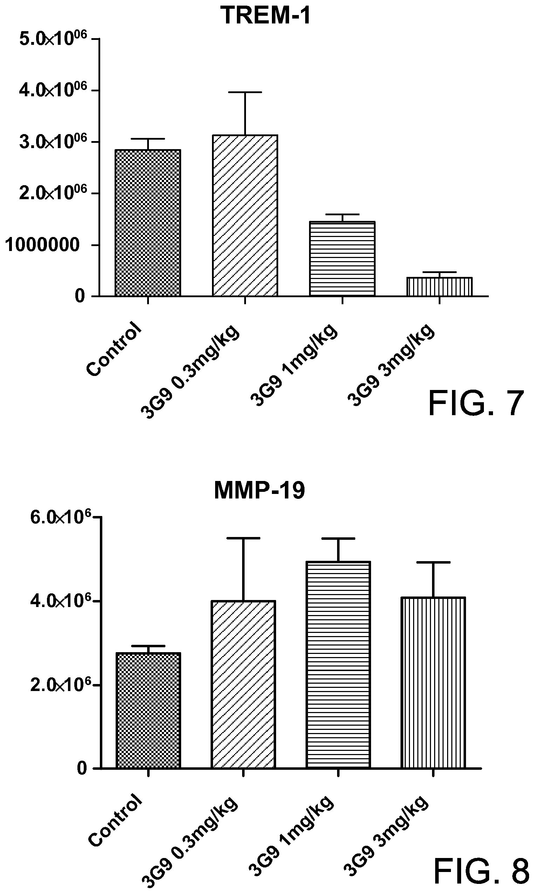

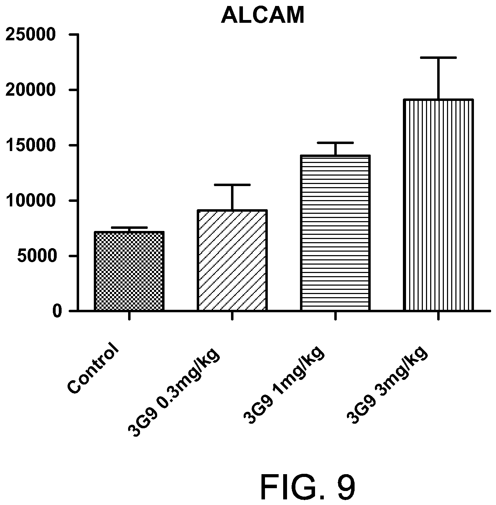

3G9 treatment did not significantly affect the expression of Cathepsin L or Legumain (Figures 3 and 4). However, 3G9 treatment increased the expression of MMP19 and ALCAM, and reduced the expression of osteopontin, TREM-1 and PAI-1 (Figures 5-9).

## Claims

1. A method for predicting whether a human subject who has an αvβ6-mediated disorder will respond to treatment with an αvβ6-integrin inhibitor, the method comprising:
a) providing a biological sample that has been obtained from the human subject after having been administered the αvβ6-integrin inhibitor; and
b) measuring the expression level of one or more gene(s) or protein(s) selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in the biological sample, wherein
a decrease in the expression level of the one or more gene(s) or protein(s) relative to a control expression level predicts that the human subject will respond, or has an increased likelihood of responding, to treatment with the αvβ6-integrin inhibitor, wherein the αvβ6-mediated disorder is selected from the group consisting of lung fibrosis, idiopathic pulmonary fibrosis, radiation induced fibrosis, bleomycin induced fibrosis, asbestos induced fibrosis, lung cancer, and acute lung injury, scleroderma, wherein the αvβ6-integrin inhibitor is an anti-αvβ6-integrin antibody, and wherein the biological sample is bronchoalveolar lavage fluid.

2. The method of claim 1, further comprising determining the phosphorylation status of SMAD2 protein in the biological sample, wherein a decrease in the phosphorylation status of SMAD2 protein after administration of the αvβ6 integrin inhibitor is a further predictor that the human subject will respond, or has an increased likelihood of responding, to treatment with the αvβ6-integrin inhibitor.

3. The method of claim 1, wherein a decrease in the expression level of at least one of arachidonate 5-lipoxygenase 5 (ALOX5), oxidized low density lipoprotein receptor 1 (OLR1), plasminogen activator inhibitor-1 (PAI-1 or SERPINE1), transglutaminase 2 (TGM2), or triggering receptor expressed on myeloid cells 1 (TREM1) in the biological sample is measured and predicts that the human subject will respond, or has an increased likelihood of responding, to treatment with the αvβ6-integrin inhibitor.

4. An αvβ6-integrin inhibitor for use in a method for predicting responsiveness of a human subject who has an αvβ6-mediated disorder to the αvβ6-integrin inhibitor, the method comprising:
(a) measuring the expression level of a gene or protein selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in a first biological sample obtained from the human subject before the αvβ6-integrin inhibitor is administered; and
(b) measuring the expression level of the gene or protein selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in a second biological sample obtained from the human subject after the αvβ6-integrin inhibitor has been administered,
wherein a decrease in the level of expression of the gene or protein measured in step (b), compared to the level of expression of the gene or protein measured in step (a) predicts that the human subject will respond, or has an increased likelihood of responding, to treatment with the αvβ6-integrin inhibitor, wherein the αvβ6-mediated disorder is selected from the group consisting of lung fibrosis, idiopathic pulmonary fibrosis, radiation induced fibrosis, bleomycin induced fibrosis, asbestos induced fibrosis,, lung cancer, and acute lung injury, scleroderma, wherein the αvβ6-integrin inhibitor is an anti-αvβ6-integrin antibody, and wherein the biological sample is bronchoalveolar lavage fluid.

5. A therapeutically effective amount of an αvβ6 integrin inhibitor for use in treating an αvβ6mediated disorder in a human subject in need thereof, wherein the human subject has been identified as having: an increased expression level of a gene or protein selected from the group consisting of arachidonate 5-lipoxygenase 5 (ALOX5), fibronectin (FN1), oxidized low density lipoprotein receptor 1 (OLR1), plasminogen activator inhibitor-1 (PAI-1 or SERPINE1), transglutaminase 2 (TGM2), and triggering receptor expressed on myeloid cells 1 (TREM1) in a biological sample obtained from the human subject, compared to a control expression level,
wherein the αvβ6-mediated disorder is selected from the group consisting of lung fibrosis, idiopathic pulmonary fibrosis, radiation induced fibrosis, bleomycin induced fibrosis, asbestos induced fibrosis, lung cancer, and acute lung injury, scleroderma, wherein the αvβ6-integrin inhibitor is an anti-αvβ6-integrin antibody, and wherein the biological sample is bronchoalveolar lavage fluid.

6. The method of claim 1, wherein the response to treatment with the αvβ6-integrin inhibitor is a response indicative of the success or failure of a patient's treatment with an αvβ6 integrin inhibitor.

7. The inhibitor for the use of claim 5, wherein the human subject has previously been treated with the αvβ6 integrin inhibitor.

8. The method of any one of claims 1, 3 and 6 or the inhibitor for the use of claim 5 or claim 7, wherein the anti-αvβ6-integrin antibody has the same CDRs as an antibody produced by a hybridoma selected from the group consisting of: 6.1A8 (ATCC accession number PTA-3647); hybridoma 6.3G9 (ATCC accession number PTA-3649); 6.8G6 (ATCC accession number PTA-3645); 6.2E5 (ATCC accession number PTA-3897); 6.2B1 (ATCC accession number PTA-3646); 7.1G10 (ATCC accession number PTA-3898); 7.7G5 (ATCC accession number PTA-3899); 7.1C5 (ATCC accession number PTA-3900); and 6.3G9 (ATCC accession number PTA-3649), except that the light chain CDR 1 of 6.3G9 contains an asparagine to serine substitution such that the light chain CDR 1 sequence is the sequence of SASSSVSSSYLY (SEQ ID NO:1196).

9. The method of any one of claims 1, 3 and 6 or the inhibitor for the use of claim 5 or claim 7, wherein the anti-αvβ6-integrin antibody comprises a heavy chain variable region comprising the amino acid sequence set forth in SEQ ID NO: 1210.

10. The method of claim 9 or the inhibitor for the use of claim 9, wherein the anti-αvβ6-integrin antibody further comprises a light chain variable region comprising the amino acid sequence set forth in SEQ ID NO: 1211.

11. The method of claims 1 or 3, or the inhibitor for the use of claims 4 or 5, wherein the mRNA level of the gene is measured or wherein the expression level of the protein is measured.

12. A biomarker panel comprising a probe for each of ALOX5, FN1, OLR1, SERPINE1, TGM2, and TREM1 and no additional genes or proteins other than one or more of the genes or proteins selected from any one of SEQ ID NOs:1-1175.

13. The biomarker panel of claim 12, wherein the probe is a nucleotide probe, optionally a DNA probe or an antibody.

## Patentansprüche

1. Verfahren zur Vorhersage, ob ein menschlicher Patient, der an einer αvβ6-vermittelten Erkrankung leidet, auf die Behandlung mit einem αvβ6-Integrin-Inhibitor ansprechen wird, wobei das Verfahren Folgendes umfasst:
a) Bereitstellen einer biologischen Probe, die von dem menschlichen Patienten nach Verabreichung des ανβ6-Integrin-Inhibitors erhalten wurde; und
b) Messen des Expressionsniveaus eines Gens oder Proteins oder mehrerer Gene oder Proteine, die ausgewählt sind aus der Gruppe, bestehend aus Arachidonat-5-Lipoxygenase 5 (ALOX5), Fibronectin (FN1), Oxidized-Low-Density-Lipoprotein-Rezeptor 1 (OLR1), Plasminogen-Aktivator-Inhibitor-1 (PAI-1 oder SERPINE1), Transglutaminase 2 (TGM2) und Triggering Receptor Expressed on Myeloid Cells 1 (TREM1), in der biologischen Probe, wobei:
eine Abnahme des Expressionsniveaus des Gens oder Proteins oder der mehreren Gene oder Proteine relativ zu einem Kontrollexpressionsniveau vorhersagt, dass der menschliche Patient auf eine Behandlung mit dem ανβ6-Integrin-Inhibitor ansprechen wird oder mit erhöhter Wahrscheinlichkeit ansprechen wird, wobei die ανβ6-vermittelte Erkrankung ausgewählt ist aus der Gruppe, bestehend aus Lungenfibrose, idiopathischer Lungenfibrose, strahleninduzierter Fibrose, bleomycininduzierter Fibrose, asbestinduzierter Fibrose, Lungenkrebs und akuter Lungenverletzung, Sklerodermie, wobei der αvβ6-Integrin-Inhibitor ein Anti-αvβ6-Integrin-Antikörper ist und wobei die biologische Probe ein bronchoalveoläres Lavagefluid ist.

2. Verfahren nach Anspruch 1, ferner umfassend das Bestimmen des Phosphorylierungsstatus des SMAD2-Proteins in der biologischen Probe, wobei eine Abnahme des Phosphorylierungsstatus des SMAD2-Proteins nach Verabreichung des ανβ6-Integrin-Inhibitors ein weiterer Prädiktor dafür ist, dass der menschliche Patient auf die Behandlung mit dem ανβ6-Integrin-Inhibitor ansprechen wird oder mit erhöhter Wahrscheinlichkeit ansprechen wird.

3. Verfahren nach Anspruch 1, wobei eine Abnahme des Expressionsniveaus von mindestens einem von Arachidonat-5-Lipoxygenase 5 (ALOX5), Oxidized-Low-Density-Lipoprotein-Rezeptor 1 (OLR1), Plasminogen-Aktivator-Inhibitor-1 (PAI-1 oder SERPINE1), Transglutaminase 2 (TGM2) oder Triggering Receptor Expressed on Myeloid Cells 1 (TREM1) in der biologischen Probe gemessen wird und vorhersagt, dass der menschliche Patient auf die Behandlung mit dem ανβ6-Integrin-Inhibitor ansprechen wird oder mit erhöhter Wahrscheinlichkeit ansprechen wird.

4. αvβ6-Integrin-Inhibitor zur Verwendung in einem Verfahren zur Vorhersage des Ansprechverhaltens eines menschlichen Patienten, der an einer ανβ6-vermittelten Erkrankung leidet, auf den αvβ6-Integrin-Inhibitor, wobei das Verfahren Folgendes umfasst:
(a) Messen des Expressionsniveaus eines Gens oder Proteins, das ausgewählt ist aus der Gruppe, bestehend aus Arachidonat-5-Lipoxygenase 5 (ALOX5), Fibronectin (FN1), Oxidized-Low-Density-Lipoprotein-Rezeptor 1 (OLR1), Plasminogen-Aktivator-Inhibitor-1 (PAI-1 oder SERPINE1), Transglutaminase 2 (TGM2) und Triggering Receptor Expressed on Myeloid Cells 1 (TREM1), in einer ersten biologischen Probe, die von dem menschlichen Patienten vor Verabreichung des ανβ6-Integrin-Inhibitors erhalten wurde; und
(b) Messen des Expressionsniveaus des Gens oder Proteins das ausgewählt ist aus der Gruppe, bestehend aus Arachidonat-5-Lipoxygenase 5 (ALOX5), Fibronectin (FN1), Oxidized-Low-Density-Lipoprotein-Rezeptor 1 (OLR1), Plasminogen-Aktivator-Inhibitor-1 (PAI-1 oder SERPINE1), Transglutaminase 2 (TGM2) und Triggering Receptor Expressed on Myeloid Cells 1 (TREM1), in einer zweiten biologischen Probe, die von dem menschlichen Patienten nach der Verabreichung des ανβ6-Integrin-Inhibitors erhalten wurde,
wobei eine Abnahme des Expressionsniveaus des Gens oder Proteins, das in Schritt (b) gemessen wurde, im Vergleich zu dem Expressionsniveau des Gens oder Proteins, das in Schritt (a) gemessen wurde, vorhersagt, dass der menschliche Patient auf eine Behandlung mit dem αvβ6-Integrin-Inhibitor ansprechen wird oder mit erhöhter Wahrscheinlichkeit ansprechen wird, wobei die ανβ6-vermittelte Erkrankung ausgewählt ist aus der Gruppe, bestehend aus Lungenfibrose, idiopathischer Lungenfibrose, strahleninduzierter Fibrose, bleomycininduzierter Fibrose, asbestinduzierter Fibrose, Lungenkrebs und akuter Lungenverletzung, Sklerodermie, wobei der ανβ6-Integrin-Inhibitor ein Anti-αvβ6-Integrin-Antikörper ist und wobei die biologische Probe ein bronchoalveoläres Lavagefluid ist.

5. Therapeutisch wirksame Menge eines ανβ6-Integrin-Inhibitors zur Verwendung bei der Behandlung einer ανβ6-vermittelten Erkrankung bei einem menschlichen Patienten, der diese benötigt, wobei festgestellt wurde, dass der menschliche Patient mindestens Folgendes aufweist: ein erhöhtes Expressionsniveau eines Gens oder Proteins, das ausgewählt ist aus der Gruppe, bestehend aus Arachidonat-5-Lipoxygenase 5 (ALOX5), Fibronectin (FN1), Oxidized-Low-Density-Lipoprotein-Rezeptor 1 (OLR1), Plasminogen-Aktivator-Inhibitor-1 (PAI-1 oder SERPINE1), Transglutaminase 2 (TGM2) und Triggering Receptor Expressed on Myeloid Cells 1 (TREM1), in einer biologischen Probe, die von dem menschlichen Patienten erhalten wurde, im Vergleich zu einem Kontrollexpressionsniveau,
wobei die ανβ6-vermittelte Erkrankung ausgewählt ist aus der Gruppe, bestehend aus Lungenfibrose, idiopathischer Lungenfibrose, strahleninduzierter Fibrose, bleomycininduzierter Fibrose, asbestinduzierter Fibrose, Lungenkrebs und akuter Lungenverletzung, Sklerodermie, wobei der αvβ6-Integrin-Inhibitor ein Anti-αvβ6-Integrin-Antikörper ist und wobei die biologische Probe ein bronchoalveoläres Lavagefluid ist.

6. Verfahren nach Anspruch 1, wobei das Ansprechen auf die Behandlung mit dem ανβ6-Integrin-Inhibitor ein Ansprechen ist, das den Erfolg oder das Fehlschlagen der Behandlung eines Patienten mit einem αvβ6-Integrin-Inhibitor angibt.

7. Inhibitor zur Verwendung nach Anspruch 5, wobei der menschliche Patient bereits zuvor mit dem ανβ6-Integrin-Inhibitor behandelt worden ist.

8. Verfahren nach einem der Ansprüche 1, 3 und 6 oder Inhibitor zur Verwendung nach Anspruch 5 oder Anspruch 7, wobei der Anti-αvβ6-Integrin-Antikörper die gleichen CDRs aufweist wie ein Antikörper, der von einem Hybridom hergestellt wird, das ausgewählt ist aus der Gruppe, bestehend aus: 6.1A8 (ATCC-Hinterlegungsnummer PTA-3647); Hybridom 6.3G9 (ATCC-Hinterlegungsnummer PTA-3649); 6.8G6 (ATCC-Hinterlegungsnummer PTA-3645); 6.2E5 (ATCC-Hinterlegungsnummer PTA-3897); 6.2B1 (ATCC-Hinterlegungsnummer PTA-3646); 7.1G10 (ATCC-Hinterlegungsnummer PTA-3898); 7.7G5 (ATCC-Hinterlegungsnummer PTA-3899)7.1C5 (ATCC-Hinterlegungsnummer PTA-3900) und 6.3G9 (ATCC-Hinterlegungsnummer PTA-3649), mit der Ausnahme, dass die CDR 1 der leichten Kette von 6.3G9 eine Substitution von Asparagin zu Serin aufweist, derart, dass die Sequenz der CDR 1 der leichten Kette die Sequenz SASSSVSSSYLY (SEQ-ID-NR.: 1196) ist.

9. Verfahren nach einem der Ansprüche 1, 3 und 6 oder Inhibitor zur Verwendung nach Anspruch 5 oder Anspruch 7, wobei der Anti-αvβ6-Integrin-Antikörper eine variable Region der schweren Kette umfasst, die die in SEQ-ID-NR.: 1210 angegebene Aminosäuresequenz umfasst.

10. Verfahren nach Anspruch 9 oder Inhibitor zur Verwendung nach Anspruch 9, wobei der Anti-αvβ6-Integrin-Antikörper ferner eine variable Region der leichten Kette umfasst, die die in SEQ-ID-NR.: 1211 angegebene Aminosäuresequenz umfasst.

11. Verfahren nach Anspruch 1 oder 3, oder Inhibitor zur Verwendung nach Anspruch 4 oder 5, wobei das mRNA-Niveau des Gens gemessen wird oder wobei das Expressionsniveau des Proteins gemessen wird.

12. Biomarker-Panel, das eine Sonde für jedes von ALOX5, FN1, OLR1, SERPINE1, TGM2 und TREM1 und keine zusätzlichen Gene oder Proteine außer einem oder mehreren der Gene oder Proteine, die aus einer der SEQ-ID-NR.: 1-1175 ausgewählt sind, umfasst.

13. Biomarker-Panel nach Anspruch 12, wobei die Sonde eine Nukleotidsonde, optional eine DNS-Sonde oder ein Antikörper, ist.

## Revendications

1. Procédé pour prédire si un sujet humain qui a un trouble médié par αvβ6 répondra au traitement avec un inhibiteur de l'intégrine αvβ6, le procédé comprenant :
a) la fourniture d'un échantillon biologique qui a été obtenu auprès du sujet humain après l'administration de l'inhibiteur de l'intégrine αvβ6 ; et
b) la mesure du niveau d'expression d'un(e) ou de plusieurs gène(s) ou protéine(s) choisi(e)s dans le groupe constitué de l'arachidonate 5-lipoxygénase 5 (ALOX5), de la fibronectine (FN1), du récepteur 1 des lipoprotéines oxydées de basse densité (OLR1), de l'inhibiteur de l'activateur de plasminogène-1 (PAI-1 ou SERPINE1), de la transglutaminase 2 (TGM2) et du récepteur de déclenchement exprimé sur les cellules myéloïdes 1 (TREM1) dans l'échantillon biologique, dans lequel
une diminution du niveau d'expression des un(e) ou plusieurs gène(s) ou protéine(s) par rapport à un niveau d'expression de contrôle prédit que le sujet humain répondra, ou a une probabilité accrue de répondre au traitement avec l'inhibiteur de l'intégrine αvβ6, dans lequel le trouble médié par αvβ6 est choisi dans le groupe constitué de la fibrose pulmonaire, de la fibrose pulmonaire idiopathique, de la fibrose induite par rayonnement, de la fibrose induite par la bléomycine, de la fibrose induite par l'amiante, du cancer du poumon et de la lésion pulmonaire aiguë, de la sclérodermie, dans lequel l'inhibiteur de l'intégrine αvβ6 est un anticorps anti-intégrine αvβ6, et dans lequel l'échantillon biologique est un liquide de lavage bronchoalvéolaire.

2. Procédé selon la revendication 1, comprenant en outre la détermination de l'état de phosphorylation de la protéine SMAD2 dans l'échantillon biologique, dans lequel une diminution de l'état de phosphorylation de la protéine SMAD2 après l'administration de l'inhibiteur de l'intégrine αvβ6 est un autre prédicteur que le sujet humain répondra, ou a une probabilité accrue de répondre au traitement avec l'inhibiteur d'intégrine αvβ36.

3. Procédé selon la revendication 1, dans lequel une diminution du niveau d'expression d'au moins l'un de l'arachidonate 5-lipoxygénase 5 (ALOX5), du récepteur 1 des lipoprotéines oxydées de basse densité (OLR1), de l'inhibiteur de l'activateur de plasminogène-1 (PAI-1 ou SERPINE1), de la transglutaminase 2 (TGM2), ou du récepteur de déclenchement exprimé sur les cellules myéloïdes 1 (TREM1) dans l'échantillon biologique est mesurée et prédit que le sujet humain répondra, ou a une probabilité accrue de répondre au traitement avec l'inhibiteur de l'intégrine αvβ6.

4. Inhibiteur de l'intégrine αvβ6 destiné à être utilisé dans un procédé pour prédire la réponse d'un sujet humain qui a un trouble médié par αvβ36 à l'inhibiteur de l'intégrine αvβ6, le procédé comprenant :
(a) la mesure du niveau d'expression d'un gène ou d'une protéine choisi(e) dans le groupe constitué de l'arachidonate 5-lipoxygénase 5 (ALOX5), de la fibronectine (FN1), du récepteur 1 des lipoprotéines oxydées de basse densité (OLR1), de l'inhibiteur de l'activateur de plasminogène-1 (PAI-1 ou SERPINE1), de la transglutaminase 2 (TGM2) et du récepteur de déclenchement exprimé sur les cellules myéloïdes 1 (TREM1) dans un premier échantillon biologique obtenu auprès du sujet humain avant l'administration de l'inhibiteur de l'intégrine αvβ6 ; et
(b) la mesure du niveau d'expression du gène ou de la protéine choisi(e) dans le groupe constitué de l'arachidonate 5-lipoxygénase 5 (ALOX5), de la fibronectine (FN1), du récepteur 1 des lipoprotéines oxydées de basse densité (OLR1), de l'inhibiteur de l'activateur de plasminogène-1 (PAI-1 ou SERPINE1), de la transglutaminase 2 (TGM2) et du récepteur de déclenchement exprimé sur les cellules myéloïdes 1 (TREM1) dans un second échantillon biologique obtenu auprès du sujet humain après l'administration de l'inhibiteur de l'intégrine αvβ6,
dans lequel une diminution du niveau d'expression du gène ou de la protéine mesuré à l'étape (b), par rapport au niveau d'expression du gène ou de la protéine mesuré à l'étape (a) prédit que le sujet humain répondra, ou a une probabilité accrue de répondre au traitement avec l'inhibiteur de l'intégrine αvβ6, dans lequel le trouble médié par αvβ6 est choisi dans le groupe constitué de la fibrose pulmonaire, de la fibrose pulmonaire idiopathique, de la fibrose induite par rayonnement, de la fibrose induite par la bléomycine, de la fibrose induite par l'amiante, du cancer du poumon et de la lésion pulmonaires aiguë, de la sclérodermie, dans lequel l'inhibiteur de l'intégrine αvβ6 est un anticorps anti-intégrine αvβ6, et dans lequel l'échantillon biologique est un fluide de lavage bronchoalvéolaire.

5. Quantité thérapeutiquement efficace d'un inhibiteur de l'intégrine αvβ6 à utiliser dans le traitement d'un trouble médié par αvβ6 chez un sujet humain qui en a besoin, dans laquelle le sujet humain a été identifié comme ayant : un niveau d'expression accru d'un gène ou d'une protéine choisi(e) dans le groupe constitué de l'arachidonate 5-lipoxygénase 5 (ALOX5), de la fibronectine (FN1), du récepteur 1 des lipoprotéines oxydées de basse densité (OLR1), de l'inhibiteur de l'activateur de plasminogène-1 (PAI-1 ou SERPINE1), de la transglutaminase 2 (TGM2) et du récepteur de déclenchement exprimé sur les cellules myéloïdes 1 (TREM1) dans un échantillon biologique obtenu auprès du sujet humain, par rapport à un niveau d'expression de contrôle,
dans lequel le trouble médié par αvβ6 est choisi dans le groupe constitué de la fibrose pulmonaire, de la fibrose pulmonaire idiopathique, de la fibrose induite par rayonnement, de la fibrose induite par la bléomycine, de la fibrose induite par l'amiante, du cancer du poumon et de la lésion pulmonaire aiguë, de la sclérodermie, dans lequel l'inhibiteur de l'intégrine αvβ6 est un anticorps anti-intégrine αvβ6, et dans lequel l'échantillon biologique est un liquide de lavage bronchoalvéolaire.

6. Procédé selon la revendication 1, dans lequel la réponse au traitement avec l'inhibiteur de l'intégrine αvβ6 est une réponse indiquant le succès ou l'échec du traitement d'un patient avec un inhibiteur de l'intégrine αvβ6.

7. Inhibiteur destiné à être utilisé selon la revendication 5, dans lequel le sujet humain a été préalablement traité avec l'inhibiteur de l'intégrine αvβ6.

8. Procédé selon l'une quelconque des revendications 1, 3 et 6 ou inhibiteur destiné à être utilisé selon la revendication 5 ou la revendication 7, dans lequel l'anticorps anti-intégrine αvβ6 a les mêmes CDR qu'un anticorps produit par un hybridome choisi dans le groupe constitué : de 6.1A8 (numéro d'accès ATCC PTA-3647) ; de l'hybridome 6.3G9 (numéro d'accès ATCC PTA-3649) ; de 6.8G6 (numéro d'accès ATCC PTA-3645) ; de 6.2E5 (numéro d'accès ATCC PTA-3897) ; de 6.2B1 (numéro d'accès ATCC PTA-3646) ; de 7.1G10 (numéro d'accès ATCC PTA-3898) ; de 7.7G5 (numéro d'accès ATCC PTA-3899) ; de 7.1C5 (numéro d'accès ATCC PTA-3900) ; et de 6.3G9 (numéro d'accès ATCC PTA-3649), sauf que la CDR 1 à chaîne légère de 6.3G9 contient une substitution de l'asparagine à la sérine de sorte que la séquence CDR 1 à chaîne légère est la séquence de SASSSVSSSYLY (SEQ ID NO: 1196).

9. Procédé selon l'une quelconque des revendications 1, 3 et 6 ou inhibiteur destiné à être utilisé selon la revendication 5 ou la revendication 7, dans lequel l'anticorps anti-intégrine αvβ6 comprend une région variable à chaîne lourde comprenant la séquence d'acides aminés présentée dans SEQ ID NO: 1210.

10. Procédé selon la revendication 9 ou inhibiteur destiné à être utilisé selon la revendication 9, dans lequel l'anticorps anti-intégrine αvβ6 comprend en outre une région variable à chaîne légère comprenant la séquence d'acides aminés présentée dans SEQ ID NO: 1211.

11. Procédé selon les revendications 1 ou 3, ou inhibiteur destiné à être utilisé selon les revendications 4 ou 5, dans lequel le niveau d'ARNm du gène est mesuré ou dans lequel le niveau d'expression de la protéine est mesuré.

12. Groupe de biomarqueurs comprenant une sonde pour chacun d'ALOX5, de FN1, d'OLR1, de SERPINE1, de TGM2 et de TREM1, et aucun(e) gène ou protéine supplémentaire autre qu'un ou plusieurs des gènes ou protéines choisis parmi l'une quelconque des SEQ ID NO: 1-1175.

13. Groupe de biomarqueurs selon la revendication 12, dans lequel la sonde est une sonde nucléotidique, éventuellement une sonde ADN ou un anticorps.
